# EUROPÄISCHE PATENTSCHRIFT

(11) **EP 0 331 616 B1**
(45) Veröffentlichungstag und Bekanntmachung des Hinweises auf die Patenterteilung: **08.11.1995**
(21) Anmeldenummer: 89730046.3
(22) Anmeldetag: 27.02.1989
(51) Int. Cl.: C08G 69/48, C08G 73/02, A61K 49/00

(54) **Polymer-gebundene Komplexbildner, deren Komplexe, Verfahren zu ihrer Herstellung und diese enthaltende pharmazeutische Mittel**
Polymer-bonded complexe builders, their complexes, process for their preparation and pharmaceutical agents containing the same
Formateur de complexe lié à un polymère, ces complexes, procédé de préparation et milieu pharmaceutique les contenant

(30) Priorität: 29.02.1988 DE 3806795
(43) Veröffentlichungstag der Anmeldung: 06.09.1989
(73) Patentinhaber: SCHERING AKTIENGESELLSCHAFT, 13342 Berlin (DE)
(72) Erfinder: Deutsch, Julius, Dr., D-1000 Berlin 19 (DE); Schmitt-Willich, Heribert, Dr., D-1000 Berlin 65 (DE); Neumeier, Reinhard, Dr., D-1000 Berlin 27 (DE); Conrad, Jürgen, Dr., D-1000 Berlin 41 (DE); Gries, Heinz, Dr., D-1000 Berlin 31 (DE)

(56) Entgegenhaltungen:
- EP-A- 0 169 299
- EP-A- 0 268 707
- EP-A- 0 277 088
- FR-A- 2 539 996
- FR-A- 2 590 484
- Biochem. Biophys. Res. Comm., Band 77, N. 2, 1977, S. 581-585 (Krejcarek et al.)
- Conjugate Chem., 1992, 3, S. 212-217 (Maisano et al.)

## Beschreibung

Die Erfindung betrifft den in den Patentansprüchen gekennzeichneten Gegenstand, das heißt neue Polymer-Komplexe, diese Verbindungen enthaltende Mittel, ihre Verwendung zur Herstellung Diagnostika sowie Verfahren zur Herstellung dieser Verbindungen und Mittel.

Die Anwendung von Komplexbildnern oder Komplexen bzw. deren Salzen in der Medizin ist seit langem bekannt. Als Beispiele seien genannt:
Komplexbildner als Stabilisatoren pharmazeutischer Präparate, Komplexe und deren Salze als Hilfsmittel zur Verabreichung schlecht löslicher Ionen (z.B. Eisen), Komplexbildner und Komplexe (bevorzugt Calcium- oder Zink-), gegebenenfalls als Salze mit anorganischen und/oder organischen Basen, als Antidots zur Entgiftung bei versehentlicher Inkorporation von Schwermetallen oder deren radioaktiven Isotopen und Komplexbildner als Hilfsmittel in der Nuklearmedizin unter Verwendung radioaktiver Isotope wie ^{99m}Tc für die Szintigraphie sind bekannt.

In der Patentschrift DE-OS 3401052 sind neuerdings paramagnetische Komplexsalze als Diagnostika, vorwiegend als NMR-Diagnostika vorgeschlagen worden.

Diese Komplexe oder Komplexsalze sind recht gut verträglich und gewährleisten eine weitestgehend vollständige Ausscheidung der paramagnetischen Ionen. Der Nachteil ist allerdings, daß sie sich nur unspezifisch im Extrazellulärraum verteilen und daher nur im Ausnahmefall für eine Erkennung pathologisch veränderter Gewebe taugen.

Der Ansatz, zumindest einen Teil dieser Probleme durch Verwendung von Komplexbildnern, die einerseits durch ionische Bindung an das jeweils geeignete Metall (siehe unten) sowie andererseits durch Bindung an eine funktionelle Gruppe oder ein als Carrier-Molekül dienendes nicht-toxisches und möglichst organ-spezifisches Molekül gebunden sind, zu lösen, war bisher nur sehr begrenzt erfolgreich.

So ist beispielsweise die Anzahl paramagnetischer Zentren in den Komplexen, die in den Europäischen Patentanmeldungen No. 88 695 und No. 150 884 beschrieben sind, für eine organspezifische Bildgebung nicht ausreichend.

Erhöht man die Anzahl der benötigten Metallionen durch mehrfache Einführung komplexierender Einheiten in ein Makromolekül, so ist das immer mit einer nicht tolerierbaren Beeinträchtigung der Affinität und/oder Spezifizität dieses Makromoleküls verbunden [J. Nucl. Med. 24, 1158 (1983)].

In Biophysica Acta. 883 (1986), 460 - 467 und der Europäischen Patentanmeldung EP-A- 0 268 707 wird die Möglichkeit vorgestellt, durch Umsetzung mit cyclischem DTPA-Anhydrid bzw. gemischtem DTPA-Anhydrid [Methode nach Krejcarek et al., Biochem. Biophys. Res. Comm. 77, 581 (1977)] gewonnene paramagnetische Ion-konjugierte monoklonale Antiköper als spezifische NMR-Kontrastmittel für die Bildgebung zu verwenden.

Die erfindungsgemäßen Verbindungen unterscheiden sich von diesen Verbindungen dadurch, daß sie nicht an ein weiteres Makromolekül gebunden sind.

Es besteht daher für vielfältige Zwecke ein Bedarf an stabilen, gut löslichen, aber auch besser verträglichen, gut zugänglichen Komplexverbindungen, die eine möglichst große Anzahl der benötigten Metallionen im Komplex enthalten, ohne daß ihre Affinität und/oder Spezifizität verloren geht. Der Erfindung liegt somit die Aufgabe zugrunde, diese Verbindungen und Mittel zur Verfügung zu stellen, sowie ein möglichst einfaches Verfahren zu ihrer Herstellung zu schaffen. Diese Aufgabe wird durch die Erfindung gelöst.

Es wurde gefunden, daß sich Polymer-Komplexe, die aus einem Carbonsäure-gruppen enthaltenden, mit Amid-, Hydrazid- und/oder alkylierten oder acylierten Imino-Untereinheiten versehenen Liganden und mindestens einem Ion eines Elements der Ordnungszahlen 21 -29, 42, 44 oder 57 - 83 sowie gegebenenfalls Kationen anorganischer und/oder organischer Basen, Aminosäuren oder Aminosäureamide bestehen, überraschenderweise hervorragend zur Herstellung von NMR-, Röntgen- und Ultraschall-Diagnostika eignen, da sie vor allem die für diese Verwendung benötigte Anzahl von Metallionen im Komplex stabil gebunden enthalten.

Die erfindungsgemäßen Polymere weisen als Polymer-Einheiten komplexbildende Strukturen der allgemeinen Formel I auf 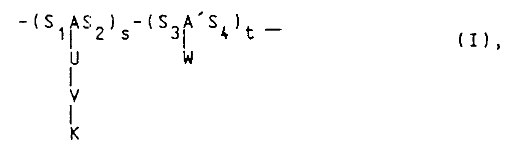
worin
- A und A': jeweils 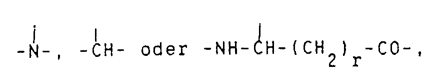 mit r in der Bedeutung der Ziffern 0 oder 1,
- s: die ganzen Zahlen von 7 bis 20.000
- t: die ganzen Zahlen von 0 bis 20.000,
- U: eine direkte Bindung, die Gruppe 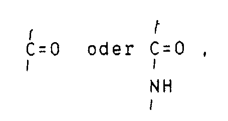
- V, S₁,S₂,S₃ und S₄: jeweils eine gegebenenfalls Imino-, Phenylen-, Phenylenoxy-, Phenylenimino-, Amid-, Hydrazid-, Estergruppe(n), Sauerstoff-, Schwefel- und/oder Stickstoff-Atom(e) enthaltende gegebenenfalls durch Hydroxy-, Mercapto-, Imino-, Epoxy-, Oxo-, Thioxo- und/oder Aminogruppe-(n) substituierte geradkettige, verzweigte, gesättigte oder ungesättigte C₀-C₂₀-Alkylengruppe,
- K: ein Komplexbildner der allgemeinen Formeln IA, IB, IC oder ID 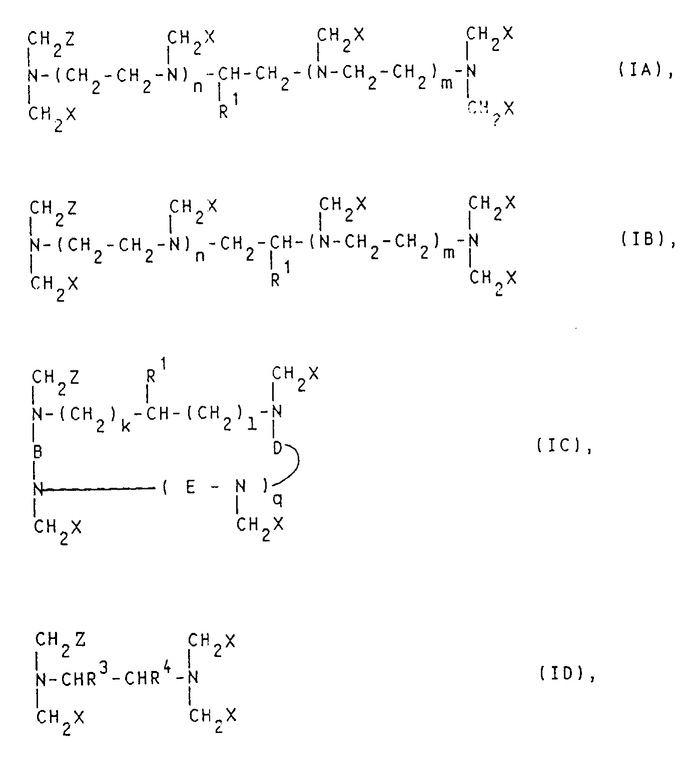 wobei
n und m jeweils für die Ziffern 0, 1, 2, 3 oder 4, wobei n und m zusammen nicht mehr als 4 ergeben,
k für die Ziffern 1, 2, 3, 4 oder 5,
l für die Ziffern 0, 1, 2, 3, 4 oder 5,
q für die Ziffern 0, 1 oder 2,
X unabhängig voneinander jeweils für die Reste -COOH oder V' steht, worin
V' den am Ende eine funktionelle Gruppe aufweisenden Rest V bedeutet, wobei, falls das Molekül V' enthält, mindestens 0,1% der Substituenten X für V' stehen,
- B, D und E,: die gleich oder verschieden sind, jeweils für die Gruppe 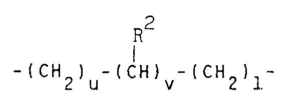 mit
R² in der Bedeutung von Wasserstoff oder einer gegebenenfalls Sauerstoff- und/oder Stickstoffatom(e) enthaltenden gegebenenfalls durch Hydroxy-und/oder Aminogruppe(n) substituierten geradkettigen, verzweigten, gesättigten oder ungesättigten C₁-C₂₀-Alkylgruppe,
u in der Bedeutung der Ziffern 0, 1, 2, 3, 4 oder 5,
v in der Bedeutung der Ziffern 0 oder 1,
wobei B, D und E jeweils mindestens 2 und maximal 5 Kohlenstoffatome enthalten,
- Z: für die Gruppe 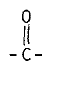 oder den Rest X,
- R¹: für eine direkte Bindung oder ein Wasserstoffatom,
- R³ und R⁴: gemeinsam für eine gegebenenfalls durch 1-2 Hydroxy- oder 1-3 C₁-C₄-Alkylgruppen substituierte Dimethylen- oder Trimethylen-methingruppe, stehen,
mit der Maßgabe, daß Z nur dann für die Gruppe 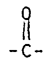 steht, wenn R¹ gleichzeitig ein Wasserstoffatom bedeutet, und daß Z nur dann für den Rest X steht, wenn R¹ gleichzeitig eine direkte Bindung bedeutet,
- W: ein Wasserstoffatom, die Gruppe U_{w}-V_{w}-K_{w}, wobei U_{w}, V_{w} und K_{w} jeweils eine der für U, V und K genannten Bedeutungen haben, V' oder die Gruppe 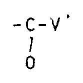
bedeuten, mit der Maßgabe, daß gewünschtenfalls ein Teil der COOH-Gruppen als Ester und/oder Amid vorliegt.
Unter einer Cₒ-Alkylenkette ist eine direkte Bindung zu verstehen.
Als bevorzugte Polymer-Einheiten seien die komplexbildenden Strukturen der allgemeinen Formel Iα genannt: 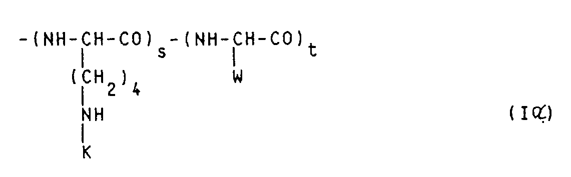
Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21-29, 42, 44 und 57-70. Geeignete Ionen sind beispielsweise das Chrom(III)-, Mangan (II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium (III)-, Dysprosium(III)-, Holmium(III)-, Erbium(III)- und Eisen(III)-ion.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion und die oben genannten Ionen der Lanthanidenreihe.

Die erfindungsgemäßen Polymer-Komplexe enthalten mindestens ein Ion eines Elements der oben genannten Ordnungszahl.

Die für V stehende Alkylengruppe sowie die für R², R und R' stehende Alkylgruppe kann geradkettig, verzweigt, cyclisch, aliphatisch, aromatisch oder arylaliphatisch sein und bis zu 20 Kohlenstoffatome aufweisen. Bevorzugt sind geradkettige Mono- bis Decamethylengruppen sowie C₁-C₄-Alkylenphenylgruppen.
Zur Verdeutlichung seien folgende Alkylengruppen beispielhaft genannt:
-CH₂-O-C₆H₄-CH₂-; -CH₂-CH(OH)-CH₂-O-C₆H₄-CH₂-; -C(=NH)-O-C₆H₄-CH₂-; -(CH₂)₄-NH-CO-CH₂-O-C₆H₄-CH₂-; -(CH₂)₄-NH-CH₂-CH(OH)-CH₂-O-C₆H₄-CH₂-; -(CH₂)₃-O-C₆H₄-CH₂-; -CH₂-CO-NH-(CH₂)₃-O-CH₂-; -CH₂-CO-NH-NH-; -CH₂-CONH-(CH₂)₂-; -CH₂-CO-NH(CH₂)₁₀-; -CH₂-CONH-(CH₂)₂-S-; -(CH₂)₄-NH-CO-(CH₂)₈-; -CH₂-CO-NH-(CH₂)₃-NH-; -(CH₂)₃-NH;
Bevorzugte funktionelle Gruppen, die sich am Ende der V''-Alkylengruppe befinden sind beispielsweise die Maleimidobenzoyl-, 3-Sulfomaleimidobenzoyl-, 4-(Maleimidomethyl)-cyclohexylcarbonyl-, 4-[3-Sulfo-(maleimidomethyl)-cyclohexyl-carbonyl-, 4-(p-Maleimidophenyl)-butyryl-, 3-(2-Pyridyldithio)propionyl-, Methacryloyl-(pentamethylen)amido-, Bromacetyl-, Jodacetyl-, 3-Jodpropyl-, 2-Bromethyl-, 3-Mercaptopropyl-, 2-Mercaptoethyl-, Phenylenisothiocyanat-, 3-Aminopropyl-, Benzylester-, Ethylester-, t-Butylester-, Amino-, C₁-C₆-Alkylamino-, Aminocarbonyl-, Hydrazino-, Hydrazinocarbonyl-, Maleimido-, Methacrylamido-, Methacryloylhydrazinocarbonyl-, Maleimidamidocarbonyl-, Halogeno-, Mercapto-, Hydrazinotrimethylenhydrazinocarbonyl-, Aminodimethylenamidocarbonyl-, Bromcarbonyl-, Phenylendiazonium-, Isothiocyanat-, Semicarbazid-, Thiosemicarbazid-Gruppe.
Zur Verdeutlichung seien einige ausgewählte Gruppen aufgeführt: 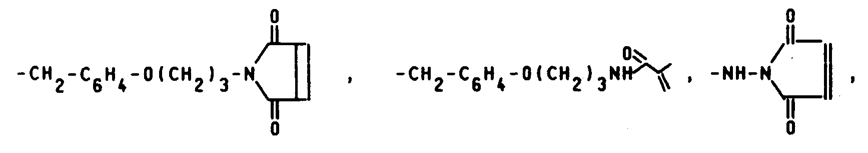
-CH₂-C₆H₄-O(CH₂)₅CO₂CH₂C₆H₅, -CH₂-C₆H₄-O-CH₂-CO₂CH₂C₆H₅, -CH₂-C₆H₄-O(CH₂)₅CONHNH₂, 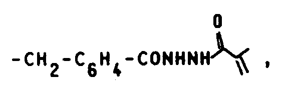
-CH₂-C₆H₄-O(CH₂)₄-SH, -CH₂-C₆H₄-O(CH₂)₃NHNH₂, 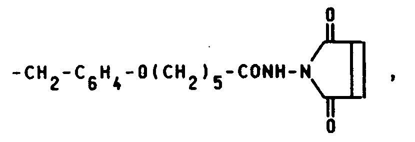
-CH₂-C₆H₄-O(CH₂)₃Br, -CH₂-C₆H₄-O(CH₂)₅CONHNH-(CH₂)₃-NHNH₂, -CH₂-NHNH₂, -CH₂-SH, -CH₂CONHNH₂, -(CH₂)₃SH, -CH₂-C₆H₄-O-CH₂COBr, -C₆H₄NHCOCH₂Br, 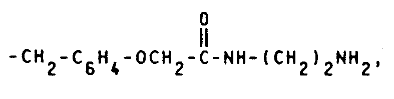
-CH₂-C₆H₄-NH₂, -C₆H₄-N₂, -C₆H₄NHCS, 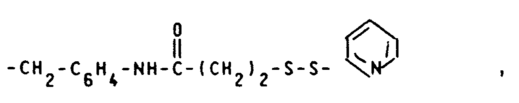
-NHCO-NH-NH₂, -NHCS-NH-NH₂, 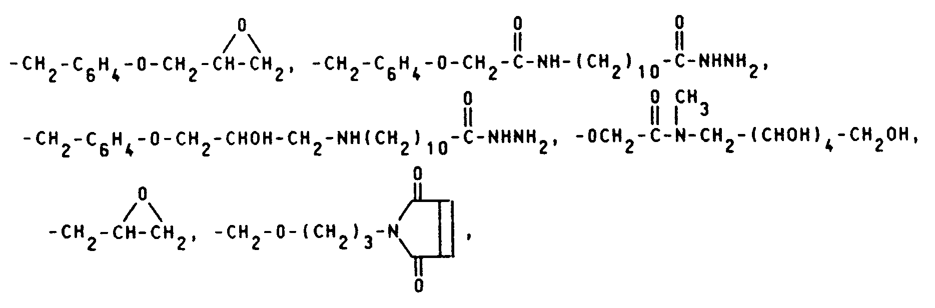
-CH₂-O-(CH₂)₄-SH, -CH₂-O-(CH₂)₃-NHNH₂, 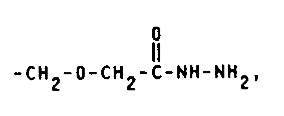
-CH₂-O-CH₂-CH₂-NH₂, 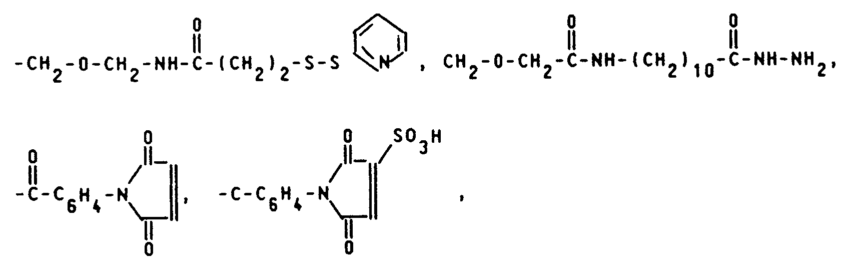
-C≡C-C≡C-R, -C≡C-CH=CRR', 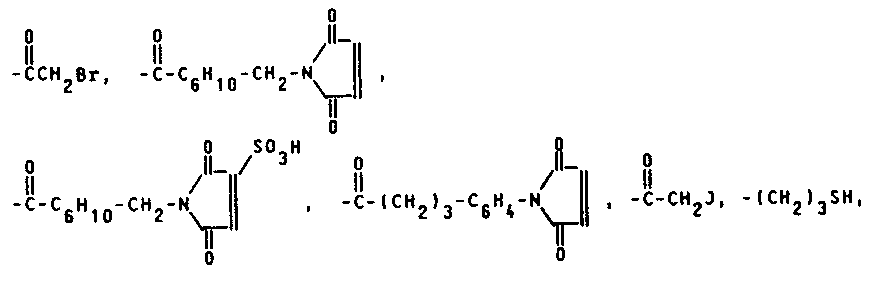
-(CH₂)₃NH₂, -C₆H₄SCN, 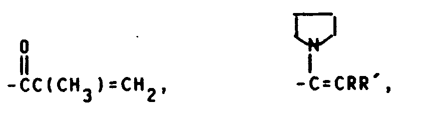
C₆H₄CH₂Br, 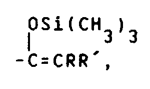
-CH₂Br, -CH₂J, -CH=CH-CH₂Br, -OSO₂C₆H₄CH₃, -SO₂Cl, -SOCl, 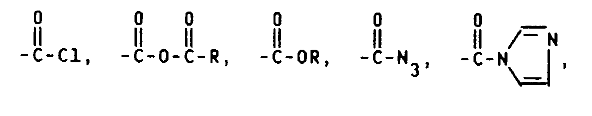
-CH=CH-CO₂R,
wobei R und R' gleich oder verschieden sind und jeweils für ein Wasserstoffatom, einen gesättigten oder ungesättigten gegebenenfalls durch eine Phenylgruppe substituierten C₁-C₂₀-Alkylrest oder eine Phenylgruppe stehen.

Als Beispiele für die Komplexbildner-Reste K seien diejenigen der Ethylendiamintetraessigsäure, Diethylentriaminpentaessigsäure, trans-1,2-Cyclohexandiamintetraessigsäure, 1,4,7,10-Tetraazacyclododecantetraessigsäure, 1,4,7-Triazacyclononan-triessigsäure, 1,4,8,11-Tetraazatetradecantetraessigsäure und 1,5-9-Triazacyclododecantriessigsäure genannt, die über (jeweils in K enthaltend) ein Kohlenstoffatom oder eine Carbonylgruppe an die Reste der Polymer-Einheiten gebunden sind. Gewünschtenfalls kann ein Teil der Carbonsäuren als Ester und/oder Amid vorliegen.

Als für die Herstellung der erfindungsgemäßen Polymer-Komplexe geeignete Polymere seien beispielhaft Polyethylenimin, Polylysin, Polyasparaginsäure, Polyethyleniminpolyessigsäureester und Polyacrylester genannt.

Die restlichen aziden Wasserstoffatome, das heißt diejenigen, die nicht durch das Zentralion substituiert worden sind, können gegebenenfalls ganz oder teilweise durch Kationen anorganischer und/oder organischer Basen oder Aminosäuren ersetzt sein. Die entsprechenden Säuregruppen können auch ganz oder teilweise in Ester oder Amide überführt sein.

Geeignete anorganische Kationen sind beispielsweise das Lithiumion, das Kaliumion, das Calciumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.
Geeignete Ester sind vorzugsweise diejenigen mit einem C₁-C₆-Alkylrest; genannt seien beispielsweise der Methyl-, Ethyl- und tertiär-Butylrest.
Sollen die Carbonsäuregruppen zumindest teilweise als Amide vorliegen, so sind tertiäre Amide bevorzugt. Als Reste kommen gesättigte, ungesättigte, gerad- oder verzweigtkettige oder cyclische Kohlenwasserstoffe mit bis zu 5 C-Atomen infrage, die gegebenenfalls durch 1 bis 3 Hydroxy- oder C₁-C₄-Alkoxy-Gruppen substituiert sind. Beispielsweise genannt seien die Methyl-, Ethyl-, 2-Hydroxyethyl-, 2-Hydroxy-1-(hydroxymethyl)-ethyl-, 1-(Hydroxymethyl)-ethyl, Propyl-, Isopropenyl-, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxybutyl-, 4-Hydroxybutyl-,2-, 3- und 4-Hydroxy2-methylbutyl-, 2- und 3-Hydroxyisobutyl-, 2,3,4-Trihydroxybutyl-, 1,2,4-Trihydroxybutyl-, Pentyl-, Cyclopentyl- und 2-Methoxyethyl-gruppe. Der Amidrest kann auch ein unter Einschluß des Amid-Stickstoffs gebildeter heterocyclischer 5- oder 6-Ring sein. Beispielhaft genannt seien:
der Pyrrolidinyl-, Piperidyl-, Pyrazolidinyl-, Pyrrolinyl-, Pyrazolinyl-, Piperazinyl-, Morpholinyl-, Imidazolidinyl-, Oxazolidinyl-, Thiazolidinyl-Ring.

Die erfindungsgemäßen Polymer-Komplexe enthalten die für ihre Verwendung benötigte große Anzahl von Metallionen im Komplex stabil gebunden.

So ergeben z.B. Gleichgewichts- bzw. Umkomplexierungs-Untersuchungen mit dem als Stand der Technik anzusehendem, in der Fachwelt als gutes Kontrastmittel anerkanntem Gadolinium-Komplex der Diethylentriaminpentaessigsäure DTPA (Europäische Patentschrift EP 71 564), daß die erfindungsgemäßen Polymer-Komplexe überraschenderweise über den gesamten durch die Indexziffern bestimmten Molekularbereich (5-2000 kD) stabiler sind als dieser.

Auch die Verträglichkeit der Polymer-Komplexe ist beispielsweise der organspezifischer monomerer Komplexe überlegen.

Überraschend hoch ist der Wert für die ein Maß der Bildgebung darstellende Größe der Relaxivität: Sie ist für die erfindungsgemäßen Polymer-Komplexe um den Faktor 3-1000 höher als für Gadolinium-DTPA. Hierdurch wird, um eine bestimmte Signalstärke bei der Bildgebung zu erhalten, eine entsprechend geringere molare Menge an Komplex im Vergleich zu den Monomeren benötigt.

Als weiterer wichtiger Vorteil der erfindungsgemäßen Polymer-Komplexe ist ihr Ausscheidungsverhalten anzuführen. Die je nach Anwendungszweck erwünschte Ausscheidungsgeschwindigkeit läßt sich dabei sehr gezielt und einfach über das zu wählende Molekulargewicht einstellen.

Die erfindungsgemäßen Polymer-Komplexe weisen eine überraschend hohe Gewebespezifität auf. So erhält man beispielsweise bereits wenige Minuten nach intravenöser Injektion einer N-Methylglucaminsalzlösung vom Gadolinium(III)-Komplex des Polyethylenimin-poly-DTPA (s. Beispiel 1) im Kernresonanzbild eine deutliche Kontrastverstärkung in peripherem Tumorgewebe, die längere Zeit anhält und einen deutlichen diagnostischen Zugewinn bringt.

Überraschenderweise können auch mit Hilfe der erfindungsgemäßen Polymer-Komplexe Blutgefäße ohne Anwendung spezieller Puls-Sequenzen in-vivo dargestellt werden, womit sie unter anderem als Perfusionsagentien Verwendung finden können.

Die Herstellung der erfindungsgemäßen Polymere erfolgt dadurch, daß man mit Amino-, Imino- und/oder 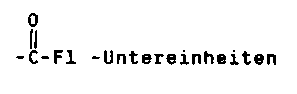
versehene Polymere, worin Fl für eine Fluchtgruppe steht, durch Alkylierung, Acylierung, Amidierung und/oder Hydrazinierung in Verbindungen umwandelt, die als Polymer-Einheiten Strukturen der allgemeinen Formel I' 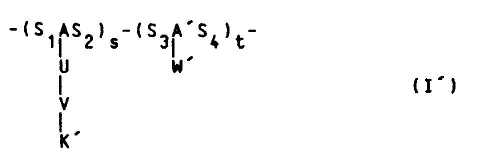
aufweisen, worin
A, A', U, V, S₁, S₂, S₃, S₄, s und t die oben genannte Bedeutung haben und K' Komplexbildner der allgemeinen Formeln I'A, I'B, I'C und I'D, die identisch mit den für IA, IB, IC und ID angegebenen Formeln sind, jedoch anstelle der Substituenten X und Z jeweils X' und Z' tragen, wobei
X' unabhängig voneinander für -COOH und
Z' für die Gruppe 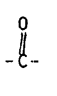
oder den Rest X' stehen, mit der Maßgabe, daß gewünschtenfalls ein Teil der COOH-Gruppen als Ester und/oder Amid vorliegt,
W' ein Wasserstoffatom, die Gruppe U_{w}-V_{w}-K'_{w}, wobei U_{w} und V_{w} die oben genannte Bedeutung haben und K'_{w} die für K genannte Bedeutung hat,
V'' oder die Gruppe 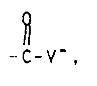
wobei V'' für den am Ende eine funktionelle Gruppe aufweisenden Rest V steht,
bedeuten,
diese dann in an sich bekannter Weise gewünschtenfalls mit mindestens einem Metalloxid oder Metallsalz eines Elements der Ordnungszahlen 21-29, 42, 44, oder 57-83 umsetzt und gewünschtenfalls K' und/oder W' durch Umwandlung mindestens einer der in K' oder W' enthaltenen -CO₂H -Gruppen in die gewünschte, am Ende eine funktionelle Gruppe aufweisende Alkylengruppe in K und W überführt, in beliebiger Reihenfolge durchgeführt werden können, und gegebenenfalls anschließend in den so erhaltenen Polymer-Komplexen noch vorhandene azide Wasserstoffatome durch Kationen anorganischer und/oder organischer Basen, Aminosäuren oder Aminosäureamide substituiert bzw. die entsprechenden Säuregruppen ganz oder teilweise in Ester oder Amide überführt.

Man geht dabei aus von Polymeren wie zum Beispiel Polyethylenimin, Polyethyleniminpolyessigsäurederivaten, Polyacrylester oder Polylysin, die gegebenenfalls 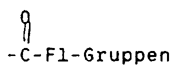
enthalten, worin Fl für eine Fluchtgruppe wie zum Beispiel Cl, Br, J, NH₂, OCH₃, OC₂H₅, OCH₂C₆H₅, OC₃H₇, Mesylat oder Tosylat steht.

Diese Verbindungen werden in an sich bekannter Weise amidiert, hydraziniert (Houben-Weyl, Methoden der organischen Chemie, Band VIII/3 Georg Thieme Verlag, Stuttgart (1952), 654 und 676), acyliert (J. March, Advanced Organic Chemistry, McGraw-Hill, 2nd ed.,(1977) 377-382) und/oder alkyliert (Houben-Weyl, Methoden der organischen Chemie, Band VI/3 Georg Thieme Verlag, Stuttgart (1965), 187).

Als Substrat zur Einführung der Komplexbildner-Einheiten K-V bzw. K_{w}-V_{w} dienen Verbindungen der allgemeinen Formeln I'A, I'B, I'C, I'D, I''AB und I''C 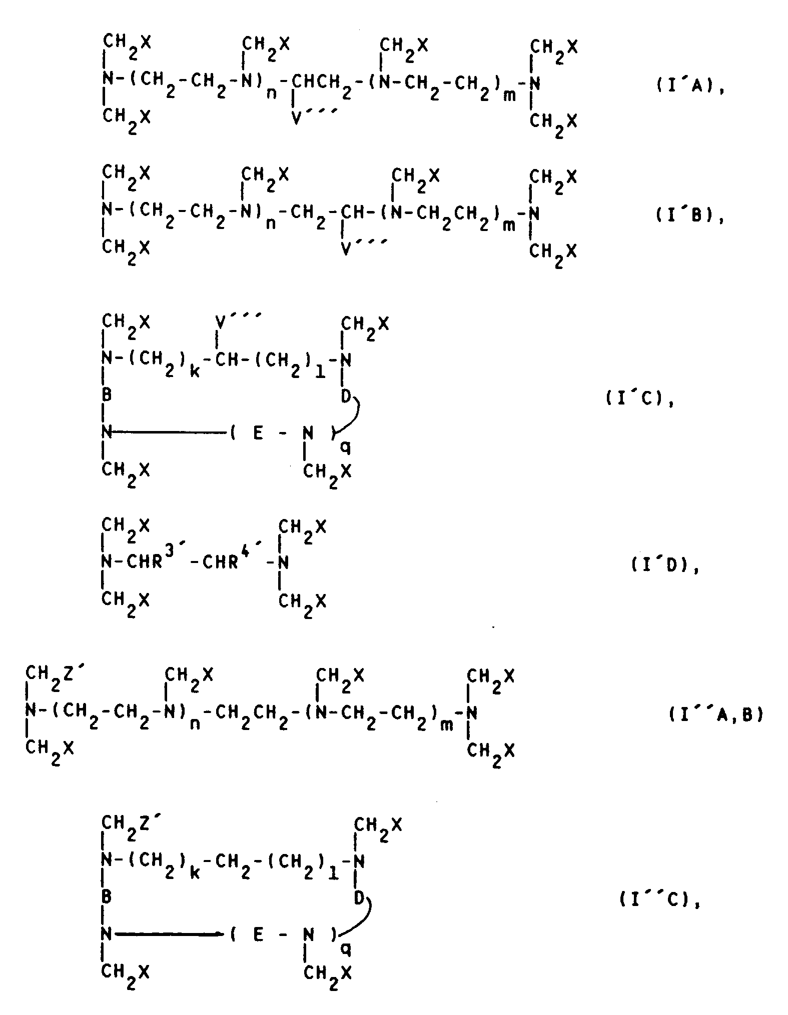
worin V''' für einen in V oder V'' umzuwandelnden Substituenten, R^{3'} und R^{4'} für R³ und R⁴, die den Substituenten V''' enthalten und Z' für eine aktivierte Carbonylgruppe stehen.

Als Beispiel für eine aktivierte Carbonylgruppe seien Anhydrid, p-Nitrophenylester und Säurechlorid genannt.

Die zur Einführung der Komplexbildner-Einheiten vorgenommene Alkylierung bzw. Acylierung wird mit Reagenzien durchgeführt, die den gewünschten K-V- bzw.-K_{w}-V_{w}-Substituenten (gebunden an eine Fluchtgruppe) enthalten oder aus denen der gewünschte Substituent, gegebenenfalls nach Modifikation durch Folgereaktion(en), durch die Reaktion generiert wird. Als Beispiele für die erstgenannten seien Halogenide, Mesylate, Tosylate und Anhydride genannt. Zur zweiten Gruppe gehören zum Beispiel Oxirane, Thiirane, Azirane, α,β-ungesättigte Carbonylverbindungen oder deren Vinyloge, Aldehyde, Ketone, Isothiocyanate und Isocyanate.

Als Beispiele für Folgereaktionen seien Esterspaltungen, Hydrierungen, Veresterungen, Oxidationen, Veretherungen und Alkylierungen genannt, die nach dem Fachmann bekannten Literaturverfahren durchgeführt werden.

Die als Edukte benötigten Verbindungen I' sind bekannt (z.B. Europäische Patentanmeldung Publikation Nr. 0154788) oder können aus den entsprechenden Polyaminen (wobei vorhandene funktionelle Gruppen gegebenenfalls geschützt sind) durch Alkylierung mit einem Ester der allgemeinen Formel II

HalCH₂COOY (II),

worin Hal für Chlor, Brom oder Jod und Y, für eine Säureschutzgruppe steht, hergestellt werden.

Die Umsetzung erfolgt in polaren aprotischen Lösungsmitteln wie zum Beispiel Dimethylformamid, Dimethylsulfoxid oder Hexamethylphosphorsäuretriamid in Gegenwart eines Säurefängers, wie zum Beispiel tertiäres Amin (zum Beispiel Triäthylamin, Trimethylamin, N,N-Dimethylaminopyridin, 1,5-Diazabicyclo[4.3.0], nonen-5(DBN), 1,5-Diazabicyclo[5.4.0]-undecen-5-(DBU), Alkali-, Erdalkalicarbonat oder -hydrogencarbonat (zum Beispiel Natrium-, Magnesium-, Calcium-, Barium-, Kalium- carbonat und -hydrogen-carbonat) bei Temperaturen zwischen -10°C und 120°C, vorzugsweise zwischen 0°C und 50°C.

Als Säureschutzgruppen Y kommen niedere Alkyl-, Aryl- und Aralkylgruppen, beispielsweise die Methyl-, Ethyl-, Propyl-, Butyl-, Phenyl-, Benzyl-, Diphenylmethyl, Triphenylmethyl-, bis-(p-Nitrophenyl)-methylgruppe, sowie Trialkylsilylgruppen in Frage.

Die Abspaltung der Schutzgruppen Y erfolgt nach den dem Fachmann bekannten Verfahren, beispielsweise durch Hydrolyse, alkalische Verseifung der Ester mit Alkali in wäßrig-alkoholischer Lösung bei Temperaturen von 0°C bis 50°C oder im Fall von tert.-Butylestern mit Hilfe von Trifluoressigsäure.

Die Herstellung der aktivierten Carbonylderivate I'' (z.B. gemischtes Anhydrid, N-Hydroxysuccinimidester, Acylimidazole, Trimethylsilylester) erfolgt nach literaturbekannten Methoden [Houben-Weyl, Methoden der organischen Chemie, Georg Thieme Verlag, Stuttgart, Band E 5(1985), 633; Org. React. 12,157(1962)] oder wird im experimentellen Teil beschrieben.

Die für die Herstellung der Polyamin-polysäuren der allgemeinen Formel I'A als Edukte benötigten entsprechenden Polyamine werden analog literaturbekannten Methoden (zum Beispiel Canad. Patent No. 1 178 951, Eur. I. Med. Chem.-Chim.Ther. 1985,20,509 und 1986, 21,333) hergestellt, indem man von Aminosäuren ausgeht, die in gegebenenfalls ethylenaminsubstituierte Amide (zum Beispiel mit N-(2-Aminoethyl)-carbaminsäurebenzylester) überführt und anschließend (gegebenenfalls nach Abspalten der Schutzgruppen) zu den gewünschten Aminen (vorzugsweise mit Diboran oder Lithiumaluminiumhydrid) reduziert werden.

Will man die Polyamin-Edukte für die Verbindungen der allgemeinen Formel I'B synthetisieren, so ist es notwendig, vor der Reduktion ein derartiges Amid durch Umsetzung mit zum Beispiel Ethyloxamat in einem polaren Lösungsmittel wie zum Beispiel Tetrahydrofuran, Dimethylsulfoxid oder Dimethoxyethan bei einer Temperatur zwischen 50°C und 250°C, vorzugsweise 70°C bis 150°C (gegebenenfalls in einem Druckbehälter) an der α-Aminogruppe zu substituieren, so daß man ein 3-Aza-2-oxo-glutarsäurediamid-Derivat als Zwischenprodukt erhält.

Die Herstellung der als Edukte für I'C bzw. I''C benötigten cyclischen Polyamine erfolgt durch Cyclisierung zweier Reaktanten, von denen (im Falle der Synthese von I'C) der eine V'''-substituiert ist.

Die Cyclisierung wird nach literaturbekannten Methoden, zum Beispiel Org. Synth. 58,86 (1978), Macrocyclic Polyether Syntheses, Springer Verlag, Berlin, Heidelberg, New York 1982, Coord. Chem. Rev. 3,3 (1968), Ann. Chem. 1976, 916, durchgeführt: einer der beiden Reaktanten trägt am Kettenende zwei Fluchtgruppen, der andere zwei Stickstoffatome, die nukleophil diese Fluchtgruppen verdrängen. Als Beispiel seien genannt die Umsetzung von endständigen, gegebenenfalls ein oder zwei Stickstoffatom(e) enthaltenden, Dibrom-, Dimesyloxy-, Ditosyloxy- oder Dialkoxycarbonylalkylenverbindungen mit endständigen, gegebenenfalls ein oder zwei zusätzliche Stickstoffatom(e) in der Alkylenkette enthaltenden Diazaalkylenverbindungen, von denen (im Falle der Synthese von I'C) einer der beiden Reaktanten V'''substituiert ist.

Die Stickstoffatome sind gegebenenfalls geschützt, zum Beispiel als Tosylate, und werden vor der nachfolgenden Alkylierungsreaktion nach literaturbekannten Verfahren freigesetzt.

Werden Diester in die Cyclisierungsreaktion eingesetzt, so müssen die so erhaltenen Diketoverbindungen nach dem Fachmann bekannten Verfahren, zum Beispiel mit Diboran, reduziert werden.
Als Substituenten V''', der in V oder in den am Ende eine für eine Bindung an ein Makro- oder Biomolekül geeignete funktionelle Gruppe aufweisenden Substituenten V'' überführt werden kann, sind unter anderem Hydroxy-und Nitrobenzyl-, Hydroxy-und Carboxyalkyl- sowie Thioalkylreste mit bis zu 20 Kohlenstoffatomen geeignet. Sie werden nach dem Fachmann bekannten Literaturverfahren (Chem. Pharm. Bull. 33,674 (1985), Compendium of Org. Synthesis Vol. 1-5, Wiley and Sons, Inc., Houben-Weyl. Methoden der organischen Chemie, Band VIII, Georg Thieme Verlag, Stuttgart, J. Biochem. 92, 1413,1982) in die gewünschten Substituenten ( zum Beispiel mit der Amino-, Hydrazino-, Hydrazinocarbonyl-, Epoxid-, Anhydrid-, Methacryloylhydrazinocarbonyl-, Maleimidamidocarbonyl-, Halogeno-, Halogenocarbonyl-, Mercapto-, Isothiocyanatgruppe als funktioneller Gruppe) umgewandelt, wobei im Falle des Nitrobenzylrestes zunächst eine katalytische Hydrierung (zum Beispiel nach P.N. Rylander, Catalytic Hydrogenation over Platinum Metals, Academic Press 1967) zum Aminobenzylderivat vorgenommen werden muß.
Beispiele für die Umwandlung von an aromatische oder aliphatische Reste gebundenen Hydroxy- oder Aminogruppen sind die in wasserfreien, aprotischen Lösungsmitteln wie Tetrahydrofuran, Dimethoxyethan oder Dimethylsulfoxid in Gegenwart eines Säurefängers wie zum Beispiel Natriumhydroxid, Natriumhydrid oder Alkali oder Erdalkalicarbonaten wie zum Beispiel Natrium-, Magnesium-, Kalium-,Calciumcarbonat bei Temperaturen zwischen 0 °C und dem Siedepunkt des jeweiligen Lösungsmittels, vorzugsweise jedoch zwischen 20 °C und 60 °C, durchgeführten Umsetzungen mit einem Substrat der allgemeinen Formel III

Nf-L-Fu (III),

worin Nf für ein Nucleofug wie z.B. Cl, Br, J, CH₃C₆H₄SO₃, oder CF₃SO₃, L für einen aliphatischen, aromatischen, arylaliphatischen, verzweigten, geradkettigen oder cyclischen Kohlenwasserstoffrest mit bis zu 20 Kohlenstoffatomen und Fu für die gewünschte endständige funktionelle Gruppe, gegebenenfalls in geschützter Form, stehen (DE-OS 34 17 413).

Als Beispiele für Verbindungen der allgemeinen Formel III seien genannt
Br(CH₂)₂NH₂, Br(CH₂)₃OH, BrCH₂COOCH₃, BrCH₂CO₂^{t}Bu, ClCH₂CONHNH₂, Br(CH₂)₄CO₂C₂H₅, BrCH₂COBr, BrCH₂CONH₂, ClCH₂COOC₂H₅, BrCH₂CONHNH₂, 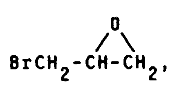
CF₃SO₃(CH₂)₃Br, BrCH₂C≡CH, BrCH₂CH=CH₂.

Umwandlungen von Carboxy-Gruppen können zum Beispiel nach der Carbodiimid-Methode (Fieser, Reagents for Organic Syntheses 10,142), über ein gemischtes Anhydrid [Org. Prep. Proc. Int. 7,215(1975)] oder über einen aktivierten Ester (Adv. Org. Chem. Part B, 472) durchgeführt werden.

Die Herstellung der als Ausgangssubstanzen für die Cyclisierung benötigten Amine erfolgt analog literaturbekannten Methoden.

Ausgehend von einer N-geschützten Aminosäure erhält man durch Umsetzung mit einem partiell geschützten Diamin (zum Beispiel nach der Carbodiimidmethode), Abspaltung der Schutzgruppen und Diboranreduktion ein Triamin.

Die Umsetzung eines aus Aminosäuren erhältlichen Diamins (Eur. J. Med. Chem.-Chim. Ther. 21,333 (1986)) mit der doppelten molaren Menge einer N-geschützten ω-Aminosäure liefert ein Tetramin nach geeigneter Aufarbeitung.

In beiden Fällen ist die Anzahl der Kohlenstoffatome zwischen den N-Atomen durch die Art der als Kopplungspartner eingesetzten Diamine bzw. Aminosäuren bestimmbar.

Ein Teil der über die Komplexbildner-Einheiten K bzw. K_{w} eingeführten Säuregruppen der so erhaltenen Polymer-Verbindungen kann gewünschtenfalls nach dem Fachmann bekannten Verfahren weiter funktionalisiert werden, zum Beispiel durch Überführung in Ester-, Amid-, Hydrazid-, Maleimido- oder andere Gruppen, die zur Kopplung an Bio- oder Makromoleküle geeignet sind.

Die Herstellung der erfindungsgemäßen Metallkomplexe erfolgt in der Weise, wie sie in der Patentschrift DE-OS 34.01.052 offenbart worden ist, indem man das Metalloxid oder ein Metallsalz (beispielsweise das Nitrat, Acetat, Carbonat, Chlorid oder Sulfat) des Elements der Ordnungszahlen 21-29, 42, 44, 57-83 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene azide Wasserstoffatome der Säure- bzw. Phenolgruppen durch Kationen anorganischer und/oder organischer Basen oder Aminosäuren substituiert.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl-und N,N-Dimethylglucamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Eine andere Möglichkeit, zu neutralen Komplexverbindungen zu kommen, besteht darin, die verbleibender Säuregruppen im Komplex ganz oder teilweise in zum Beispiel Ester oder Amide zu überführen. Dies kann durch nachträgliche Reaktion am fertigen Polymer-Komplex geschehen (z.B. durch erschöpfende Umsetzung der freien Carboxy-Gruppen mit Dimethylsulfat) wie auch durch Verwendung eines geeignet derivatisierten Substrats zur Einführung der Komplexbildner-Einheiten K-V bzw. K_{w}-V_{w} der allgemeinen Formeln I'A, I'B, I'C, I'D, I''AB, I''C (z. B. N³-(2,6-Dioxomorpholinomethyl)-N⁶-(ethoxycarbonylmethyl)-3,6-diazaoctandisäure)

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel Diethylentriaminpentaessigsäure) oder, falls erforderlich, Elektrolyte wie zum Beispiel Natriumchlorid oder, falls erforderlich, Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)}, Myrj^{(R)} und/oder Aromastuff(en) zur Geschmackskorrektur (z.B. ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 1 µMol - 1 Mol/l des Metalls in Form seines Komplexsalzes und werden in der Regel in Mengen von 0,001-5 mMol Metall/kg Körpergewicht dosiert. Sie sind zur enteralen und parenteralen Applikation bestimmt.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit der erfindungsgemäßen Mittel erlaubt es, hochkonzentrierte Lösungen herzustellen, damit die Volumenbelastung des Kreislaufs in vertretbaren Grenzen zu halten und die Verdünnung durch Körperflüssigkeit auszugleichen, das heißt NMR-Diagnostika müssen 100-1000-fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht konvalent gebundenen - an sich giftigen -Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,001 - 5 mMol Metall/kg Körpergewicht, vorzugsweise 0,005 - 0,5 mMol Metall/kg Körpergewicht, dosiert. Details der Anwendung werden zum Beispiel in H. J. Weinmann et al., Am. J. of Roentgenology 142, 619 (1984) diskutiert.
Besonders niedrige Dosierungen (unter 1 mg/kg Körpergewicht) von organspezifischen NMR-Diagnostika sind zum Beispiel zum Nachweis von Tumoren und von Herzinfarkt einsetzbar.
Ferner können die erfindungsgemäßen Komplexverbindungen vorteilhaft als Shift- und als Suszeptibilitäts- Reagenzien verwendet werden.

Die erfindungsgemäßen Mittel sind ebenfalls als Röntgenkontrastmittel geeignet, wobei besonders hervorzuheben ist, daß sie auch im Vergleich zu den bisher ge bräuchlichen jodhaltigen Kontrastmitteln eine für die Diagnostik wesentlich günstigeren Pharmakokinetik erkennen lassen. Besonders wertvoll sind sie weiterhin wegen der günstigen Absorptionseigenschaften in Bereichen höherer Röhrenspannungen für digitale Substraktionstechniken.
Im allgemeinen werden die erfindungsgemäßen Mittel für die Anwendung als Röntgenkontrastmittel in Analogie zu zum Beispiel Meglumin-Diatrizoat in Mengen von 0,1 -5 mMol Metall/kg Körpergewicht, vorzugsweise 0,25 - 1 mMol Metall/kg Körpergewicht, dosiert.

Details der Anwendung von Röntgenkontrastmitteln werden zum Beispiel in Barke, Röntgenkontrastmittel, G. Thieme, Leipzig (1970) und P. Thurn, E. Bücheler - "Einführung in die Röntgendiagnostik", G. Thieme, Stuttgart, New York (1977) diskutiert.

Die erfindungsgemäßen Mittel sind - da ihre akustische Impedanz höher ist als die von Körperflüssigkeiten und Geweben - auch als Kontrastmittel für die Ultraschalldiagnostik geeignet, insbesondere in Form von Suspensionen. Sie werden im allgemeinen in Mengen von 0,1 bis 5 mMol/kg Körpergewicht, vorzugsweise von 0,25 bis 1 mMol/kg Körpergewicht dosiert.

Details der Anwendung von Ultraschalldiagnostika werden zum Beispiel in T.B. Tyler et al., Ultrasonic Imaging 3.323 (1981), J.I. Haft, "Clinical Echokardiography", Futura, Mount Kisco, New York 1978 und G. Stefan "Echokardiographie" G. Thieme Stuttgart/New York 1981, beschrieben.

Insgesamt ist es gelungen, neue Polymer-Komplexe zu synthetisieren, die neue Möglichkeiten in der diagnostischen und therapeutischen Medizin erschließen. Vor allem die Entwicklung neuartiger bildgebender Verfahren in der medizinischen Diagnostik läßt diese Entwicklung wünschenswert erscheinen.

Die nachfolgenden Beispiele dienen zur näheren Erläuterung des Erfindungsgegenstandes.

### BEISPIEL 1

a) O-Benzyl-N-trifluoracetyltyrosin
112,5 g (0,41 mMol) O-Benzyltyrosin werden in 1 Liter trockenem Methanol suspendiert und bei Raumtemperatur mit 58,9 ml (0,42 Mol) Triethylamin versetzt. Nach Zugabe von 67 ml (0,53 Mol) Trifluoressigsäuremethylester wird 130 Stunden bei Raumtemperatur unter Wasserausschluß gerührt. Man trennt von unumgesetztem Ausgangsmaterial ab und entfernt flüchtige Komponenten durch Schütteln mit Essigester/wäßriger Salzsäure. Die Essigesterphase wird mit Aktivkohle entfärbt. Nach Verdampfen der Lösungsmittel erhält man 120,7 g (80% der Theorie) farbloser Kristalle.
Schmelzpunkt: 149-150°C

| | | | | | | |
|---|---|---|---|---|---|---|
| Analyse: | Ber.: | C 58,85 | H 4,39 | N 3,81 | O 17,42 | F 15,51 |
| | Gef.: | C 58,78 | H 4,29 | N 3,79 | | F 15,57 |

b) O-Benzyl-N-trifluoracetyltyrosin-(2-carbobenzoxyaminoethylen)-amid
18,5 g (50,4 mMol) O-Benzyl-N-trifluoracetyltyrosin (Beispiel 1a) werden in 200 ml trockenem Tetrahydrofuran gelöst, mit 7 ml Triethylamin versetzt und dann tropfenweise 4,8 ml (50,8 mMol) Chlorameisensäureethylester zugefügt, wobei die Temperatur auf unter -10°C gehalten wird. Nach Beendigung der Zugabe wird 30 Minuten bei dieser Temperatur gerührt, nochmals mit der gleichen Menge vorgekühltem Triethylamin versetzt und eine eiskalte Lösung von 11,6 g (50,4 mMol) N-(2-Aminoethyl)-carbaminsäurebenzylester-Hydrochlorid in 100 ml Dimethylformamid zugetropft. Man rührt noch 30 Minuten bei -10°C, läßt dann unter Rühren auf Raumtemperatur kommen und erwärmt dann 10 Minuten auf 30°C. Danach entfernt man das Lösungsmittel am Rotationsverdampfer und gießt auf 750 ml Eiswasser. Das Kristallisat wird abgesaugt, mit Eiswasser gewaschen und getrocknet. Die Ausbeute beträgt 26,9 g (94% der Theorie).
Schmelzpunkt: 189-190°C

| | | | | | | |
|---|---|---|---|---|---|---|
| Analyse: | Ber.: | C 61,87 | H 5,19 | N 7.73 | O 14,71 | F 10,48 |
| | Gef.: | C 61,90 | H 5,08 | N 7,77 | | F 10,43 |

c) O-Benzyltyrosin-(2-carbobenzoxyaminoethylen)-amid
25,9 g (47,8 mMol) O-Benzyl-N-trifluoracetyltyrosin-(2-carbobenzoxyaminoethylen)-amid (Beispiel 1b) werden in 300 ml Ethanol suspendiert und portionsweise mit 7,2 g (191 mMol) Natriumborhydrid versetzt. Nach Rühren über Nacht bei Raumtemperatur wird mit 50 ml Aceton versetzt, vom Lösungsmittel befreit, mit 500 ml Wasser versetzt und mehrmals mit Essigester extrahiert, Die organische Phase liefert nach Trocknen und Einengen 18,8 g (88% der Theorie) weißer Kristalle vom Schmelzpunkt 145°C.

| | | | | | |
|---|---|---|---|---|---|
| Analyse: | Ber.: | C 69,77 | H 6,53 | N 9,38 | O 14,29 |
| | Gef.: | C 69,79 | H 6,53 | N 9,35 | |

d) Tyrosin-(2-aminoethylen)-amid
42,3 g (94,6 mMol) der Verbindung nach Beispiel 1c löst man in 1,1 Liter Methanol, fügt 2 g 10% Palladium-Kohle zu und hydriert unter Rühren, bis keine weitere Wasserstoffaufnahme mehr erfolgt. Der Katalysator wird abfiltriert und das Losungsmittel abgedampft. Man löst in der Hitze in Methanol und fällt mit Ether. Man erhält 17 g (86% der Theorie) farblose Kristalle.
Schmelzpunkt: 138-141°C

| | | | | | |
|---|---|---|---|---|---|
| Analyse: | Ber.: | C 59,17 | H 7,67 | N 18,81 | O 14,33 |
| | Gef.: | C 59,23 | H 7,51 | N 18,90 | |

e) 3-Aza-1-(4-hydroxybenzyl)-pentan-1,5-diamin . Trihydrochlorid
6,55 g (29,3 mMol) der Verbindung nach Beispiel 1d werden in 130 ml trockenem Tetrahydrofuran suspendiert und ein langsamer Strom von Diboran (aus 5,8 g Natriumborhydrid in 75 ml Diethylenglykoldimethylether und 54 ml Bortrifluorid-Etherat-Komplex) mit trockenem Stickstoff unter stetigem Rühren durch die Losung getrieben. Man rührt über Macht bei 60°C, tropft danach bei 20°C 30 ml Methanol zu und leitet unter Eiskühlung Chlorwasserstoff ein. Man kocht danach kurz auf und saugt ab. Das Trihydrochlorid wird in Form farbloser Kristalle (8,04 g; 86% der Theorie) erhalten.
Schmelzpunkt: 250°C (Zersetzung)

| | | | | | | |
|---|---|---|---|---|---|---|
| Analyse: | Ber.: | C 41,45 | H 6,95 | N 13,18 | O 5,02 | Cl 33,37 |
| | Gef.: | C 41,37 | H 6,89 | N 13,14 | | Cl 33,51 |

f) 3,6,9-Triaza-4-(4-hydroxybenzyl)-3,6,9-tris-(tert.-butoxycarbonylmethyl)-undecandisäure-bis-(tert.-butyl)-diester
2,07 g (6,5 mMol) der Verbindung nach Beispiel 1e und 5,2 g Natriumhydrogencarbonat werden in 60 ml Dimethylformamid (getrocknet über Natriumhydrid) vorgelegt und bei 35°C 6,34 g (82,2 mMol) Bromessigsäure-tert.-butylester in 30 ml Dimethylformamid zugetropft. Man rührt noch weitere 2,5 Stunden bei 35°C, wonach kein Ausgangsprodukt mehr dünnschichtchromatographisch nachzuweisen ist. Man filtriert von ausgefallenem Natriumbromid ab und engt das Filtrat ein. Der Rückstand wird mit Wasser versetzt und mehrmals mit Ether extrahiert. Nach Trocknen und Einengen wird der Etherextrakt über eine Kieselgelsäule von unumgesetztem Bromessigsäure-tert.-butylester gereinigt. Man erhält 3,54 g (68,8% der Theorie) eines farblosen Öls.

| | | | | | |
|---|---|---|---|---|---|
| Analyse: | Ber.: | C 63,13 | H 8,91 | N 5,38 | O 22,56 |
| | Gef.: | C 63,21 | H 8,90 | N 5,42 | |

g) 3,6,9-Triaza-4-(4-benzyloxycarbonylmethoxybenzyl)-3,6,9-tris-(tert.-butoxycarbonylmethyl)-undecandisäure-bis-(tert.-butyl)-diester
1,98 g (2,54 mMol) der Verbindung noch Beispiel 1f werden mit 70 mg Natriumhydrid (80% in Paraffin) (2,5 mMol) in 30 ml trockenem Tetrahydrofuran unter Rühren langsam zusammengegeben und dann bei Raumtemperatur 0,54 g Bromessigsäurebenzylester (2,54 mMol) in 20 ml trockenem Tetrahydrofuran zugetropft. Nach Rühren über Nacht saugt man von ausgefallenem Natriumbromid ab, engt ein, nimmt in Diethylether auf und entfernt die übrigen anorganischen Bestandteile durch Waschen mit Wasser. Nach Trocknen über Magnesiumsulfat wird vom Lösungsmittel befreit und über eine Kieselgelsäule gereinigt. Man erhält 1,35 g (1,45 mMol) eines farblosen Sirups (62% der Theorie).

| | | | | | |
|---|---|---|---|---|---|
| Analyse: | Ber.: | C 64,70 | H 8,36 | N 4,52 | O 22,4 |
| | Gef.: | C 64,91 | H 8,31 | N 4,55 | |

h) 3,6,9-Triaza-3,6,9-tris-(tert.-butoxycarbonylmethyl)-4-(4-carboxymethylbenzyl)-undecandisäure-bis-(tert.-butyl)-diester
7,83 g (8,43 mMol) der Verbindung nach Beispiel 1g werden in 70 ml trockenem Tetrahydrofuran gelöst und in Gegenwart von 1,4 g 10% Palladium-Kohle hydriert, bis keine weitere Wasserstoffaufnahme stattfindet. Nach Absaugen wird das Lösungsmittel am Rotationsverdampfer entfernt und die Substanz bei 0,01 Torr getrocknet. Man erhält 4,2 g eines farblosen Öls (Ausbeute 74% der Theorie).

| | | | | | |
|---|---|---|---|---|---|
| Analyse: | Ber.: | C 61,62 | H 8,53 | N 5,01 | O 24,81 |
| | Gef.: | C 61,73 | H 8,53 | N 5,10 | |

i) Poly-〈4-[2,6-di-(bis-(carboxymethyl)-amino)-4-(carboxymethyl)-aza-hexyl]-phenoxyacetyl〉-polyethylenimin
7,16 g (8,5 mMol) der Verbindung nach Beispiel 1h werden in 80 ml Tetrahydrofuran mit 1,28 g (9,4 mMol) Chlorameisensäureisobutylester und 1,9 g (18,8 mMol) Triethylamin, jeweils in 20 ml Tetrahydrofuran gelöst, bei 0°C unter Rühren versetzt.
Nach einer Stunde fügt man unter Beibehaltung der Kühlung eine Lösung von 533,2 mg (entspricht 12,4 mMol monomere Untereinheit) Polyethylenimin (Polymin wasserfrei^{(R)}) in Wasser hinzu und läßt unter Rühren auf Raumtemperatur kommen. Ausgefallenes Salz wird abfiltriert, das Lösungsmittel abgedampft und der Rückstand durch Erwärmen mit 150 ml Ameisensäure gelöst. Nach 3 Stunden bei 60°C gießt man in 2 Liter Eiswasser und dialysiert. Nach Gefriertrocknung bleiben 4,35 g weißes, feinkristallines Pulver zurück.

| | | | |
|---|---|---|---|
| Analyse: | C 51,75 | H 6,03 | N 10,54 |

j) Gadolinium-Komplex
2,36 g des polymeren Komplexbildners von Beispiel 1i werden in 30 ml Wasser unter Zusatz von wenigen Tropfen verdünnter Ammoniaklösung gelöst und mit 3,92 ml einer 1 m Lösung von Gadoliniumacetat in 0,1 m Ammonacetatpuffer pH 4,5 versetzt, wobei der pH-Wert durch Zugabe von verdünnter Ammoniaklösung auf einem Wert über 5,5 gehalten wird. Man dialysiert und unterwirft einer Gefriertrocknung. Es bleiben 2,96 g eines weißen kristallinen Pulvers zurück.

| | | | | |
|---|---|---|---|---|
| Analyse: | C 41,26 | H 4,41 | N 8,39 | Gd 20,67 |

### Natrium-Salz des Gadolinium-Komplexes

1,8 g des Polymer-Komplexes werden in 20 ml Wasser unter Rühren und Zugabe von 1n Natronlauge gelöst, wobei der pH-Wert 7,5 nicht übersteigen darf (Verbrauch 4,73 ml). Nach Gefriertrocknung liegt das Natriumsalz in Form feiner weißer Kristalle vor. Die Ausbeute beträgt 1,89 g.

| | | | | | |
|---|---|---|---|---|---|
| Analyse: | C 39,0 | H 3,92 | N 7,94 | Na 5,71 | Gd 19,54 |

### N-Methylglucaminsalz des Komplexes

Man legt 2,16 g des Gadoliniumkomplexes in 50 ml Wasser vor und titriert mit einer 1 m wässrigen Lösung N-Methylglucamin auf einen pH-Wert zwischen 7,3 und 7,4. Der Verbrauch beträgt 6,2 ml. Nach Gefriertrocknung liegen 3,37 g farblose Kristalle vor.

| | | | | |
|---|---|---|---|---|
| Analyse: | C 42,62 | H 4,21 | N 8,12 | Gd 13,9 |

### BEISPIEL 2

### Poly-〈4-[2,6-di-(bis-(carboxymethyl)-amino)-4-(carboxymethyl)-aza-hexyl]-phenoxyacetyl〉-polyethylenimin-poly-[2-(maleimido)-ethylenamid]

3,81 g des nach Beispiel 1 erhaltenen Komplexbildners werden in 8 ml Wasser unter Zusatz von 110 mg Kaliumcarbonat gelost und mit 150 ml Dimethylsulfoxid (DMSO) rasch aufgefüllt. Man versetzt dann mit 200 mg Dimethylsulfat in 1 ml DMSO und erwärmt 30 Minuten auf 60°C. Danach fügt man 1,3 g (9,3 mMol) 2-(Maleimido)-ethylamin zu, erwärmt weiter 30 Minuten auf 80°C und gießt in die dreifache Menge Wasser. Nach Dialyse und Gefriertrocknung liegen 4,0 g weiße kristalline Substanz vor. UV-spektroskopisch wird ein Gehalt von 78.9 mg (0,8 mMol) Maleimid/ g Substanz bestimmt ( =280 nm).

| | | | |
|---|---|---|---|
| Analyse: | C 51,75 | H 6,03 | N 10,56 |

Der Gadolinium-Komplex und dessen Salze werden wie in Beispiel 1j beschrieben, erhalten.
**Gadolinium-Komplex**

| | | | |
|---|---|---|---|
| C 41,28 | H 4,41 | N 8,42 | Gd 20,63 |

**Natrium-Salz**

| | | | | |
|---|---|---|---|---|
| C 39,02 | H 3,92 | N 7,96 | Na 5,7 | Gd 19,40 |

**N-Methylglucamin-Salz**

| | | | |
|---|---|---|---|
| C 42,63 | H 4,21 | N 8,14 | Gd 13,88 |

### Alternative Methode

Führt man die Reaktion nach der obigen Vorschrift aus, verwendet jedoch an Stelle des Komplexbildners den fertigen Gd-Komplex aus Beispiel 1j, so erhält man nach analoger Aufarbeitung ebenfalls den derivatisierten Komplex. Aus 7,85 g des Gadolinium-Komplexes mit 20,6 Gew% Gd werden 7,78 g des mit Amidoethylen-(maleimido)-Gruppen belegten Komplexes erhalten. Das Produkt zeigt einen Gadoliniumgehalt von 20,1 Gew% und 0,78 mg Maleimid pro Gramm Substanz.

### BEISPIEL 3

### Poly-〈4-[2,6-di-(bis-(carboxymethyl)-amino)-4-(carboxymethyl)-aza-hexyl]-phenoxyacetyl〉-polyethylenimin-poly-hydrazid

Wie für Beispiel 2 beschrieben, erhält man das Hydrazid des Komplexbildners, wenn man statt 2-(Maleimido)-ethylamin eine äquivalente Menge an Hydrazin-Hydrat zufügt. Der Hydrazingehalt wird colorimetrisch (p-Dimethylaminobenzaldehyd) zu 22,8 mg (0,8 mMol) Hydrazin/ g Substanz ermittelt. Aus 4,02 g Komplexbildner erhält man 3,87 g Hydrazid als weiße, kristalline Substanz.

| | | | |
|---|---|---|---|
| Analyse: | C 51,68 | H 6,07 | N 10,61 |

Der Gadolinium-Komplex und dessen Salze werden wie in Beispiel 1j beschrieben. erhalten.
**Gadolinium-Komplex**

| | | | |
|---|---|---|---|
| C 41,21 | H 4,44 | N 8,46 | Gd 20,65 |

**Natrium-Salz**

| | | | | |
|---|---|---|---|---|
| C 38,96 | H 3,95 | N 8,00 | Na 5,70 | Gd 19,52 |

**N-Methylglucamin-Salz**

| | | | |
|---|---|---|---|
| C 42,58 | H 4,23 | N 8,17 | Gd 13,89 |

### BEISPIEL 4

### Poly-〈4-[2,6-di-(bis-(carboxymethyl)-amino)-4-(carboxymethyl)-aza-hexyl]-phenoxyacetyl〉-polyethylenimin-poly-[10-(hydrazinocarbonyl)-decylamid]

Verwendet man als Stickstoffbase in dem für Beispiel 2 beschriebenen Verfahren das 11-Amino-undecansäure-(tert.-butoxycarbonyl)-hydrazid anstelle von 2-(Maleimido)-ethylamin, so erhält man das Poly-〈4-[2,6-di-(bis-(carboxymethyl)-amino)-4-(carboxymethyl)-aza-hexyl]-phenoxyacetyl〉-polyethylenimin-poly-[10-((tert.butoxycarbonyl)-hydrazinocarbonyl)decylamid, aus dem mit Ameisensäure, wie für Beispiel 1i beschrieben, die tert.-Butoxycarbonylschutzgruppen entfernt werden. Man erhält so aus 6,30 g Komplexbildner 6,41 g weiße Festsubstanz. Der Hydrazingehalt wird colorimetrisch zu 18,7 mg (0,67 mMol) pro Gramm Substanz bestimmt.

| | | | |
|---|---|---|---|
| Analyse: | C 51,81 | H 6,07 | N 10,67 |

Der Gadolinium-Komplex und dessen Salze werden wie in Beispiel 1j beschrieben, erhalten.
**Gadolinium-Komplex**

| | | | |
|---|---|---|---|
| C 41,38 | H 4,45 | N 8,52 | Gd 20,52 |

**Natrium-Salz**

| | | | | |
|---|---|---|---|---|
| C 39,13 | H 3,96 | N 8,06 | Na 5,67 | Gd 19,40 |

**N-Methylglucamin-Salz**

| | | | |
|---|---|---|---|
| C 42,69 | H 4,24 | N 8,21 | Gd 13,83 |

### BEISPIEL 5

a) 3,6,9-Triaza-3,6,9-tris-(tert.-butoxycarbonylmethyl)-4-(4-oxiranylmethoxy benzyl)-undecandisäure-bis-(tert.-butyl)-diester
16,35 g (21,0 mMol) 3,6,9-Triaza-4-(4-hydroxybenzyl)-3,6,9-tris-(tert.-butoxycarbonylmethyl)-undecandisäure-bis-(tert.-butyl)-diester (Beispiel 1f) werden mit 630 mg (21 mMol) Natriumhydrid (80% in Paraffin) in 300 ml Toluol unter Rühren gelöst und bei 40°C tropfenweise mit einer Lösung von 1,95 g (21 mMol) Epichlorhydrin in 20 ml Toluol versetzt. Nach einer Stunde wird vorsichtig mit 100 ml Wasser versetzt. Nach Schütteln werden die Phasen getrennt. Anschließend wird die organische Phase nach Trocknen eingeengt. Nach chromatographischer Reinigung liegen 15,4 g (88% der Theorie) farbloses Öl vor.

| Analyse: | | | | |
|---|---|---|---|---|
| Ber.: | C 63,20 | H 8,80 | N 5,02 | O 22,96 |
| Gef.: | C 63,35 | H 8,76 | N 5,09 | |

b) Poly-〈3-[4-[2,6-di-(bis-(carboxymethyl)-amino)-4-(carboxymethyl)-aza-hexyl]-phenoxy]-2-hydroxypropyl〉-polyethylenimin
12,3 g (14,7 mMol) 3,6,9-Triaza-3,6,9-tris-(tert.-butoxycarbonylmethyl)-4-(4-oxiranylmethoxybenzyl)-undecandisäure-bis-(tert.-butyl)-diester werden in 150 ml Methanol mit 6,30 mg (14,65 mMol monomere Untereinheiten) Polyethylenimin gelöst, wobei zunächst die Temperatur unter 5°C gehalten wird. Man läßt innerhalb einer Stunde auf Raumtemperatrur aufwärmen und erhitzt dann noch eine Stunde auf 60°C. Nach Abziehen des Lösungsmittels erwärmt man in 100 ml Ameisensäure 3 Stunden auf 60°C, gießt in 2 Liter Wasser und dialysiert. Nach Gefriertrocknung liegen 6,22 g eines feinkristallinen weißen Pulvers vor, das oberhalb 150°C zu sintern beginnt.

| | | | |
|---|---|---|---|
| Analyse: | C 52,17 | H 6,41 | N 9,42 |

Der Gadolinium-Komplex und dessen Salze werden wie in Beispiel 1j beschrieben, erhalten.
Gadolinium-Komplex

| | | | |
|---|---|---|---|
| C 41,51 | H 4,70 | N 7,50 | Gd 20,83 |

Natrium-Salz

| | | | | |
|---|---|---|---|---|
| C 39,22 | H 4,19 | N 7,08 | Na 5,75 | Gd 19,69 |

N-Methylglucamin-Salz

| | | | |
|---|---|---|---|
| C 42,79 | H 4,40 | N 7,52 | Gd 13,97 |

### BEISPIEL 6

### Poly-〈3-[4-[2,6-di-(bis-(carboxymethyl)-amino)-4-(carboxymethyl)-aza-hexyl]-phenoxy]-2-hydroxypropyl〉-polyethylenimin-poly-[2-maleimido)-ethylenamid

Analog der für Beispiel 2 angegebenen Vorschrift werden 2,9 g des nach Beispiel 5 erhaltenen Komplexbildners umgesetzt. Man erhält 2,8 g der Titelverbindung.
Maleimidgehalt (UV) 0,31 Gew%

| | | | |
|---|---|---|---|
| Analyse: | C 52,17 | H 6,41 | N 9,45 |

Der Gadolinium-Komplex und dessen Salze werden wie in Beispiel 1j beschrieben, erhalten.
**Gadolinium-Komplex**

| | | | |
|---|---|---|---|
| C 41,53 | H 4,70 | N 7,52 | Gd 20,79 |

**Natrium-Salz**

| | | | | |
|---|---|---|---|---|
| C 39,25 | H 4,19 | N 7,11 | Na 5,74 | Gd 19,65 |

**N-Methylglucamin-Salz**

| | | | |
|---|---|---|---|
| C 42,80 | H 4,41 | N 7,59 | Gd 13,96 |

### BEISPIEL 7

### Poly-〈3-[4-[2,6-di-(bis-(carboxymethyl)-amino)-4-(carboxymethyl)-aza-hexyl]-phenoxy]-2-hydroxypropyl〉-polyethylenimin-poly-hydrazid

Analog zur Vorschrift für Beispiel 3 wird, ausgehend von 3,5 g des nach Beispiel 5 erhaltenen Komplexbildners, 3,4 g des Hydrazids erhalten. Der Hydrazingehalt beträgt 14,0 mg pro Gramm Substanz.

| | | | |
|---|---|---|---|
| Analyse: | C 52,06 | H 6,47 | N 9,54 |

Der Gadolinium-Komplex und dessen Salze werden wie in Beispiel 1j beschrieben, erhalten.
**Gadolinium-Komplex**

| | | | |
|---|---|---|---|
| C 41,44 | H 4,75 | N 7,60 | Gd 20,80 |

**Natrium-Salz**

| | | | | |
|---|---|---|---|---|
| C 39,16 | H 4,24 | N 7,18 | Na 5,74 | Gd 19,65 |

**N-Methylglucamin-Salz**

| | | | |
|---|---|---|---|
| C 42,74 | H 4,44 | N 7,58 | Gd 13,96 |

### BEISPIEL 8

### Poly-〈3-[4-[2,6-di-(bis-(carboxymethyl)-amino)-4-(carboxymethyl)-aza-hexyl]-phenoxy]-2-hydroxypropyl〉-polyethylenimin-poly-[10-(hydrazinocarbonyl)-decylamid]

Analog der für Beispiel 4 angegebenen Vorschrift werden 4,5 g des nach Beispiel 5 erhaltenen Komplexbildners umgesetzt. Man erhält 4,6 g der Titelverbindung.

| | | | |
|---|---|---|---|
| Analyse: | C 52,22 | H 6,44 | N 9,54 |

Der Gadolinium-Komplex und dessen Salze werden wie in Beispiel 1j beschrieben, erhalten.
**Gadolinium-Komplex**

| | | | |
|---|---|---|---|
| C 41,61 | H 4,73 | N 7,60 | Gd 20,71 |

**Natrium-Salz**

| | | | | |
|---|---|---|---|---|
| C 39,33 | H 4,22 | N 7,18 | Na 5,72 | Gd 19,57 |

**N-Methylglucamin-Salz**

| | | | |
|---|---|---|---|
| C 42,85 | H 4,43 | N 7,59 | Gd 13,92 |

### BEISPIEL 9

a) 3-Aza-1-(4-hydroxybenzyl)-1,3,5-tri-tosyl-pentan-1,5-diamin
24,0 g (75,3 mMol) 3-Aza-1-(4-hydroxybenzyl)-pentan-1,5-diamin (als Trihydrochlorid Beispiel 1e) werden in 250 ml trockenem Pyridin vorgelegt und bei 0°C unter gutem Rühren 37,9 g (198,7 mMol) Tosylchlorid, gelöst in 100 ml Pyridin, über eine Stunde zugetropft. Man läßt über Nacht bei 4°C stehen, dampft den Hauptteil des Pyridins im Vakuum ab und nimmt in 300 ml Dichlormethan auf. Durch mehrmaliges Waschen mit verdünnter Salzsäure, wäßriger Bicarbonatlösung und bidestilliertem Wasser entfernt man restliches Pyridin und überschüssige Toluolsulfonsäure. Nach Trocknen wird an Kieselgel mit Essigester/Hexan chromatographiert. Man erhält das Tritosylat in Form farbloser Kristalle. Ausbeute 36,9 g (73% der Theorie)
Schmelzpunkt: 145-146°C

| | | | | | | |
|---|---|---|---|---|---|---|
| Analyse: | Ber.: | C 57,20 | H 5,55 | N 6,25 | O 16,66 | S 14,31 |
| | Gef.: | C 57,15 | H 5,32 | N 6,24 | | S 14,20 |

b) 2-(4-Hydroxybenzyl)-1,4,7,10-tetra-tosyl-1,4,7,10-tetraazacyclododecan
10,95 g (16,3 mMol) 3-Aza-1-(4-hydroxybenzyl)-1,3,5-tri-tosyl-pentan-1,5-diamin werden in 100 ml Dimethylformamid gelöst und mit 1,35 g (45 mMol) Natriumhydrid (80% in Paraffin) 30 Minuten bei 35-40°C gerührt. Danach versetzt man langsam mit einer Lösung von 9,51 g (16,3 mMol) 3-Aza-1,3,5-tritosyl-1,5-dihydroxypentan in 100 ml DMF. Man rührt vier Stunden bei 130°C, läßt über Nacht erkalten und destilliert das Lösungsmittel ab. Der Rückstand kristallisiert beim Verreiben mit wenig Methanol. Nach Waschen mit verdünnter Salzsäure und Umkristallisieren aus Acetonitril erhält man 7,4 g (48% der Theorie) farblose Kristalle vom Schmelzpunkt 192-193°C.

| | | | | | | |
|---|---|---|---|---|---|---|
| Analyse: | Ber.: | C 53,84 | H 5,25 | N 5,84 | O 21,68 | S 13,37 |
| | Gef.: | C 53,66 | H 5,17 | N 5,81 | | S 13,30 |

c) 2-(4-Hydroxybenzyl)-1,4,7,10-tetraazacyclododecan . Trihydrobromid
12,0 g (12,7 mMol) 2-(4-Hydroxybenzyl-1,4,7,10-tetra-tosyl-1,4,7,10-tetra-azacyclododecan werden in 50 ml Eisessig mit 33% Bromwasserstoff vier Stunden auf 100°C erhitzt. Man gießt in 300 ml Diethylether, saugt ab und resuspendiert zweimal in jeweils 300 ml Diethylether. Alle Operationen werden unter Stickstoffschintzatmosphäre durchgeführt. Nach dem Trocknen im Vakuum liegen 5,16 g (78% der Theorie) farbloser Kristalle vor, die bei 115-117°C unter Zersetzung schmelzen.

| | | | | | | |
|---|---|---|---|---|---|---|
| Analyse: | Ber.: | C 34,57 | H 5,60 | N 10,75 | O 3,07 | Br 45,99 |
| | Gef.: | C 34,75 | H 5,61 | N 10,77 | | Br 45,64 |

d) 2-(4-Hydroxybenzyl)-1,4,7,10-tetrakis-(tert.-butoxy-carbonylmethyl)-1,4,7, 10-tetraazacyclododecan
8,49 g (16,3 mMol) 2-(4-Hydroxybenzyl)-1,4,7,10-tetraazacyclododecan (als Trihydrobromid) werden in 150 ml Dimethylformamid suspendiert, mit 9,66 g (115 mMol) Natriumhydrogencarbonat versetzt und bei 60°C mit einer Lösung von 12,7 g (65,2 mMol) Bromessigsäure-tert.-butylester in 100 ml DMF versetzt. Nach 2 Stunden Rühren bei dieser Temperatur wird das Lösungsmittel abgezogen und der ölige Rückstand an Kieselgel mit Ether/Hexan chromatographiert. Man erhält ein farbloses viskoses Öl. Ausbeute 9,0 g (79%)

| | | | | | |
|---|---|---|---|---|---|
| Analyse: | Ber.: | C 63,73 | H 9,05 | N 7,62 | O 19,59 |
| | Gef.: | C 63,70 | H 8,97 | N 7,50 | |

e) 2-[4-(Oxiranylmethoxy)-benzyl]-1,4,7,10-tetrakis-(tert.-butoxycarbonylmethyl)-1,4,7,10-tetraazacyclododecan
12,72 g (18,2 mMol) 2-(4-Hydroxybenzyl)-1,4,7,10-tetrakis-(tert.butoxycarbonylmethyl)-1,4,7,10-tetraazacyclododecan (Beispiel 9d) werden in 250 ml Toluol gelöst und mit 350 mg Natriumhydrid (80% in Paraffin, 18,3 mMol) versetzt. Man erwärmt auf 80 bis 100°C und versetzt tropfenweise mit einer Lösung von 1,74 g (18,2 mMol) Epichlorhydrin in 50 ml Toluol. Nach zweistündigem Erhitzen auf Rückflußtemperatur wird eingeengt und über eine Kieselgelsäule gereinigt. Man erhält ein farbloses Öl.
Ausbeute: 11,53 g (79,7% der Theorie)

| | | | | | |
|---|---|---|---|---|---|
| Analyse: | Ber.: | C 63,44 | H 9,38 | N 7,04 | O 20,12 |
| | Gef.: | C 63,34 | H 9,15 | N 7,12 | |

f) Poly-〈4-[2,5,8,11-tetrakis-(carboxymethyl)-2,5,8,11-tetraazacyclododecylmethyl]-3-phenoxy-2-hydroxypropyl〉-polyethylenimin
Wie für Beispiel 5 beschrieben erhält man aus 12 g (15,1 mMol) 2-[4-(Oxiranylmethoxy)-benzyl]-1,4,7,10-tetrakis-(tert.-butoxycarbonylmethyl)-1,4,7,10-tetraazacyclododecan und 645 mg (15 mMol monomere Untereinheiten) Polyethyllenimin 7,49 g weißes feinkristallines Pulver, das sich oberhalb 165°C zersetzt.

| | | | |
|---|---|---|---|
| Analyse: | C 55,16 | H 7,11 | N 11,52 |

Der Gadolinium-Komplex und dessen Salze werden wie in Beispiel 1j beschrieben, erhalten.
**Gadolinium-Komplex**

| | | | |
|---|---|---|---|
| C 44,03 | H 5,28 | N 9,18 | Gd 20,55 |

**Natrium-Salz**

| | | | | |
|---|---|---|---|---|
| C 42,80 | H 5,01 | N 8,93 | Na 2,92 | Gd 19,98 |

**N-Methylglucamin-Salz**

| | | | |
|---|---|---|---|
| C 44,30 | H 4,99 | N 8,87 | Gd 16,55 |

### BEISPIEL 10

### Poly-〈4-[2,5,8,11-tetrakis-(carboxymethyl)-2,5,8,11-tetraazacyclododecylmethyl]-3-phenoxy-2-hydroxypropyl〉-polyethylenimin-poly-[2-(maleimido)-ethylenamid]

Analog der für Beispiel 2 angegebenen Vorschrift werden 3,9 g des nach Beispiel 9 erhaltenen Komplexbildners umgesetzt. Man erhält 4,0 g der Titelverbindung.
Maleimid-Gehalt (UV): 0,48 Gew%

| | | | |
|---|---|---|---|
| Analyse: | C 55,05 | H 7,16 | N 11,63 |

Der Gadolinium-Komplex und dessen Salze werden wie in Beispiel 1j beschrieben, erhalten.
**Gadolinium-Komplex**

| | | | |
|---|---|---|---|
| C 43,96 | H 5,32 | N 9,29 | Gd 20,52 |

**Natrium-Salz**

| | | | | |
|---|---|---|---|---|
| C 42,74 | H 5,04 | N 9,03 | Na 2,91 | Gd 19,95 |

**N-Methylglucamin-Salz**

| | | | |
|---|---|---|---|
| C 44,24 | H 5,03 | N 8,95 | Gd 16,53 |

### BEISPIEL 11

### Poly-〈4-[2,5,8,11-tetrakis-(carboxymethyl)-2,5,8,11-tetraazacyclododecylmethyl]-3-phenoxy-2-hydroxypropyl〉-polyethylenimin-polyhydrazid

Analog der für Beispiel 3 angegebenen Vorschrift werden 2,5 g des nach Beispiel 9 erhaltenen Komplexbildners umgesetzt. Man erhält 2,3 g der Titelverbindung.
Hydrazin-Gehalt (colorim.):0,35 Gew%

| | | | |
|---|---|---|---|
| Analyse: | C 55,18 | H 7,14 | N 11,61 |

Der Gadolinium-Komplex und dessen Salze werden wie in Beispiel 1j beschrieben, erhalten.
**Gadolinium-Komplex**

| | | | |
|---|---|---|---|
| C 42,89 | H 5,31 | N 9,28 | Gd 20,43 |

**Natrium-Salz**

| | | | | |
|---|---|---|---|---|
| C 42,89 | H 5,04 | N 9,03 | Na 2,9 | Gd 19,86 |

**N-Methylglucamin-Salz**

| | | | |
|---|---|---|---|
| C 44,37 | H 5,02 | N 8,95 | Gd 16,47 |

### BEISPIEL 12

### Poly-〈4-[2,5,8,11-tetrakis-(carboxymethyl)-2,5,8,11-tetraazacyclododecylmethyl]-3-phenoxy-2-hydroxypropyl〉-polyethylenimin-poly-[10-hydrazinocarbonyl)-decylamid]

Analog der für Beispiel 4 angegebenen Vorschrift werden 5,8 g des nach Beispiel 9 hergestellten Komplexbildners umgesetzt. Man erhält 5,9 g der Titelverbindung.
Hydrazin-Gehalt (colorim.): 0,35 Gew%

| | | | |
|---|---|---|---|
| Analyse: | C 55,14 | H 7,10 | N 11,55 |

Der Gadolinium-Komplex und dessen Salze werden wie in Beispiel 1j beschrieben, erhalten.
**Gadolinium-Komplex**

| | | | |
|---|---|---|---|
| C 44,06 | H 5,28 | N 9,22 | Gd 20,48 |

**Natrium-Salz**

| | | | | |
|---|---|---|---|---|
| C 42,84 | H 5,01 | N 8,97 | Na 2,91 | Gd 19,91 |

**N-Methylglucamin-Salz**

| | | | |
|---|---|---|---|
| C 44,32 | H 4,99 | N 8,91 | Gd 16,50 |

### BEISPIEL 13

a) N³-(2,6-Dioxomorpholinoethyl)-N⁶-(ethoxycarbonylmethyl)-3,6-diazaoctandisäure
Eine Suspension von 21,1 g (50 mMol) N³,N⁶-Bis-(carboxymethyl)-N⁹-(ethoxycarbonylmethyl)-3,6,9-triazaundecandisäure (J. Pharm. Sci. 68, 1979, 194) in 250 ml Essigsäureanhydrid läßt man nach Zugabe von 42,2 ml Pyridin drei Tage bei Raumtemperatur rühren. Dann saugt man den Niederschlag ab, wäscht ihn dreimal mit je 50 ml Essigsäureanhydrid und verrührt ihn anschließend mehrere Stunden mit absolutem Diethylether. Nach Absaugen, Waschen mit absolutem Diethylether und Trocknen im Vakuum bei 40°C erhält man 18,0 g (=89% der Theorie) eines weißen Pulvers vom Schmelzpunkt 195-196°C.

| Analyse (bezogen auf wasserfreie Substanz): | | | |
|---|---|---|---|
| Ber.: | C 47,64 | H 6,25 | N 10,42 |
| Gef.: | C 47,54 | H 6,30 | N 10,22 |

b) Poly-N-[10-carboxy-3,6,9-tris(carboxymethyl)-3,6,9-triazadecanoyl]-polyethylenimin
380 mg Polyethylenimin (8,8 mMol monomere Untereinheiten) werden in 50 ml Wasser gelöst. Unter Eiskühlung gibt man portionsweise 2,37 g (5,9 mMol) des nach 13a erhaltenen Monoanhydrids zu, wobei der pH-Wert mit 1 n Natronlauge auf Werten über 9 gehalten wird. Nach einer Stunde Rühren wird dialysiert und gefriergetrocknet. Ausbeute 1,48 g

| | | | |
|---|---|---|---|
| Analyse: | C 46,55 | H 6,46 | N 14,51 |

c) Der Gadolinium-Komplex und dessen Salze werden analog Beispiel 1j erhalten.
Gadolinium-Komplex

| | | | |
|---|---|---|---|
| C 35,10 | H 4,41 | N 10,51 | Gd 26,22 |

Natrium-Salz

| | | | | |
|---|---|---|---|---|
| C 33,25 | H 4,02 | N 10,33 | Na 3,72 | Gd 25,85 |

N-Methylglucamin-Salz

| | | | |
|---|---|---|---|
| C 36,95 | H 4,21 | N 10,02 | Gd 20,40 |

d) Der Dysprosium-Komplex und dessen Salze werden, wie in Beispiel 1j beschrieben, erhalten, wenn man anstelle von Gadoliniumacetat Dysprosiumacetat einsetzt.
**Dysprosium-Komplex**

| | | | |
|---|---|---|---|
| C 34,24 | H 4,25 | N 10,66 | Dy 26,94 |

**Natrium-Salz**

| | | | | |
|---|---|---|---|---|
| C 33,04 | H 3,94 | N 10,28 | Na 3,67 | Dy 25,99 |

**N-Methylglucamin-Salz**

| | | | |
|---|---|---|---|
| C 36,87 | H 4,15 | N 9,93 | Dy 20,61 |

### BEISPIEL 14

### Poly-N-[10-carboxy-3,6,9-tris(carboxymethyl)-3,6,9-triazadecanoyl]-polyethylenimin-poly-[2-(maleimido)-ethylenamid]

Analog der für Beispiel 2 angegebenen Vorschrift werden 5,2 g des nach Beispiel 13 hergestellten Komplexbildners umgesetzt. Man erhält 4,5 g der Titelverbindung.
Maleimid-Gehalt (UV): 0,27 Gew%

| | | | |
|---|---|---|---|
| Analyse: | C 46,53 | H 6,51 | N 14,51 |

Der Gadolinium-Komplex und dessen Salze werden, wie in Beispiel 1j beschrieben, erhalten.
**Gadolinium-Komplex**

| | | | |
|---|---|---|---|
| C 34,58 | H 4,28 | N 10,78 | Gd 26,24 |

**Natrium-Salz**

| | | | | |
|---|---|---|---|---|
| C 33,35 | H 3,95 | N 10,35 | Na 3,65 | Gd 25,30 |

**N-Methylglucamin-Salz**

| | | | |
|---|---|---|---|
| C 37,12 | H 4,15 | N 10,00 | Gd 20,05 |

### BEISPIEL 15

### Poly-N-[10-carboxy-3,6,9-tris(carboxymethyl)-3,6,9-triazadecanoyl]-polyethylenimin-polyhydrazid

Analog der für Beispiel 3 angegebenen Vorschrift werden 2,5 g des nach Beispiel 13 hergestellten Komplexbildners umgesetzt. Man erhält 2,2 g der Titelverbindung.
Hydrazin-Gehalt (colorim.): 0,47 Gew%

| | | | |
|---|---|---|---|
| Analyse: | C 64,47 | H 6,44 | N 14,49 |

Der Gadolinium-Komplex und dessen Salze werden, wie in Beispiel 1j beschrieben, erhalten.
**Gadolinium-Komplex**

| | | | |
|---|---|---|---|
| C 34,32 | H 4,26 | N 10,75 | Gd 26,38 |

**Natrium-Salz**

| | | | | |
|---|---|---|---|---|
| C 33,36 | H 3,96 | N 10,31 | Na 3,73 | Gd 25,39 |

**N-Methylglucamin-Salz**

| | | | |
|---|---|---|---|
| C 37,04 | H 4,15 | N 9,95 | Gd 20,19 |

### BEISPIEL 16

### Poly-N-[10-carboxy-3,6,9-tris(carboxymethyl)-3,6,9-triazadecanoyl]-polyethylenimin-poly-[10-(hydrazinocarbonyl)-decylamid]

Analog der für Beispiel 4 angegebenen Vorschrift werden 2,8 g des nach Beispiel 13 hergestellten Komplexbildners umgesetzt. Man erhält 2,9 g der Titelverbindung.
Hydrazin-Gehalt (colorim.): 0,31 Gew%

| | | | |
|---|---|---|---|
| Analyse: | C 46,66 | H 6,43 | N 14,55 |

Der Gadolinium-Komplex und dessen Salze werden, wie in Beispiel 1j beschrieben, erhalten.
**Gadolinium-Komplex**

| | | | |
|---|---|---|---|
| C 34,44 | H 4,27 | N 10,72 | Gd 26,20 |

**Natrium-Salz**

| | | | | |
|---|---|---|---|---|
| C 33,39 | H 3,96 | N 10,35 | Na 3,72 | Gd 25,48 |

**N-Methylglucamin-Salz**

| | | | |
|---|---|---|---|
| C 37,07 | H 4,19 | N 9,96 | Gd 20,00 |

### BEISPIEL 17

a) 3-Aza-2-(4-benzyloxybenzyl)-4-oxoglutarsäurediamid (Methode A)
3,62 g (13,3 mMol) 0-Benzyltyrosinamid werden mit 2,7 g Ethyloxamat (23 mMol) 14 Stunden in Dimethoxyethan am Rückfluß gekocht. Nach Abziehen des Lösungsmittels wäscht man sukzessive mit Wasser, Ethanol und Ether. Nach Trocknen erhält man 2,73 g weißer Kristalle (60% der Theorie).
Schmelzpunkt: 270°C

| | | | | | |
|---|---|---|---|---|---|
| Analyse: | Ber.: | C 63,33 | H 5,61 | N 12,30 | O 18,74 |
| | Gef.: | C 63,24 | H 5,52 | N 12,14 | |

oder nach Methode B
α) 3-Aza-2-(4-benzyloxybenzyl)-4-oxoglutarsäure-5-ethylester-1-amid
3 g (11,1 mMol) O-Benzyltyrosinamid werden in 30 ml Dimethoxyethan gelöst, mit 1,56 ml Triethylamin versetzt und bei 0°C 1,53 g (11,1 mMol) Oxalsäureethylesterchlorid zugetropft. Nach 30 Minuten bei 0°C gießt man auf 100 ml Eis, saugt ab und trocknet. Die Ausbeute beträgt 3,87 g (94% der Theorie).
Schmelzpunkt: 142°C

| | | | | | |
|---|---|---|---|---|---|
| Analyse: | Ber.: | C 64,85 | H 5,98 | N 7,56 | O 21,59 |
| | Gef.: | C 64,71 | H 6,11 | N 7,46 | |

β) 3,6 g (9,72 mMol) der Verbindung nach Beispiel aα werden mit 40 ml einer Lösung von 1 Mol NH₃/l Methanol übergossen. Nach einer Stunde filtriert man das ausgefallene Produkt ab. Nach Trocknen werden 3,13 g (95% der Theorie) der Titelverbindung in Form farbloser Kristalle erhalten.
Schmelzpunkt: 269°C

| | | | | | |
|---|---|---|---|---|---|
| Analyse: | Ber.: | C 63,33 | H 5,61 | N 12,30 | O 18,74 |
| | Gef.: | C 63,25 | H 5,63 | N 12,17 | |

b) 3-Aza-2-(4-hydroxybenzyl)-4-oxoglutarsäurediamid
1 g (2,9 mMol) der Verbindung von Beispiel 17a wird mit 100 mg 10% Palladium-Kohle und einigen Tropfen konzentrierter Salzsäure in 20 ml Methanol suspendiert und bis zum Ende der Wasserstoffaufnahme hydriert. Nach Abfiltrieren vom Katalysator erhält man 690 mg farblose Kristalle (93% der Theorie).
Schmelzpunkt: 245-250°C (Zersetzung)

| | | | | | |
|---|---|---|---|---|---|
| Analyse: | Ber.: | C 52,58 | H 5,21 | N 16,72 | O 25,47 |
| | Gef.: | C 52,83 | H 5,19 | N 16,84 | |

c) 3-Aza-2-(4-hydroxybenzyl)-pentan-1,5-diamin - Trihydrochlorid 1 g der Verbindung nach Beispiel b werden nach der für Beispiel 1e gegebenen Vorschrift umgesetzt. Das erhaltene farblose Kristallisat wiegt 1,19 g (93,7% der Theorie).
Schmelzpunkt: 238°C

| | | | | | | |
|---|---|---|---|---|---|---|
| Analyse: | Ber.: | C 41,61 | H 6,98 | N 13,23 | O 5,03 | Cl 33,13 |
| | Gef.: | C 41,60 | H 6,95 | N 13,17 | | Cl 33,33 |

d) 3,6,9-Triaza-3,6,9-tris-(tert.-butoxycarbonylmethyl)-5-(4-hydroxybenzyl)-undecandisäure-bis-(tert.-butyl)-diester
Nach der für Beispiel 1f gegebenen Vorschrift werden 5,19 g (16,3 mMol) der Titelverbindung von Beispiel c zu 7,75 g (61% der Theorie) der Titelverbindung in Form einer zähviskosen klaren Flüssigkeit umgesetzt.

| | | | | | |
|---|---|---|---|---|---|
| Analyse: | Ber.: | C 63,13 | H 8,91 | N 5,38 | O 22,56 |
| | Gef.: | C 63,00 | H 8,92 | N 5,29 | |

e) 3,6,9-Triaza--5-(4-benzyloxycarbonylmethoxybenzyl)-3,6,9-tris-(tert.-butoxycarbonylmethyl)-undecandisäure-bis-(tert.-butyl)-diester
5,0 g (6,4 mMol) der Titelverbindung von Beispiel d werden nach der Vorschrift für Beispiel 1g mit Bromessigsäurebenzylester umgesetzt zu 4,6 g (74,8 % der Theorie) eines farblosen, zähflüssigen Öls.

| | | | | | |
|---|---|---|---|---|---|
| Analyse: | Ber.: | C 64,70 | H 8,26 | N 4,52 | O 22,40 |
| | Gef.: | C 64,46 | H 8,30 | N 4,49 | |

f) 3,6,9-Triaza-3,6,9-tris-(tert.-butoxycarbonylmethyl)-5-(4-carboxymethoxybenzyl)-undecandisäure-bis-(tert.-butyl)-diester
Aus 4,9 g (5,16 mMol) des in der vorigen Reaktionsstufe erhaltenen Benzylesters werden nach der unter Beispiel 1h angegebenen Vorschrift 4,1 g eines farblosen zähen Öls erhalten (93,2 % der Theorie).

| | | | | | |
|---|---|---|---|---|---|
| Analyse: | Ber.: | C 61,62 | H 8,53 | N 5,01 | O 24,81 |
| | Gef.: | C 61,66 | H 8,45 | N 5,15 | |

g) Poly-[4-〈5-{bis-(carboxymethyl)-amino}-3-(carboxymethyl)-aza-2-[2-(bis-(carboxymethyl)-amino -methyl]-pentyl〉-phenoxyacetyl]-polyethylenimin
Die Titelverbindung wird nach der für Beispiel 1i angegebenen Vorschrift aus 3,27 g des in der vorigen Reaktionsstufe beschriebenen Pentaesters und 245 mg Polyethylenimin synthetisiert. Man erhält so 2,0 g des polymeren Komplexbildners als weißes kristallines Pulver.

| | | | |
|---|---|---|---|
| Analyse: | C 51,76 | H 6,04 | N 10,56 |

Der Gadolinium-Komplex und dessen Salze werden, wie in Beispiel 1j beschrieben, erhalten.
**Gadolinium-Komplex**

| | | | |
|---|---|---|---|
| C 41,32 | H 4,42 | N 8,45 | Gd 20,70 |

**Natrium-Salz**

| | | | | |
|---|---|---|---|---|
| C 38,91 | H 3,91 | N 7,94 | Na 5,71 | Gd 19,58 |

**N-Methylglucamin-Salz**

| | | | |
|---|---|---|---|
| C 42,60 | H 4,23 | N 8,13 | Gd 13,87 |

### BEISPIEL 18

### Poly-[4-〈5-{bis-(carboxymethyl)-amino}-3-(carboxymethyl)-aza-2-[2-(bis-(carboxymethyl)-amino)-methyl]-pentyl〉-phenoxyacetyl]polyethylenimin-poly-[2-(maleimido)-ethylenamid]

Analog der für Beispiel 2 angegebenen Vorschrift werden 4,3 g des nach Beispiel 17 erhaltenen Komplexbildners umgesetzt. Man erhält 4,1 g der Titelverbindung. Maleimidgehalt (UV) 0,68 Gew%

| | | | |
|---|---|---|---|
| Analyse: | C 51,74 | H 6,05 | N 10,55 |

Der Gadolinium-Komplex und dessen Salze werden, wie in Beispiel 1j beschrieben, erhalten.
**Gadolinium-Komplex**

| | | | |
|---|---|---|---|
| C 41,09 | H 4,43 | N 8,43 | Gd 20,69 |

**Natrium-Salz**

| | | | | |
|---|---|---|---|---|
| C 38,95 | H 3,93 | N 7,98 | Na 5,71 | Gd 19,50 |

**N-Methylglucamin-Salz**

| | | | |
|---|---|---|---|
| C 42,54 | H 4,21 | N 8,15 | Gd 13,88 |

### BEISPIEL 19

### Poly-[4-〈5-{bis-(carboxymethyl)-amino}-3-(carboxymethyl)-aza-2-[2-(bis-(carboxymethyl)-amino)-methyl]-pentyl〉-phenoxyacetyl]-polyethylenimin-polyhydrazid

Analog der für Beispiel 3 angegebenen Vorschrift werden 2,6 g des nach Beispiel 17 erhaltenen Komplexbildners umgesetzt. Man erhält 2,5 g der Titelverbindung.
Hydrazin-Gehalt (colorim.) 0,32 Gew%

| | | | |
|---|---|---|---|
| Analyse: | C 51,91 | H 6,05 | N 10,52 |

Der Gadolinium-Komplex und dessen Salze werden, wie in Beispiel 1j beschrieben, erhalten.
**Gadolinium-Komplex**

| | | | |
|---|---|---|---|
| C 41,06 | H 4,41 | N 8,43 | Gd 20,71 |

**Natrium-Salz**

| | | | | |
|---|---|---|---|---|
| C 39,13 | H 3,93 | N 7,93 | Na 5,73 | Gd 19,48 |

**N-Methylglucamin-Salz**

| | | | |
|---|---|---|---|
| C 42,57 | H 4,21 | N 8,11 | Gd 13,92 |

### BEISPIEL 20

### Poly-[4-〈5-{bis-(carboxymethyl)-amino}-3-(carboxymethyl)-aza-2-[2-(bis-(carboxymethyl)-amino)-methyl]-pentyl〉-phenoxyacetyl]-polyethylenimin-poly-[10-(hydrazinocarbonyl)-decylamid]

Analog der für Beispiel 4 angegebenen Vorschrift werden 2,8 g des nach Beispiel 17 erhaltenen Komplexbildners umgesetzt. Man erhält 2,9 g der Titelverbindung.
Hydrazin-Gehalt (colorim.) 0,77 Gew%

| | | | |
|---|---|---|---|
| Analyse: | C 52,03 | H 6,09 | N 10,65 |

Der Gadolinium-Komplex und dessen Salze werden, wie in Beispiel 1j beschrieben, erhalten.
**Gadolinium-Komplex**

| | | | |
|---|---|---|---|
| C 41,29 | H 4,47 | N 8,52 | Gd 20,52 |

**Natrium-Salz**

| | | | | |
|---|---|---|---|---|
| C 39,14 | H 3,96 | N 8,06 | Na 5,67 | Gd 19,40 |

**N-Methylglucamin-Salz**

| | | | |
|---|---|---|---|
| C 42,76 | H 4,24 | N 8,17 | Gd 13,79 |

### BEISPIEL 21

a) 3,6-Diaza-3,6-bis-(tert.-butoxycarbonylmethyl)-4-(4-hydroxybenzyl)-suberinsäure -bis-(tert.-butyl)-diester
15,31 g (0,064 Mol) 4-Hydroxybenzyl-1,2-ethan-diamin als Dihydrochlorid und 71,14 g (0,71 Mol) Kaliumhydrogencarbonat werden in 380 ml Dimethylformamid (getrocknet über Natriumhydrid) vorgelegt und bei 35°C 50 g (0,26 Mol) Bromessigsäure-tert.-butylester in 80 ml Dimethylformamid zugetropft. Man rührt noch weitere 2,5 Stunden bei 35°C, wonach kein Ausgangsprodukt mehr dünnschichtchromatographisch nachzuweisen ist. Man filtriert von ausgefallenem Kaliumbromid ab und engt das Filtrat ein. Der Rückstand wird mit Wasser versetzt und mehrmals mit Ether extrahiert. Nach Trocknen und Einengen wird der Etherextrakt über eine Kieselgelsäule von unumgesetztem Bromessigsäure-tert.butylester gereinigt. Man erhält 24,8 g (63% der Theorie) eines farblosen Öls.

| | | | | | |
|---|---|---|---|---|---|
| Analyse: | Ber.: | C 63,64 | H 8,73 | N 4,49 | O 23,12 |
| | Gef.: | C 63,78 | H 8,69 | N 4,41 | |

b) Poly-[4-〈2,3-di-(bis-(carboxymethyl))-aminopropyl〉-phenyliminocarbamat]-polyethylenimin
7,2 g (11,56 mMol) der Titelverbindung von a) werden in 150 ml Methanol gelöst und portionsweise mit 1,4 g (13,3 mMol) Bromcyan versetzt, wobei gleichzeitig eine äquimolare Menge 0,1 n methanolischer Kalilauge zugetropft wird. Die Temperatur darf dabei 10°C nicht überschreiten. Nach 15 Minuten versetzt man mit einer Lösung von 470 mg (10,9 mMol monomere Untereinheiten) Polyethylenimin in 20 ml Methanol und erwärmt langsam auf 40°C. Nach einer halben Stunde bei dieser Temperatur dampft man das Lösungsmittel ab (nachdem man vorher einen eventuellen Niederschlag abfiltriert hat) und löst den Rückstand in 150 ml Ameisensäure unter Erwärmen. Nach 2 Stunden bei 60°C gießt man in 2 Liter Eiswasser, dialysiert und unterwirft das Retentat einer Gefriertrocknung. Das weiße, flockige Kristallisat beginnt sich oberhalb von 80°C zu zersetzen. Die Ausbeute beträgt 3,56 g.

| | | | |
|---|---|---|---|
| Analyse: | C 51,52 | H 5,65 | N 12,13 |

Der Gadolinium-Komplex und dessen Salze werden, wie in Beispiel 1j beschrieben, erhalten.
**Gadolinium-Komplex**

| | | | |
|---|---|---|---|
| C 38,59 | H 3,77 | N 9,14 | Gd 25,10 |

**Natrium-Salz**

| | | | | |
|---|---|---|---|---|
| C 37,45 | H 3,47 | N 8,80 | Na 3,57 | Gd 24,37 |

**N-Methylglucamin-Salz**

| | | | |
|---|---|---|---|
| C 40,14 | H 3,78 | N 8,76 | Gd 19,46 |

### BEISPIEL 22

### Poly-[4-〈2,3-di-(bis-(carboxymethyl))-aminopropyl]-phenyliminocarbamat〉-polyethylenimin-poly-[2-(maleimido)-ethylamid]

Analog der für Beispiel 2 angegebenen Vorschrift werden 2,5 g des nach Beispiel 21 erhaltenen Komplexbildners umgesetzt. Man erhält 2,2 g der Titelverbindung.
Maleimid-Gehalt (UV) 0,67 Gew%

| | | | |
|---|---|---|---|
| Analyse: | C 51,46 | H 5,66 | N 12,18 |

Der Gadolinium-Komplex und dessen Salze werden, wie in Beispiel 1j beschrieben, erhalten.
**Gadolinium-Komplex**

| | | | |
|---|---|---|---|
| C 38,85 | H 3,77 | N 9,10 | Gd 25,17 |

**Natrium-Salz**

| | | | | |
|---|---|---|---|---|
| C 37,63 | H 3,48 | N 8,80 | Na 3,55 | Gd 24,42 |

**N-Methylglucamin-Salz**

| | | | |
|---|---|---|---|
| C 40,36 | H 3,79 | N 8,80 | Gd 19,50 |

### BEISPIEL 23

### Poly-[4-〈2,3-di-(bis-(carboxymethyl))-aminopropyl]-phenyliminocarbamat〉-polyethylenimin-polyhydrazid

Analog der für Beispiel 3 angegebenen Vorschrift werden 4,4 g des nach Beispiel 21 erhaltenen Komplexbildners umgesetzt. Man erhält 4,2 g der Titelverbindung.
Hydrazin-Gehalt (colorium.) 0,32 Gew%

| | | | |
|---|---|---|---|
| Analyse: | C 51,64 | H 5,66 | N 12,08 |

Der Gadolinium-Komplex und dessen Salze werden, wie in Beispiel 1j beschrieben, erhalten.
Gadolinium-Komplex

| | | | |
|---|---|---|---|
| C 38,79 | H 3,77 | N 9,09 | Gd 25,21 |

Natrium-Salz

| | | | | |
|---|---|---|---|---|
| C 37,57 | H 3,48 | N 8,77 | Na 3,54 | Gd 24,44 |

N-Meglumin-Salz

| | | | |
|---|---|---|---|
| C 40,41 | H 3,79 | N 8,80 | Gd 19,48 |

### BEISPIEL 24

### Poly-[4-〈2,3-di-(bis-(carboxymethyl))-aminopropyl]-phenyliminocarbamat〉-polyethylenimin-poly-[10-(hydrazinocarbonyl)-decylamid]

Analog der für Beispiel 4 angegebenen Vorschrift werden 3,1 g des nach Beispiel 21 erhaltenen Komplexbildners umgesetzt. Man erhält 3,2 g der Titelverbindung.
Hydrazin-Gehalt (colorim.) 0,17Gew%

| | | | |
|---|---|---|---|
| Analyse: | C 51,43 | H 5,70 | N 12,16 |

Der Gadolinium-Komplex und dessen Salze werden, wie in Beispiel 1j beschrieben, erhalten.
Gadolinium-Komplex

| | | | |
|---|---|---|---|
| C 39,03 | H 3,81 | N 9,24 | Gd 25,02 |

Natrium-Salz

| | | | | |
|---|---|---|---|---|
| C 37,41 | H 3,51 | N 8,93 | Na 3,53 | Gd 24,35 |

N-Meglumin-Salz

| | | | |
|---|---|---|---|
| C 40,56 | H 3,82 | N 8,81 | Gd 19,40 |

### BEISPIEL 25

a) N-Carbobenzoxyserin-(2-carbobenzoxyaminoethylen)-amid
7,34 g (30,7 mMol) N-Carbobenzoxyserin werden in 120 ml trockenem Tetrahydrofuran gelöst, mit 5 ml Et₃N versetzt und dann tropfenweise 2,9 ml Chlorameisensäureethylester zugefügt, wobei die Temperatur auf unter -10°C gehalten wird. Nach Beendigung der Zugabe wird 30 Minuten bei dieser Temperatur gerührt, nochmals mit der gleichen Menge vorgekühltem Triethylamin versetzt und eine eiskalte Lösung von 7,1 g (30,7 mMol) N-(2-Aminoethyl)-carbaminsäurebenzylester-hydrochlorid in 70 ml Dimethylformamid zugetropft. Man rührt noch 30 Minuten bei -10°C, läßt dann unter Rühren auf Raumtemperatur kommen und erwärmt dann 10 Minuten auf 30°C. Danach entfernt man das Lösungsmittel am Rotationsverdampfer und gießt auf 500 ml Eiswasser. Das Kristallisat wird abgesaugt, mit Eiswasser gewaschen und getrocknet. Die Ausbeute beträgt 10,33 g (81% der Theorie).
Schmelzpunkt: 167°C

| | | | | | |
|---|---|---|---|---|---|
| Analyse: | Ber.: | C 60,71 | H 6,06 | N 10,11 | O 23,10 |
| | Gef.: | C 60,75 | H 5,98 | N 10,15 | |

b) (2-Aminoethyl)-serinamid
13,46 g (32,4 mMol) N-Carbobenzoxyserin-(2-carbobenzoxyaminoethylen)-amid werden in 200 ml Methanol in Gegenwart von 1,37 g 10% Palladium/Kohle solange hydriert, bis kein Wasserstoff mehr aufgenommen wird. Man filtriert vom Katalysator ab und entfernt alle flüchtigen Anteile an der Ölpumpe. Es hinterbleibt ein zähes, teilweise kristallines Öl.
Ausbeute 4,67 g (98% der Theorie)

| | | | | | |
|---|---|---|---|---|---|
| Analyse: | Ber.: | C 40,80 | H 8,89 | N 28,55 | O 21,74 |
| | Gef.: | C 40,71 | H 8,85 | N 28,30 | |

c) 1-Hydroxymethyl-1,3,5-triazapentan . Trihydrochlorid
4,3 g (29,3 mMol) (2-Aminoethyl)-serinamid (Beispiel 25b) werden in 130 ml trockenem Tetrahydrofuran suspendiert und ein langsamer Strom von B₂H₆ (aus 5,6 g NaBH₄ in 75 ml Diethylenglykoldimethylether und 54 ml Bortrifluorid-Etherat-Komplex) mit trockenem Stickstoff unter stetigem Rühren durch die Lösung getrieben. Man rührt über Nacht bei 60°C, tropft danach bei 20°C 30 ml Methanol zu und leitet unter Eiskühlung Chlorwasserstoff ein. Man kocht danach kurz auf und saugt ab. Das Trihydrochlorid wird als weißes, kristallines Pulver in 69 %iger Ausbeute erhalten.
Schmelzpunkt: 236°C (Zersetzung)

| | | | | | | |
|---|---|---|---|---|---|---|
| Analyse: | Ber.: | C 24,75 | H 7,47 | N 17,32 | O 6,59 | Cl 43,84 |
| | Gef.: | C 24,71 | H 7,40 | N 17,41 | | Cl 43,75 |

d) 3,6,9-Triaza-3,6,9-tris-(tert.-butoxycarbonylmethyl)-4-hydroxymethyl-unde candisäure-bis-(tert.-butyl)-diester
17,85 g (73,59 mMol) des nach 25c) erhaltenen Triamins werden in 450 ml Dimethylformamid vorgelegt und mit 54,4 g (648 mMol) Natriumhydrogencarbonat versetzt. Bei 35°C tropft man unter Rühren 78,95 g (404,72 mMol) Bromessigsäure-tert.-butylester zu, rührt danach noch 3 Stunden bei 35°C nach und filtriert vom ausgefallenen Salz ab. Nach Einengen versetzt man den Rückstand mit Wasser und extrahiert mehrmals mit Ether. Man trennt vom unumgesetzten Bromessigsäureester an einer Kieselgelsäule ab und erhält nach Abziehen der Lösungsmittel 42,32 g (60,12 mMol) eines farblosen zähen Öls. (81,7 % der Theorie)

| | | | | | |
|---|---|---|---|---|---|
| Analyse: | Ber.: | C 59,72 | H 9,30 | N 5,97 | O 25,0 |
| | Gef.: | C 59,66 | H 9,17 | N 5,92 | |

e) 3,6,9-Triaza-3,6,9-tris-(tert.-butoxycarbonylmethyl)-4-[(oxiranylmethoxy)-methyl]-undecandisäure-bis-(tert.-butyl)-diester
24,16 g (34,32 mMol) der nach 25d) erhaltenen Hydroxymethyl-Verbindung werden mit 1,47 g (37,76 mMol) Natriumamid in 500 ml trockenem Toluol gelöst und bei 40°C langsam mit einer Lösung von 3,46 g (37,41 mMol) Epichlorhydrin in 50 ml Toluol versetzt. Nach einer Stunde Rühren bei dieser Temperatur saugt man Unlösliches ab, engt ein und reinigt über eine Kieselgelsäule. Es bleiben 24,0 g (31,58 mMol) farbloses Öl zurück. (92% der Theorie)

| | | | | | |
|---|---|---|---|---|---|
| Analyse: | Ber.: | C 60,05 | H 9,15 | N 5,53 | O 25,26 |
| | Gef.: | C 60,11 | H 9,32 | N 5,49 | |

f) Poly-〈6.10-di-[bis-(carboxymethyl)-amino]-8-[(carboxymethyl)aza]-2-hydroxy-4-oxa-decyl〉-polyethylenimin
Wie für Beispiel 5 beschrieben erhält man die Titelverbindung aus 4,2 g der in der vorigen Reaktionsstufe erhaltenen Verbindung und 230 mg Polyethylenimin als farbloses kristallines Pulver in einer Ausbeute von 2,31 g. Oberhalb von 145°C sintert die Verbindung unter allmählicher Dunkelfärbung.

| | | | |
|---|---|---|---|
| Analyse: | C 45,99 | H 6,57 | N 10,77 |

Der Gadolinium-Komplex und dessen Salze werden, wie in Beispiel 1j beschrieben, erhalten.
**Gadolinium-Komplex**

| | | | |
|---|---|---|---|
| C 35,51 | H 4,63 | N 8,32 | Gd 23,18 |

**Natrium-Salz**

| | | | | |
|---|---|---|---|---|
| C 33,28 | H 4,06 | N 7,08 | Na 6,34 | Gd 21,67 |

**N-Meglumin-Salz**

| | | | |
|---|---|---|---|
| C 39,08 | H 4,32 | N 8,08 | Gd 15,05 |

### BEISPIEL 26

### Poly-〈6.10-di-[bis-(carboxymethyl)-amino]-8-[(carboxymethyl)-aza]-2-hydroxy-4-oxa-decyl〉-polyethylenimin-[2-(maleimido)-ethylenamid]

Analog der für Beispiel 2 angegebenen Vorschrift werden 2,5 g des nach Beispiel 25 erhaltenen Komplexbildners umgesetzt. Man erhalt 2,4 g der Titelverbindung.
Maleimid-Gehalt (UV) 0,30 Gew%

| | | | |
|---|---|---|---|
| Analyse: | C 46,17 | H 6,54 | N 10,8 |

Der Gadolinium-Komplex und dessen Salze werden, wie in Beispiel 1j beschrieben, erhalten.
**Gadolinium-Komplex**

| | | | |
|---|---|---|---|
| C 35,43 | H 4,65 | N 8,41 | Gd 23,20 |

**Natrium-Salz**

| | | | | |
|---|---|---|---|---|
| C 33,45 | H 4,09 | N 7,83 | Na 6,31 | Gd 21,77 |

**N-Meglumin-Salz**

| | | | |
|---|---|---|---|
| C 39,23 | H 4,35 | N 8,10 | Gd 14,90 |

### BEISPIEL 27

### Poly-〈6.10-di-[bis-(carboxymethyl)-amino]-8-[(carboxymethyl)-aza]-2-hydroxy-4-oxa-decyl〉-polyethylenimin-polyhydrazid

Analog der für Beispiel 3 angegebenen Vorschrift werden 2,3 g des nach Beispiel 25 erhaltenen Komplexbildners umgesetzt. Man erhält 2,3 g der Titelverbindung.
Hydrazin-Gehalt (colorim.) 0,37 Gew%

| | | | |
|---|---|---|---|
| Analyse: | C 45,89 | H 6,59 | N 10,75 |

Der Gadolinium-Komplex und dessen Salze werden, wie in Beispiel 1j beschrieben, erhalten.
**Gadolinium-Komplex**

| | | | |
|---|---|---|---|
| C 35,66 | H 4,64 | N 8,29 | Gd 23,11 |

**Natrium-Salz**

| | | | | |
|---|---|---|---|---|
| C 33,42 | H 4,05 | N 7,82 | Na 6,35 | Gd 21,7 |

**N-Meglumin-Salz**

| | | | |
|---|---|---|---|
| C 39,12 | H 4,32 | N 8,01 | Gd 15,00 |

### BEISPIEL 28

### Poly-〈6.10-di-[bis-(carboxymethyl)-amino]-8-[(carboxymethyl)-aza]-2-hydroxy-4-oxa-decyl〉-polyethylenimin-poly-[10-(hydrazinocarbonyl)-decylamid]

Analog der für Beispiel 4 angegebenen Vorschrift werden 3,0 g des nach Beispiel 25 erhaltenen Komplexbildners umgesetzt. Man erhält 3,1 g der Titelverbindung.
Hydrazin-Gehalt (colorim.) 0,46 Gew%

| | | | |
|---|---|---|---|
| Analyse: | C 46,06 | H 6,61 | N 10,81 |

Der Gadolinium-Komplex und dessen Salze werden, wie in Beispiel 1j beschrieben, erhalten.
**Gadolinium-Komplex**

| | | | |
|---|---|---|---|
| C 35,74 | H 4,66 | N 8,39 | Gd 23,05 |

**Natrium-Salz**

| | | | | |
|---|---|---|---|---|
| C 33,32 | H 4,08 | N 7,90 | Na 6,33 | Gd 21,6 |

**N-Meglumin-Salz**

| | | | |
|---|---|---|---|
| C 39,25 | H 4,33 | N 8,06 | Gd 14,99 |

### BEISPIEL 29

### Poly-[4-〈2,6-di-(bis-(carboxymethyl))-amino]-4-(carboxymethyl)-aza-hexyl]-phenoxyacetyl〉-polylysin

Zu einer Lösung von 3,21 g (25 mMol Lysyluntereinheiten) Polylysin und 2,8 g KOH in 150 ml Wasser tropft man bei 0°C eine Lösung, die man aus 25,14 g (30 mMol) der Titelverbindung von Beispiel 1h, 4,7 g (30 mMol) Chlorameisensäureisobutylester und 3,03 g (30 mMol) Triethylamin in 100 ml Tetrahydrofuran bei 0°C hergestellt hat. Nach beendeter Zugabe dekantiert man vom Niederschlag und wäscht diesen mit Wasser. Man löst den Niederschlag in 250 ml warmer Ameisensäure und erhitzt anschließend 2 Stunden auf 50°C. Danach gießt man in 3 Liter Wasser, dialysiert und unterwirft das Retentat einer Gefriertrocknung. Man erhält feine weiße Kristalle in einer Ausbeute von 9,07 g.

| | | | |
|---|---|---|---|
| Analyse: | C 52,25 | H 6,15 | N 10,63 |

Der Gadolinium-Komplex und dessen Salze werden, wie in Beispiel 1j beschrieben, erhalten.
**Gadolinium-Komplex**

| | | | |
|---|---|---|---|
| C 42,51 | H 4,65 | N 8,68 | Gd 19,04 |

**Natrium-Salz**

| | | | | |
|---|---|---|---|---|
| C 40,55 | H 4,20 | N 8,20 | Na 5,28 | Gd 18,07 |

**N-Meglumin-Salz**

| | | | |
|---|---|---|---|
| C 43,63 | H 4,40 | N 8,34 | Gd 13,16 |

### BEISPIEL 30

### Poly-[4-〈2,6-di-(bis-(carboxymethyl))-amino]-4-(carboxymethyl)-aza-hexyl]-phenoxyacetyl〉-polylysin-poly-[2-(maleimido)-ethylenamid]

Analog der für Beispiel 2 angegebenen Vorschrift werden 4,3 g des nach Beispiel 29 erhaltenen Komplexbildners umgesetzt. Man erhält 4,1 g der Titelverbindung.
Maleimid-Gehalt (UV) 0,35 Gew%

| | | | |
|---|---|---|---|
| Analyse: | C 52,41 | H 6,16 | N 10,64 |

Der Gadolinium-Komplex und dessen Salze werden, wie in Beispiel 1j beschrieben, erhalten.
**Gadolinium-Komplex**

| | | | |
|---|---|---|---|
| C 44,42 | H 4,66 | N 8,68 | Gd 18,84 |

**Natrium-Salz**

| | | | | |
|---|---|---|---|---|
| C 40,60 | H 4,17 | N 8,28 | Na 5,25 | Gd 17,95 |

**N-Meglumin-Salz**

| | | | |
|---|---|---|---|
| C 43,23 | H 4,38 | N 8,33 | Gd 13,01 |

### BEISPIEL 31

### Poly-〈4-[2,6-di-(bis-(carboxymethyl)-amino)-4-(carboxymethyl)-aza-hexyl]-phenoxyacetyl〉-polylysin-poly-hydrazid

Analog der für Beispiel 3 angegebenen Vorschrift werden 4,6 g des nach Beispiel 29 erhaltenen Komplexbildners umgesetzt. Man erhält 4,5 g der Titelverbindung.
Hydrazin-Gehalt (colorim.) 0,52 Gew%

| | | | |
|---|---|---|---|
| Analyse: | C 52,29 | H 6,13 | N 10,63 |

Der Gadolinium-Komplex und dessen Salze werden, wie in Beispiel 1j beschrieben, erhalten.
**Gadolinium-Komplex**

| | | | |
|---|---|---|---|
| C 42,47 | H 4,64 | N 8,67 | Gd 19,03 |

**Natrium-Salz**

| | | | | |
|---|---|---|---|---|
| C 40,34 | H 4,20 | N 8,21 | Na 5,25 | Gd 17,93 |

**N-Meglumin-Salz**

| | | | |
|---|---|---|---|
| C 43,59 | H 4,37 | N 8,32 | Gd 13,14 |

### BEISPIEL 32

### Poly-〈4-[2,6-di-(bis-(carboxymethyl)-amino)-4-(carboxymethyl)-aza-hexyl]-phenoxyacetal〉-polylysin-[10-(hydrazinocarboxyl)-decylamid]

Analog der für Beispiel 4 angegebenen Vorschrift werden 5,3 g des nach Beispiel 29 erhaltenen Komplexbildners umgesetzt. Man erhält 5,4 g der Titelverbindung.
Hydrazin-Gehalt (colorim.) 0,31 Gew%

| | | | |
|---|---|---|---|
| Analyse: | C 52,3 | H 6,17 | N 10,69 |

Der Gadolinium-Komplex und dessen Salze werden, wie in Beispiel 1j beschrieben, erhalten.
**Gadolinium-Komplex**

| | | | |
|---|---|---|---|
| C 42,77 | H 4,65 | N 8,70 | Gd 18,84 |

**Natrium-Salz**

| | | | | |
|---|---|---|---|---|
| C 40,41 | H 4,18 | N 8,29 | Na 5,24 | Gd 17,87 |

**N-Meglumin-Salz**

| | | | |
|---|---|---|---|
| C 43,34 | H 4,40 | N 8,33 | Gd 13,09 |

### BEISPIEL 33

### Poly-{3-〈4-[2,6-di-(bis-(carboxymethyl)-amino)-4-(carboxymethyl)-aza-hexyl]-phenoxy〉-2-hydroxypropyl}-polylysin

Zu einer Lösung von 1,76 g (13,75 mMol Lysyleinheiten) Polylysin in 100 ml Wasser mit 1,39 g Triethylamin gibt man bei 0°C eine Lösung von 8,1 g (14,58 mMol) 3,6,9-Triaza-3,6,9-tris-(carboxymethyl)-4-[4-(oxiranylmethoxy)-benzyl]-undecandisäure (hergestellt aus dem entsprechenden Penta-(tert.-butylester) des Beispiels 5a durch Erwärmen mit Ameisensäure entsprechend der Vorschrift des Beispiels 1i) in 150 ml 0,1 n Natronlauge. Man läßt auf Raumtemperatur erwärmen, dialysiert und unterwirft das Retentat einer Gefriertrocknung. Man erhält 8,36 g feinnadliger, farbloser Substanz.

| | | | |
|---|---|---|---|
| Analyse: | C 55,27 | H 7,14 | N 11,62 |

Der Gadolinium-Komplex und dessen Salze werden, wie in Beispiel 1j beschrieben, erhalten.
**Gadolinium-Komplex**

| | | | |
|---|---|---|---|
| C 45,01 | H 5,43 | N 9,41 | Gd 19,26 |

**Natrium-Salz**

| | | | | |
|---|---|---|---|---|
| C 43,80 | H 5,17 | N 9,18 | Na 2,74 | Gd 18,81 |

**N-Meglumin-Salz**

| | | | |
|---|---|---|---|
| C 44,79 | H 5,14 | N 4,05 | Gd 15,75 |

### BEISPIEL 34

### Poly-{3-〈4-[2,6-di-(bis-(carboxymethyl)-amino)-4-(carboxymethyl)-aza-hexyl]-phenoxy〉-2-hydroxypropyl}-polylysin-poly-[2-(maleimido)-ethylenamid]

Analog der für Beispiel 2 angegebenen Vorschrift werden 5,3 g des nach Beispiel 33 erhaltenen Komplexbildners umgesetzt. Man erhält 5,1 g der Titelverbindung.
Maleimid-Gehalt (UV) 0,44 Gew%

| | | | |
|---|---|---|---|
| Analyse: | C 55,43 | H 7,11 | N 25,90 |

Der Gadolinium-Komplex und dessen Salze werden, wie in Beispiel 1j beschrieben. erhalten.
**Gadolinium-Komplex**

| | | | |
|---|---|---|---|
| C 45,02 | H 5,43 | N 9,43 | Gd 19,14 |

**Natrium-Salz**

| | | | | |
|---|---|---|---|---|
| C 43,85 | H 5,14 | N 9,16 | Na 2,74 | Gd 18,73 |

**N-Meglumin-Salz**

| | | | |
|---|---|---|---|
| C 44,92 | H 5,13 | N 9,08 | Gd 15,65 |

### BEISPIEL 35

### Poly-{3-〈4-[2,6-di-(bis-(carboxymethyl)-amino)-4-(carboxymethyl)-aza-hexyl]-phenoxy〉-2-hydroxypropyl}-polylysin-poly-hydrazid

Analog der für Beispiel 3 angegebenen Vorschrift werden 4,7 g des nach Beispiel 33 erhaltenen Komplexbildners umgesetzt. Man erhält 4,6 g der Titelverbindung.
Hydrazin-Gehalt (colorim.) 0,12 Gew%

| | | | |
|---|---|---|---|
| Analyse: | C 55,19 | H 7,12 | N 11,59 |

Der Gadolinium-Komplex und dessen Salze werden, wie in Beispiel 1j beschrieben, erhalten.
**Gadolinium-Komplex**

| | | | |
|---|---|---|---|
| C 44,77 | H 5,40 | N 9,43 | Gd 19,17 |

**Natrium-Salz**

| | | | | |
|---|---|---|---|---|
| C 43,83 | H 5,14 | N 9,20 | Na 2,75 | Gd 18,67 |

**N-Meglumin-Salz**

| | | | |
|---|---|---|---|
| C 45,08 | H 5,11 | N 9,10 | Gd 15,66 |

### BEISPIEL 36

### Poly-{3-〈4-[2,6-di-(bis-(carboxymethyl)-amino)-4-(carboxymethyl)-aza-hexyl]-phenoxy〉-2-hydroxypropyl}-polylysin-poly-[10-(hydrazinocarbonyl)-decylamid]

Analog der für Beispiel 4 angegebenen Vorschrift werden 3,8 g des nach Beispiel 33 erhaltenen Komplexbildners umgesetzt. Man erhält 3,9 g der Titelverbindung.
Hydrazin-Gehalt (colorim.) 0,14 Gew%

| | | | |
|---|---|---|---|
| Analyse: | C 55,54 | H 7,18 | N 11,64 |

Der Gadolinium-Komplex und dessen Salze werden, wie in Beispiel 1j beschrieben, erhalten.
**Gadolinium-Komplex**

| | | | |
|---|---|---|---|
| C 45,00 | H 5,46 | N 9,46 | Gd 19,08 |

**Natrium-Salz**

| | | | | |
|---|---|---|---|---|
| C 43,82 | H 5,16 | N 9,20 | Na 2,73 | Gd 18,64 |

**N-Meglumin-Salz**

| | | | |
|---|---|---|---|
| C 45,17 | H 5,14 | N 9,10 | Gd 15,69 |

### BEISPIEL 37

### Poly-N⁶-[10-carboxy-3,6,9-tris-(carboxymethyl)-3,6,9-triazadecanoyl]-poly-L-lysin

632 mg (4,9 mMol monomere Untereinheiten) Polylysin werden in 50 ml Wasser mit 5 ml 1 n Natronlauge gelöst und unter Eiskühlung portionsweise mit 2,1 g (5,2 mMol) N³-(2,6-Dioxomorpholinoethyl)-N⁶-(ethoxycarbonylmethyl)-3,6-diazaoctandisäure (Beispiel 13a) versetzt, wobei der pH-Wert mit 1 n Natronlauge auf über 9 gehalten wird. Man läßt über Nacht unter Rühren auf Raumtemperatur aufwärmen und dialysiert. Nach Gefriertrocknung liegen 2,05 g farblose kristalline Nädelchen vor.

| | | | |
|---|---|---|---|
| Analyse: | C 48,06 | H 6,61 | N 14,21 |

Der Gadolinium-Komplex und dessen Salze werden, wie in Beispiel 1j beschrieben, erhalten.
**Gadolinium-Komplex**

| | | | |
|---|---|---|---|
| C 36,99 | H 4,66 | N 10,91 | Gd 23,34 |

**Natrium-Salz**

| | | | | |
|---|---|---|---|---|
| C 35,80 | H 4,35 | N 10,63 | Na 3,27 | Gd 22,44 |

**N-Meglumin-Salz**

| | | | |
|---|---|---|---|
| C 38,78 | H 4,46 | N 10,19 | Gd 18,20 |

### BEISPIEL 38

### Poly-N⁶-[10-carboxy-3,6,9-tris-(carboxymethyl)-3,6,9-triazadecanoyl]-poly-L-lysin-poly-[2-(maleimido)-ethylenamid]

Analog der für Beispiel 2 angegebenen Vorschrift werden 2,7 g des nach Beispiel 37 erhaltenen Komplexbildners umgesetzt. Man erhält 2,4 g der Titelverbindung.
Maleimid-Gehalt (UV) 0,88 Gew%

| | | | |
|---|---|---|---|
| Analyse: | C 48,10 | H 6,61 | N 14,26 |

Der Gadolinium-Komplex und dessen Salze werden, wie in Beispiel 1j beschrieben, erhalten.
**Gadolinium-Komplex**

| | | | |
|---|---|---|---|
| C 37,12 | H 4,64 | N 11,02 | Gd 23,14 |

**Natrium-Salz**

| | | | | |
|---|---|---|---|---|
| C 35,87 | H 4,36 | N 10,62 | Na 3,29 | Gd 22,99 |

**N-Meglumin-Salz**

| | | | |
|---|---|---|---|
| C 38,83 | H 4,49 | N 10,28 | Gd 18,15 |

### BEISPIEL 39

a) Poly-N⁶-[10-carboxy-3,6-bis-(carboxymethyl)-9-ethoxycarbonylmethyl-3,6,9-triazadecanoyl]-poly-L-lysin
   0,82 g (5 mmol) poly-L-lysin-Hydrochlorid werden in 100 ml Wasser gelöst. Unter Einhaltung eines pH-Wertes von 9,5 werden portionsweise 6,06 g (15 mmol) N³-(2,6-Dioxomorpholinoethyl)-N⁶-(ethoxycarbonylmethyl)-3,6-diazaoctandisäure (Beispiel 13a) zugegeben, mit ca. 11 ml 1 N Salzsäure auf pH 7 gestellt und über eine Ultrafiltrationsmembran (Amicon YM2) entsalzt und anschließend gefrierge- trocknet.
   Ausbeute: 2,6 g (90% der Theorie);
   Ethoxy-Bestimmung: 6,85%, das entspricht einer Acylierung des poly-Lysins von 88%.
   Schmelzpunkt: 247°C (Zersetzung)
   1 g dieser Verbindung komplexieren 240 mg Gd ³⁺.
b) Poly-N⁶-[10-carboxy-3,6,9-tris-(carboxymethyl)-3,6,9-triazadecanoyl]-poly-L-lysin-polyhydrazid
   2,4 g (4,2 mmol) des in Beispiel 39a beschriebenen Ethylesters werden in 100 ml Wasser gelöst und nach Zugabe von 5 ml (0,5 mmol) 0,1 M Hydrazinhydratlösung 4 Stunden am Rückfluß und über Nacht bei Raumtemperatur gerührt. Bei einem pH-Wert von über 9 wird ultrafiltriert, die Restlösung nach Zugabe von Amberlite IR 120(H⁺) auf einen pH-Wert von 4 eingestellt und gefriergetrocknet.
   Ausbeute: 2 g
   Hydrazid-Gehalt: 0,3 Mol%
c) Gadolinium-Komplex des
   Poly-N⁶-[10-carboxy-3,6,9-tris-(carboxymethyl)-3,6,9-triazadecanoyl]-poly-L-lysin-polyhydrazids
   1,9 g des in Beispiel 39b beschriebenen Komplexbildners werden in 200 ml Wasser gelöst, mit 548 mg Gd₂0₃ = 475 mg Gd³⁺ versetzt und eine Stunde bei 80°C gerührt; die erhaltene Lösung wird ultrafiltriert und anschließend gefriergetrocknet.
   Ausbeute: 2,35 g
   Gd-Gehalt: 20 Gew%
   λmax (H₂O) = 201 nm (ε = 9.000)
   Es wurden folgende Relaxivitäten gemessen [die Messungen der Relaxationszeiten T1 und T2 erfolgten in einem Minispec p 20 (Bruker) bei 0,46 Tesla (= 20 MHz), 37°C]:
   T₁-Relaxivität: 11,38 (L/mmol sec)
   T₂-Relaxivität: 13,13 (L/mmol sec)

### BEISPIEL 40

### Poly-N⁶-[10-carboxy-3,6,9-tris-(carboxymethyl)-3,6,9-triazadecanoyl]-poly-L-lysin-poly-[10-(hydrazino-carbonyl)-decylamid]

Analog der für Beispiel 4 angegebenen Vorschrift werden 2,9 g des nach Beispiel 37 hergestellten Komplexbildners umgesetzt. Man erhält 3,0 g der Titelverbindung.
Hydrazin-Gehalt (colorim.) 0,41 Gew%

| | | | |
|---|---|---|---|
| Analyse: | C 48,3 | H 6,60 | N 14,23 |

**Gadolinium-Komplex**

| | | | |
|---|---|---|---|
| C 37,10 | H 4,68 | N 10,96 | Gd 23,31 |

**Natrium-Salz**

| | | | | |
|---|---|---|---|---|
| C 35,84 | H 4,39 | N 10,67 | Na 3,28 | Gd 22,49 |

**N-Meglumin-Salz**

| | | | |
|---|---|---|---|
| C 38,87 | H 4,48 | N 10,29 | Gd 18,27 |

### BEISPIEL 41

a) 3,6,9-Triaza-3,6,9-tris-(tert.-butoxycarbonylmethyl)-4-[4-(3-benzyloxycarbonylaminopropoxy)-benzyl)]-undecandisäure-bis-(tert.-butyl)-diester
4,6 g (5,90 mMol) der Verbindung nach Beispiel 1f werden mit 194 mg NaH (80%ig in Paraffin) (6,48 mMol) in 40 ml trockenem Tetrahydrofuran zusammengegeben und dazu langsam 1,6 g N-(3-Brompropyl)-carbaminsäurebenzylester in 20 ml Tetrahydrofuran zugetropft. Nach Rühren über Nacht wird eingeengt und über eine Kieselgelsäule vom Paraffinöl abgetrennt. Man erhält nach Abdampfen des Lösungsmittels 4,2 g eines farblosen Öls (Ausbeute 74% der Theorie).

| | | | | | |
|---|---|---|---|---|---|
| Analyse: | Ber.: | C 64,30 | H 8,51 | N 5,76 | O 21,41 |
| | Gef.: | C 64,20 | H 8,65 | N 5,82 | |

b) 3,6,9-Triaza-3,6,9-tris-(tert.-butoxycarbonylmethyl)-4-[4-(3-aminopropoxy)-benzyl]-undecandisäure-bis-(tert.-butyl)-diester
3,9 g (4,8 mMol) 3,6,9-Triaza-3,6,9-tris-(tert.-butoxycarbonylmethyl)-4-[4-(3-benzyloxycarbonylaminopropoxy)-benzyl)]-undecandisäure-bis-(tert.-butyl)-diester (Beispiel 41a) werden in 100 ml Methanol gelöst und mit 2,13 g 10% Palladium-Kohle hydriert, bis keine weitere H₂-Aufnahme erfolgt. Danach wird vom Katalysator abfiltriert. Das zurückbleibende farblose Öl wiegt 3,17 g (97,3 % der Theorie).

| | | | | | |
|---|---|---|---|---|---|
| Analyse: | Ber.: | C 63,13 | H 9,15 | N 6,69 | O 21,02 |
| | Gef.: | C 62,97 | H 9,01 | N 6,62 | |

c) Polyacrylpoly-〈3-(4-{2,6-di-(bis-(carboxymethyl)-amino)-4-((carboxymethyl)-aza)-hexyl]-phenoxy}-propyl〉-amid
Zu einer Lösung von 10,1 g Polyacrylsäurepolyethylester in 100 ml Toluol gibt man tropfenweise bei 60 - 80°C eine Lösung von 83,7 g (100 mMol) der in der vorigen Reaktionsstufe (41b) hergestellten Verbindung in 100 ml Toluol und hält 20 Stunden bei dieser Temperatur. Man zieht das Lösungsmittel ab und erwärmt 10 Stunden mit 1 Liter Trifluoressigsäure. Nach dem Verdünnen mit 10 Liter Wasser wird dialysiert und portionsweise gefriergetrocknet. Man erhält insgesamt 45,46 g einer farblosen, faserig-kristallinen Polymers.

| | | | |
|---|---|---|---|
| Analyse: | C 51,08 | H 6,69 | N 9,45 |

**Gadolinium-Komplex**

| | | | |
|---|---|---|---|
| C 40,69 | H 4,91 | N 7,47 | Gd 20,97 |

**Natrium-Salz**

| | | | | |
|---|---|---|---|---|
| C 38,17 | H 4,36 | N 7,07 | Na 5,81 | Gd 19,94 |

**N-Meglumin-Salz**

| | | | |
|---|---|---|---|
| C 42,13 | H 4,55 | N 7,52 | Gd 14,05 |

### BEISPIEL 42

### Polyacrylpoly-〈3-{4-[2,6-di-(bis-(carboxymethyl)-amino)-4-((carboxymethyl)-aza)-hexyl]-phenoxy}-propyl〉-amid-poly-[2-(maleimido)-ethylamid]

Analog der für Beispiel 2 angegebenen Vorschrift werden 6,2 g des nach Beispiel 41 hergestellten Komplexbildners umgesetzt. Man erhält 6,0 g der Titelverbindung.
Maleimid-Gehalt (UV) 0,44 Gew%

| | | | |
|---|---|---|---|
| Analyse: | C 51,22 | H 6,7 | N 9,45 |

**Gadolinium-Komplex**

| | | | |
|---|---|---|---|
| C 40,61 | H 4,91 | N 7,54 | Gd 20,99 |

**Natrium-Salz**

| | | | | |
|---|---|---|---|---|
| C 38,36 | H 4,36 | N 7,10 | Na 5,83 | Gd 19,88 |

**N-Meglumin-Salz**

| | | | |
|---|---|---|---|
| C 42,35 | H 4,54 | N 7,53 | Gd 14,04 |

### BEISPIEL 43

### Polyacrylpoly-〈3-{4-[2,6-di-(bis-(carboxymethyl)-amino)-4-((carboxymethyl)-aza)-hexyl]-phenoxy}-propyl〉-amid-poly-[10-(hydrazinocarbonyl)-decylamid]

Analog der für Beispiel 4 angegebenen Vorschrift werden 4,3 g des nach Beispiel 41 hergestellten Komplexbildners umgesetzt, Man erhält 4,4 g der Titelverbindung.
Hydrazin-Gehalt (colorim.) 0,56 Gew%

| | | | |
|---|---|---|---|
| Analyse: | C 51,26 | H 6,73 | N 9,54 |

**Gadolinium-Komplex**

| | | | |
|---|---|---|---|
| C 40,61 | H 4,94 | N 7,58 | Gd 20,91 |

**Natrium-Salz**

| | | | | |
|---|---|---|---|---|
| C 38,35 | H 4,37 | N 7,15 | Na 5,81 | Gd 19,76 |

**N-Meglumin-Salz**

| | | | |
|---|---|---|---|
| C 42,17 | H 4,54 | N 7,57 | Gd 14,07 |

### BEISPIEL 44

### Polyacrylpoly-〈3-{4-[2,6-di-(bis-(carboxymethyl)-amino)-4-((carboxymethyl)-aza)-hexyl]-phenoxy}-propyl〉-amid-poly-hydrazid

Eine Lösung Von 3,5 g Polyacrylsäurepolyethylester in 500 ml Toluol wird mit einer Lösung von 29 g (34 mMol) der Titelverbindung von Beispiel 41a und 5 ml einer Tetrahydrofuran-Lösung, die 150 mg Hydrazin pro Liter enthielt, gleichzeitig bei Raumtemperatur versetzt. Man erwärmt langsam auf 80°C und verfährt dann so wie in Beispiel 41c beschrieben.
Ausbeute: 15,39 g (farblose Kristalle)

| | | | |
|---|---|---|---|
| Analyse: | C 50,90 | H 6,95 | N 9,43 |

**Gadolinium-Komplex**

| | | | |
|---|---|---|---|
| C 40,38 | H 4,88 | N 7,49 | Gd 21,04 |

**Natrium-Salz**

| | | | | |
|---|---|---|---|---|
| C 38,43 | H 4,37 | N 7,10 | Na 5,81 | Gd 19,87 |

**N-Meglumin-Salz**

| | | | |
|---|---|---|---|
| C 41,94 | H 4,53 | N 7,50 | Gd 14,12 |

### BEISPIEL 45

a) 3,6,9-Triaza-3,6,9-tris-(tert.-butoxycarbonylmethyl)-4-[3-(benzyloxycarbonyl)-aminopropoxymethyl]-undecandisäure-bis-(tert.-butyl)-diester
Ausgehend von 3,6,9-Triaza-3,6,9-tris-(tert.-butoxycarbonylmethyl)-4-hydroxymethyl-undecandisäure-bis-(tert.-butyl)-diester (Beispiel 25d) wird die Titelverbindung analog der Vorschrift für Beispiel 41a in 71%iger Ausbeute erhalten.

| | | | | |
|---|---|---|---|---|
| Analyse: | Ber.: | C 61,71 | H 8,78 | N 6,26 |
| | Gef.: | C 61,65 | H 8,83 | N 6,35 |

b) 3,6,9-Triaza-3,6,9-tris-(tert.-butoxycarbonylmethyl)-4-[3-aminopropoxymethyl]-undecandisäure-bis-(tert.-butyl)-diester
Ausgehend von der in der vorigen Reaktionsstufe (Beispiel 45a) erhaltenen benzyloxycarbonyl-geschützten Aminoverbindung wird die Titelverbindung analog der für das Beispiel 41b angegebenen Vorschrift in 93%iger Ausbeute erhalten.

| | | | | |
|---|---|---|---|---|
| Analyse: | Ber.: | C 59,97 | H 9,53 | N 7,36 |
| | Gef.: | C 60,11 | H 9,52 | N 7,44 |

c) Polyethyleniminpolyessigsäurepolymethylester
8,18 g Polyethylenimin (186,5 mMol monomere Untereinheiten), 20,1 g Triethylamin (200 mMol) und 30 g Bromessigsäuremethylester (196 mMol) werden unter Eiskühlung in 250 ml Methanol gemischt und über Nacht auf Raumtemperatur erwärmt. Man entfernt das Lösungsmittel und extrahiert mehrmals mit warmem Methylenchlorid. Nach dem Trocknen hinterbleiben 19 g Öl.

| | | | |
|---|---|---|---|
| Analyse: | C 60,03 | H 9,13 | N 14,14 |

d) Polyethyleniminpolyessigsäurepoly-〈3-{[2,6-di-(bis-(carboxymethyl)-amino)-4-(carboxymethyl)-aza)-hexyl]-methoxy}-propyl〉-amid
3,65 g Polyethyleniminpolyessigsäurepolymethylester (Beispiel 45c) werden in 50 ml Methanol gelöst und mit einer Lösung von 31 g (42 mMol) der Titelverbindung von Beispiel 45b versetzt. Man erwärmt 10 Stunden auf 60°C, zieht das Lösungsmittel ab und spaltet die Ester mit Ameisensäure wie für Beispiel 1i beschrieben. Nach Dialyse und Gefriertrocknung liegen 15,07 g des Komplexbildners in Form feiner kristalliner Nadeln vor.

| | | | |
|---|---|---|---|
| Analyse: | C 43,24 | H 7,07 | N 13,28 |

**Gadolinium-Komplex**

| | | | |
|---|---|---|---|
| C 33,66 | H 5,04 | N 10,27 | Gd 22,86 |

**Natrium-Salz**

| | | | | |
|---|---|---|---|---|
| C 31,40 | H 4,44 | N 9,64 | Na 6,29 | Gd 21,59 |

**N-Meglumin-Salz**

| | | | |
|---|---|---|---|
| C 37,51 | H 4,61 | N 9,34 | Gd 14,96 |

### BEISPIEL 46

### Polyethyleniminpolyessigsäurepoly-〈3-{[2,6-di-(bis-(carboxymethyl)-amino)-4-((carboxymethyl)-aza)-hexyl]-oxy}-propyl〉-amid-polyhydrazid

3,12 g Polyethyleniminpolyessigsäurepolymethylester (Beispiel 45c) werden in 100 ml Methanol gelöst und mit 1 ml einer Lösung, die 50 mg Hydrazinhydrat auf 100 ml enthielt, versetzt. Man erwärmt 30 Minuten auf 40°C und fügt dann 26 g (35,2 mMol) der Titelverbindung von Beispiel 45b gelöst in 50 ml Methanol hinzu. Die weitere Bearbeitung erfolgt wie für Beispiel 41c beschrieben. Man erhält 12,17 g feinkristalline Substanz.
Hydrazingehalt: 0,13 Gew%

| | | | |
|---|---|---|---|
| Analyse: | C 43,35 | H 7,10 | N 13,25 |

**Gadolinium-Komplex**

| | | | |
|---|---|---|---|
| C 33,45 | H 5,04 | N 10,31 | Gd 22,97 |

**Natrium-Salz**

| | | | | |
|---|---|---|---|---|
| C 31,52 | H 4,45 | N 9,68 | Na 6,28 | Gd 21,61 |

**N-Meglumin-Salz**

| | | | |
|---|---|---|---|
| C 37,62 | H 4,61 | N 9,30 | Gd 14,91 |

### BEISPIEL 47

### Polyethyleniminpolyessigsäurepoly-〈3-{[2,6-di-(bis-(carboxymethyl)-amino)-4-((carboxymethyl)-aza)-hexyl]-oxy}-propyl〉-amid-poly-[2-(maleimido)-ethylamid]

Ersetzt man in Beispiel 46 das Hydrazin durch eine äquivalente Menge 2-(Maleimido)-ethylamin (das in Form seines Trifluoracetats zugegeben wird) so erhält man das entsprechende Polymer mit Maleimid-Funktionen.
Maleimid-Gehalt (UV): 0,38 Gew%

| | | | | |
|---|---|---|---|---|
| Analyse: | C 43,05 | H 7,10 | N 13,31 | 36,46 |

**Gadolinium-Komplex**

| | | | |
|---|---|---|---|
| C 33,45 | H 5,05 | N 10,28 | Gd 22,95 |

**Natrium-Salz**

| | | | | |
|---|---|---|---|---|
| C 31,52 | H 4,46 | N 9,64 | Na 6,30 | Gd 21,56 |

**N-Meglumin-Salz**

| | | | |
|---|---|---|---|
| C 37,79 | H 4,61 | N 9,33 | Gd 14,90 |

### BEISPIEL 48

### Polyethyleniminpolyessigsäurepoly-〈3-{[2,6-di-(bis-(carboxymethyl)-amino)-4-((carboxymethyl)-aza)-hexyl]-oxy}-propyl〉-amid-poly-[10-(hydrazinocarbonyl)-decylamid]

Ersetzt man in Beispiel 46 das Hydrazin durch eine äquivalente Menge 11-Aminoundecansäure-(2-tert.-butoxycarbonyl)-hydrazid, so erhält man den mit Hydrazid als funktionelle Gruppe versehenen Komplex.
Hydrazin-Gehalt: 0,77 Gew%

| | | | |
|---|---|---|---|
| Analyse: | C 43,30 | H 7,09 | N 13,36 |

**Gadolinium-Komplex**

| | | | |
|---|---|---|---|
| C 33,62 | H 5,07 | N 10,32 | Gd 22,92 |

**Natrium-Salz**

| | | | | |
|---|---|---|---|---|
| C 31,63 | H 4,48 | N 9,74 | Na 6,30 | Gd 21,50 |

**N-Meglumin-Salz**

| | | | |
|---|---|---|---|
| C 37,68 | H 4,61 | N 9,38 | Gd 14,94 |

### BEISPIEL 49

### Polyethyleniminpolyessigsäurepoly-〈3-[4,5-di-(bis-(carboxymethyl)-amino)-2-hydroxy-cyclohexyl]-3-sulfapropyl〉-amid

2,15 g (4,9 mMol) 1,2-Bis-[di-(carboxymethyl)-amino]-5-(3-amino-1-thiapropyl)-4-hydroxy-cyclohexan (Europäische Patentanmeldung Publikations-Nr. 0154788) werden mit 0,5 g Triethylamin in 50 ml Methanol gelöst und mit einer Lösung von 470 mg (4,75 mMol monomere Untereinheiten) Polyethyleniminpolyessigsäurepolymethylester in 30 ml Methanol vereinigt. Nach 18 Stunden bei 60°C wird das Methanol abgedampft, in Wasser gelöst, dialysiert und das Retentat gefriergetrocknet.
Ausbeute: 2,21 g, weiße Plättchen, die oberhalb 85°C sintern.

| | | | | |
|---|---|---|---|---|
| Analyse: | C 44,25 | H 7,43 | N 12,91 | S 5,88 |

**Gadolinium-Komplex**

| | | | | |
|---|---|---|---|---|
| C 34,47 | H 5,36 | N 10,07 | S 4,6 | Gd 22,53 |

**Natrium-Salz**

| | | | | | |
|---|---|---|---|---|---|
| C 33,57 | H 5,05 | N 9,72 | Na 3,18 | S 4,43 | Gd 21,88 |

**N-Meglumin-Salz**

| | | | | |
|---|---|---|---|---|
| C 36,82 | H 5,05 | N 9,55 | S 3,63 | Gd 17,77 |

### BEISPIEL 50

### Polyethyleniminpolyessigsäurepoly-〈3-[4,5-di-(bis-(carboxymethyl)-amino)-2-hydroxy-cylohexyl]-3-sulfapropyl〉-amid-poly-[2-(maleimido)-ethylamid]

Die Herstellung der Titelverbindung erfolgt analog der Vorschrift für Beispiel 47 durch Umsetzung von Polyethyleniminpolyessigsäurepolymethylester mit 2-(Maleimido)-ethylamin und 1,2-Bis-[di(carboxymethyl)-amino)-5-(3-amino-1-thiapropyl)-4-hydroxycyclohexan.
Maleimid-Gehalt (UV) 0,51 Gew%

| | | | | |
|---|---|---|---|---|
| Analyse: | C 44,43 | H 7,39 | N 12,99 | S 5,84 |

**Gadolinium-Komplex**

| | | | | |
|---|---|---|---|---|
| C 34,61 | H 5,33 | N 10,05 | S 4,58 | Gd 22,38 |

**Natrium-Salz**

| | | | | | |
|---|---|---|---|---|---|
| C 33,49 | H 5,04 | N 9,79 | Na 3,16 | S 4,44 | Gd 21,86 |

**N-Meglumin-Salz**

| | | | | |
|---|---|---|---|---|
| C 36,96 | H 5,05 | N 9,55 | S 3,62 | Gd 17,80 |

### BEISPIEL 51

### Polyethyleniminpolyessigsäurepoly-〈3-[4-[4,5-di-(bis-(carboxymethyl)-amino-2-hydroxy-cyclohexyl]-3-sulfapropyl〉-amid-poly-hydrazid

Die Herstellung der Titelverbindung erfolgt analog der Vorschrift für Beispiel 46 durch Umsetzung von Polyethyleniminopolyessigsäurepolymethylester mit Hydrazinhydrat und 1,2-Bis-[di(carboxymethyl)-amino-5-(3-amino-1-thiapropyl)-4-hydroxycyclohexan.
Hydrazin-Gehalt (colorim.) 0,17 Gew%

| | | | | |
|---|---|---|---|---|
| Analyse: | C 44,14 | H 7,44 | N 12,87 | S 5,88 |

**Gadolinium-Komplex**

| | | | | |
|---|---|---|---|---|
| C 34,34 | H 5,36 | N 10,04 | S 4,6 | Gd 22,55 |

**Natrium-Salz**

| | | | | | |
|---|---|---|---|---|---|
| C 33,60 | H 5,07 | N 9,80 | Na 3,17 | S 4,45 | Gd 21,89 |

**N-Meglumin-Salz**

| | | | | |
|---|---|---|---|---|
| C 36,73 | H 5,05 | N 9,58 | S 3,61 | Gd 17,75 |

### BEISPIEL 52

### Biotinylierter Gadoliniumkomplex des Poly-N-[10-carboxy-3,6,9-tris(carboxymethyl)-3,6,9-triazadecanoyl]-polyethylenimin-polyhydrazids

572 mg (1 mmol) des in Beispiel 15 beschriebenen Gadoliniumkomplexes werden in 20 ml Wasser mit 220 mg (0,5 mmol) Sulfo-NHS-Biotin (Pierce Chemical Company) versetzt und durch Zugabe von NaHCO₃ bei pH 8-9 gehalten. Nach mehrstündigem Rühren bei Raumtemperatur verdünnt man mit Wasser und dialysiert, bis im Filtrat kein Biotin mehr nachweisbar ist. Nach Gefriertrocknung erhält man 520 mg farblose Substanz. Der Biotin-Gehalt wird colorimetrisch ermittelt (N.M. Green, Methods in Enzymology XVIII, Part A (1970), 418-424): 0,39 Mol%

### BEISPIEL 53

a) 3,6 Diaza-3,6-bis-(tert.-butoxycarbonylmethyl)-4-(4-benzyloxycarbonylmethoxybenzyl)-suberinsäure-bis-(tert-butyl)-diester
93,06 g der nach Beispiel 21a erhaltenen Substanz (0,15 Mol) werden mit 4,48 g NaH (80% in Paraffin) (0,15 Mol) in 600 ml trockenem Tetrahydrofuran unter Rühren langsam zusammengegeben und dann bei Raumtemperatur 34,4 g Bromessigsäurebenzylester (0,15 Mol) in 150 ml trockenem Tetrahydrofuran zugetropft. Nach Rühren über Nacht saugt man von ausgefallenem Natriumbromid ab, engt ein, nimmt in Diethylether auf und entfernt die übrigen anorganischen Bestandteile durch Waschen mit Wasser. Nach Trocknen mit MgSO₄ wird vom Lösungsmittel befreit und über eine Kieselgelsäule gereinigt. Man erhält 75,2 g (65% der Theorie) eines farblosen Öls.

| | | | | | |
|---|---|---|---|---|---|
| Analyse: | Ber.: | C 65,43 | H 8,10 | N 3,63 | O 22,82 |
| | Gef.: | C 65,23 | H 8,17 | N 3,58 | |

b) 3,6 Diaza-3,6-bis-(tert.-butoxycarbonylmethyl)-4-(4-carboxymethoxybenzyl)-suberinsäure-bis-(tert-butyl)-diester
9,5 g der in der vorigen Reaktionsstufe erhaltenen Verbindung (0,012 Mol) werden in 100 ml trockenem Tetrahydrofuran gelöst und in Gegenwart von 2 g 10% Pd/C hydriert, bis keine weitere Wasserstoffaufnahme stattfindet. Nach Absaugen wird das Lösungsmittel am Rotationsverdampfer entfernt und die Substanz bei 0,01 Torr weitergetrocknet. Das erhaltene zähflüssige Öl wiegt 8,33 g (99% der Theorie).

| | | | | | |
|---|---|---|---|---|---|
| Analyse: | Ber.: | C 61,74 | H 8,29 | N 4,11 | O 25,84 |
| | Gef.: | C 61,82 | H 8,17 | N 4,12 | |

c) Poly-N⁶-{4-[2,3-(N,N,N',N'-tetrakis-(tert.-butoxycarbonylmethyl)-diamino)-propyl]-phenoxymethyl-carbonyl}-poly-L-lysin
9,54 g (14 mmol) der in Beispiel 53b beschriebenen Säure werden in DMF gelöst, bei -15°C mit 9,7 ml Triethylamin, 1,48 ml (15,4 mmol) Chlorameisensäure-ethylester und mit einer Lösung von 1,16 g (7 mmol) poly-L-Lysin-Hydrochlorid in Wasser versetzt. Die entstehende Suspension läßt man ohne weitere Kühlung rühren, der Niederschlag wird abgesaugt, mit DMF und Wasser gewaschen und anschließend getrocknet.
Ausbeute: 4 g (72% der Theorie)
Fp.: 212°C (Zersetzung)
d) Poly-N⁶-{4-[2,3-(N,N,N',N'-tetrakis-(carboxymethyl)-diamino)-propyl]-phenoxymethylcarbonyl]-poly-L-lysin, Natriumsalz
3,5 g des in Beispiel 53c beschriebenen Tetra-tertiär-Butylesters werden in 80 ml 24%iger wäßriger Bromwasserstofflösung suspendiert und 6 Tage bei Raumtemperatur gerührt. Anschließend wird die entstandene Lösung mit Natronlauge auf pH 7 eingestellt, die Resttrübung abfiltriert und das Filtrat ultrafiltriert (Amicon Ultrafiltrationsmembran YM2). Nach Gefriertrocknung werden 1,6 g erhalten.
Ausbeute: 74%
Fp.: >250°C
e) Poly-N⁶-{4-[2,3-(N,N,N',N'-tetrakis-(carboxymethyl)-diamino)-propyl]-phenoxymethylcarbonyl}-poly-L-lysin-polyhydrazid
1,2 g (2 mmol) des in Beispiel 53d beschriebenen Tetrasäure-Natriumsalzes werden in 60 ml Methanol/Wasser (1:1) gelöst. Nach Zugabe von 0,026 g (0,2 mmol) Dimethylsulfat wird 6 Stunden bei Raumtemperatur gerührt, anschließend mit 5 ml 80%iger Hydrazinhydratlösung versetzt und über Nacht gerührt. Freies Hydrazin wird über eine Ultrafiltration (pH >9) entfernt und die erhaltene Restlösung durch Zugabe von Amberlite IR 120 (H⁺) auf pH ca.4 gestellt, vom Ionenaustauscher abfiltriert und gefriergetrocknet.
Ausbeute: 1,0 g
Hydrazid-Gehalt (colorimetrisch): 4,8 Mol%
f) Gadolinium-Komplex des Poly-N⁶-{4-[2,3-(N,N,N',N'-tetrakis-(carboxymethyl)-diamino)-propyl]-phenoxymethylcarbonyl}-poly-L-lysin-polyhydrazids
1,0 g des in Beispiel 53e beschriebenen Komplexbildners in 100 ml Wasser wird mit 253 mg Gd₂O₃ versetzt und eine Stunde bei 80°C gerührt; die erhaltene Lösung wird ultrafiltriert und anschließend gefriergetrocknet.
Ausbeute: 1,1 g
Gd-Gehalt: 18,0 Gew%.
Es wurden folgende Relaxivitäten gemessen (die Messungen der Relaxationszeiten T1 und T2 erfolgten in einem Minispec p 20 (Bruker) bei 0,46 Tesla (= 20 MHz), 37°C):
T₁-Relaxivität: 24,08 (L/mmol sec)
T₂-Relaxivität: 30,24 (L/mmol sec)

### BEISPIEL 54

### Gd-Komplex des Poly-N⁶-[10-carboxy-3,6-bis-(carboxymethyl)-9-ethoxycarbonyl-methyl-3,6,9-triazadecanoyl]-poly-L-lysins

1,0 g des in Beispiel 39a beschriebenen Komplexbildners werden in 100 ml Wasser gelöst und wie in Beispiel 53f beschrieben mit 277 mg Gd₂O₃ = 240 mg Gd³⁺ komplexiert.
Ausbeute: 1,2 g
Gd-Gehalt: 19,3 Gew%. λmax (H₂O) = 201 nm (ε=9.000)
Es wurden folgende Relaxivitäten gemessen (die Messungen der Relaxationszeiten T1 und T2 erfolgten in einem Minispec p 20 (Bruker) bei 0,47 Tesla (= 20 MHz), 39°C):
T₁-Relaxivität: 9,61 (L/mmol sec)
T₂-Relaxivität: 10,56 (L/mmol sec)

### BEISPIEL 55

a) Poly-N⁶-[10-carboxy-3,6-bis-(carboxymethyl)-9-ethoxycarbonylmethyl-3,6,9-triazadecanoyl]-poly-L-lysin-poly-hydrazid
   2,4 g (4,2 mmol) des in Beispiel 39a beschriebenen Ethylesters werden analog der für Beispiel 39b gegebenen Vorschrift teilweise ins Hydrazid überführt und bei einem pH-Wert ≦9 wie dort beschrieben, aufgearbeitet.
   Ausbeute: 2 g
   Hydryzid-Gehalt: 0,3 Mol%, Ethoxybestimmung: 6,3%
b) Gd-Komplex des Poly-N⁶-[10-carboxy-3,6-bis-(carboxymethyl)-9-ethoxycarbonyl-methyl-3,6,9-triazadecanoyl]-poly-L-lysin-polyhydrazids
   1,9 g des in Beispiel a beschriebenen Komplexbildners werden analog Beispiel 53f mit 525 mg Gd₂O₃ = 455 mg Gd³⁺ komplexiert.
   Ausbeute: 2,3 g; Gd-Gehalt: 17,0 Gew%; Schmelzpunkt oberhalb 250°C

### BEISPIEL 56

a) Sebacinsäure-mono(N'-t-butoxycarbonyl-hydrazid)-monomethylester
10,81 g (50 mmol) Sebacinsäure-mono-methylester werden in Tetrahydrofuran gelöst und bei -5°C nacheinander mit 8,32 ml (60 mmol) Triethylamin und 5,28 ml (55 mmol) Chlorameisensäure-ethylester [Ethyl Chlorformiat] versetzt. Nach 15 Minuten werden bei dieser Temperatur 6,61 g (50 mmol) tert.-Butylcarbazat in Tetrahydrofuran zugetropft und anschließend 2 Stunden bei Raumtemperatur gerührt. Der Niederschlag wird abgesaugt, das Filtrat eingeengt und in Essigester aufgenommen. Die organische Phase wird nacheinander mit NaHCO₃-Lösung, Zitronensäure und Wasser gewaschen und über Na₂SO₄ getrocknet. Das nach Einengen des Essigesters erhaltene Öl wird an Kieselgel in Diisopropylether chromatographiert.
Ausbeute: 11,2 g (68% der Theorie)

| | | | | |
|---|---|---|---|---|
| Analyse: | Ber.: | C 58,16 | H 9,15 | N 8,48 |
| | Gef.: | C 58,25 | H 9,13 | N 8,50 |

b) Sebacinsäure-mono(N'-t-butoxycarbonyl-hydrazid)
9,9 g (30 mmol) des in Beispiel 58 a beschriebenen Methylesters werden mit 150 ml 1n NaOH-Lösung versetzt und eine Stunde bei Raumtemperatur gerührt. Die klare wäßrige Lösung wird mit Ether gewaschen, mit Zitronensäure angesäuert und der entstehende Niederschlag in Essigester aufgenommen. Nach Trocknung über Na₂SO₄ und Eindampfen des Lösungsmittels erhält man einen öligen Rückstand.
Ausbeute: 9,3 g (98% der Thgeorie)

| | | | | |
|---|---|---|---|---|
| Analyse: | Ber.: | C 56,94 | H 8,92 | N 8,86 |
| | Gef.: | C 56,88 | H 8,96 | N 8,89 |

c) Poly-N⁶-[hydrazinocarbonyl-nonanoyl]-N⁶[10-carboxy-3,6-bis(carboxymethyl)-9-ethoxycarbonylmethyl-3,6,9-triazadecanoyl]-poly-lysin
0,48 g (1,5 mmol) Sebacinsäure-mono(N'-t-butoxycarbonyl-hydrazid) (Beispiel 58 b) werden in Tetrahydrofuran gelöst und bei -5°C nacheinander mit 4,16 ml (30 mmol) Triethylamin und 0,15 ml (1,58 mmol) Chlor-ameisensäure-ethylester versetzt. Nach 15 Minuten werden bei -20°C eine Lösung von 2,46 g (15 mmol) poly-L-Lysin-Hydrochlorid in Wasser zugegeben und auf Raumtemperatur erwärmt. Nach 3 Stunden wird Tetrahydrofuran abdestilliert, mit Wasser verdünnt und bei pH = 9 portionsweise 18,15 g (45 mmol) N³-(2,6-Dioxomorpholinoethyl)-N⁶-(ethoxycarbonylmethyl)-3,6-diaza-octandisäure (Beispiel 13 a) versetzt und anschließend mit verdünnter Salzsäure auf pH = 7 gestellt. Die Lösung wird filtriert, das Filtrat über eine Ultrafiltrationsmembran (Amicon YM 5) von niedermolekularen Bestandteilen gereinigt und schließlich gefriergetrocknet. Dünnschichtchromatographisch sind keine Verunreinigungen zu erkennen.
Ausbeute: 6,6 g
Das erhaltene polymere Boc-Hydrazid wird ohne weitere Reinigung in Trifluoressigsäure aufgenommen, 1 Stunde bei Raumtemperatur gerührt und anschließend mit Ether gefällt, abgesaugt und getrocknet. Der Rückstand wird in Wasser auf pH 7 gestellt und gefriergetrocknet.
Ausbeute: 5,5 g
Hydrazid-Gehalt: 1,5 mol%
1 g dieser Verbindung komplexiert 200 mg Gd³⁺.
d) Gadolinium-Komplex des Poly-N⁶-[hydrazinocarbonyl-nonanoyl]-N⁶[10-carboxy-3,6-bis(carboxymethyl)-9-ethoxycarbonylmethyl-3,6,9-triazadecanoyl]-polylysins
5 g des in Beispiel 58 c beschriebenen Komplexbildners werden in 500 ml Wasser gelöst, mit 1,13 g Gd₂O₃ = 980 mg Gd³⁺ versetzt und 1 Stunde bei 80°C gerührt. Die erhaltene Lösung wird ultrafiltriert und anschließend gefriergetrocknet. Ausbeute: 5,6 g; Gd-Gehalt: 16,1 Gew%
λmax(H₂O)=200 nm (ε=9.000)

### BEISPIEL 57

a) Poly-N⁶-[hydrazinocarbonyl-nonanoyl]-N⁶-[10-carboxy-3,6,9-tris(carboxymethyl)-3,6,9-triazadecanoyl]-poly-lysin
   5,5 g des in Beispiel 56 c beschriebenen polymeren DTPA-ethylesters werden in möglichst wenig 2n NaOH-Lösung gelöst und nach 2 Stunden bei Raumtemperatur durch Zugabe von verdünnter Salzsäure neutralisiert. Die klare Lösung wird ultrafiltriert (Amicon YM 5) und anschließend gefriergetrocknet.
   Ausbeute: 5,1 g
   Ethoxybestimmung: negativ
   1 g dieser Verbindung komplexiert 200 mg Gd³⁺
b) Gadolinium-Komplex des Poly-N⁶-[hydrazinocarbonyl-nonanoyl]-N⁶-(10-carboxy-3,6,9-tris(carboxymethyl)-3,6,9-triazadecanoyl]-poly-lysins
   5 g des in Beispiel 57 a beschriebenen Komplexbildners werden wie in Beispiel 56 d beschrieben mit Gd₂O₃ komplexiert.
   Ausbeute: 5,6 g
   Gd-Gehalt: 16,2 Gew%
   λmax (H₂O) = 201 nm (ε = 9.000)

### BEISPIEL 58

a) Poly-α,β-Asparaginsäure-N-[14-carboxy-7,10-bis(carboxymethyl)-13-ethoxycarbonylmethyl-5-oxo-4,7,10,13-tetraazatetradecyl]-amid
   Zu 2,1 g (10 mmol) α,β-poly-Asparaginsäure-(N-3-aminopropyl)-amid (H.N. Kovacs et al.,J. Med. Chem. 10, 904-908 (1967)) in wäßriger Natronlauge (pH 9) werden portionsweise 12,1 g (30 mmol) N³-(2,6-Dioxomorpholinoethyl)-N⁶-(ethoxycarbonylmethyl)-3,6-diaza-octandisäure (Beispiel 13 a) gegeben. Durch gleichzeitige Zugabe von 1 n NaOH-Lösung wird der pH der Lösung zwischen 9 und 9,5 gehalten. Danach wird noch 15 Minuten bei Raumtemperatur gerührt und mit verdünnter Salzsäure neutralisiert. Die Lösung wird ultrafiltriert (Amicon YM 5) und das entsalzte Polymer gefriergetrocknet.
   Ausbeute: 5,5 g (86% der Theorie)
   Ethoxy-Bestimmung: 6,33%, das entspricht einer Acylierung des Poly-Asparaginsäure-amids von 90%
   1 g dieser Verbindung komplexiert 258 mg Gd³⁺.
b) Gadolinium-Komplex des Poly-α,β-Asparaginsäure-N-[14-carboxy-7,10-bis(carboxymethyl)-13-ethoxycarbonylmethyl-5-oxo-4,7,10,13-tetraazatetradecyl]-amids
   2,96 g (5 mmol)) des in Beispiel 60 a beschriebenen Komplexbildners werden bei pH 4-5 in Wasser mit 815 mg Gd₂O₃ = 708 mg Gd³⁺ versetzt und 1 Stunde bei 80°C gerührt; die erhaltene Lösung wird ultrafiltriert und anschließend gefriergetrocknet.
   Ausbeute: 3,30 g
   Gd-Gehalt: 20,5 Gew%
   Es wurden folgende Relaxivitäten gemessen [die Messungen der Relaxationszeiten T1 und T2 erfolgten in einem Minispec p20 (Bruker) bei 0,46 Tesla (= 20 MHz), 37°C].
   T1-Relaxivität: 12.4 (L/mmol sec)
   T2-Relaxivität: 12.5 (L/mmol sec)

### BEISPIEL 59

a) Poly-α,β-Asparaginsäure-N-[14-carboxy-7,10,13-tris(carboxymethyl)-5-oxo-4,7,10,13-tetraazatetradecyl]-amid
   5,0 g des in Beispiel 60 a beschriebenen polymeren DTPA-ethylesters werden in möglichst wenig 2 N NaOH-Lösung gelöst und nach 2 Stunden bei Raumtemperatur durch Zugabe von verdünnter Salzsäure neutralisiert. Die klare Lösung wird ultrafiltriert (Amicon YM 5) und anschließend gefriergetrocknet.
   Ausbeute: 4,6 g
   Ethoxybestimmung: negativ
   1 g dieser Verbindung komplexiert 260 mg Gd³⁺.
b) Gadolinium-Komplex des Poly-α,β-Asparaginsäure-N-[14-carboxy-7,10,13-tris-(carboxymethyl)-5-oxo-4,7,10,13-tetraazatetradecyl]-amids
   3,0 g des in Beispiel 61 a beschriebenen Komplexbildners werden analog Beispiel 60 b mit Gd₂O₃ komplexiert.
   Ausbeute: 3,6 g
   Gd-Gehalt: 20,7 Gew%
   Es wurden folgende Relaxivitäten gemessen [die Messungen der Relaxationszeiten T1 und T2 erfolgten in einem Minispec p20 (Bruker) bei 0,46 Tesla (= 20 MHz), 37°C].
   T1-Relaxivität: 12.9 (L/mmol sec)
   T2-Relaxivität: 12.7 (L/mmol sec)

### BEISPIEL 60

a) 3,6-Bis(carboxymethyl)-9-(N,N-diethylamino-carbonylmethyl)-3,6,9-triazaundecandisäure
20,17 g (50 mmol) N³-(2,6-Dioxomorpholinoethyl)-N⁶-(ethoxycarbonylmethyl)-3,6-diaza-octandisäure (Beispiel 13 a) werden in 250 ml DMF mit 34,7 ml (250 mmol) Triethylamin und 5,22 ml (50 mmol) Diethylamin bei 0°C versetzt, über Nacht bei Raumtemperatur gerührt, im Vakuum eingedampft, in 2 N NaOH-Lösung gelöst und 2 Stunden bei Raumtemperatur gerührt. Die Lösung wird über Amberlite IR 120 (H⁺) gegeben und das saure Eluat gefriergetrocknet.
Ausbeute: 14,1 g (63% der Theorie)
Schmelzpunkt: 130°C

| | | | | |
|---|---|---|---|---|
| Analyse: | Ber.: | C 48,21 | H 7,19 | N 12,49 |
| | Gef.: | C 48,01 | H 7,24 | N 12,55 |

b) Poly-N⁶-[10-carboxy-3,6-bis(carboxymethyl)-9-(N,N-diethylamino-carbonylmethyl)-3,6,9-triaza-decanoyl]-poly-L-lysin
8,97 g (20 mmol) der in Beispiel 62 a beschriebenen Säure werden in 150 ml Acetanhydrid suspendiert und nach Zugabe von 9,7 ml Pyridin über Nacht bei Raumtemperatur gerührt. Die Lösung wird mehrmals aus Ether umgefällt und das leicht gelbliche pulvrige Produkt zu einer Lösung von 1,6 g (10 mmol) poly-L-Lysin-Hydrochlorid in 200 ml Wasser gegeben, wobei der pH-Wert durch Zugabe von verdünnter Natronlauge bei 9,5 gehalten wird. Anschließend wird noch 1 Stunde nachgerührt, mit verdünnter Salzsäure neutralisiert, ultrafiltriert (Amicon YM 5) und gefriergetrocknet.
Ausbeute: 4,8 g (88% der Theorie)
Schmelzpunkt: oberhalb 200°C
1 g dieser Verbindung komplexiert 236 mg Gd³⁺
c) Gadolinium-Komplex des Poly-N⁶-[10-carboxy-3,6-bis(carboxymethyl)-9-(N,N-diethylamino-carbonylmethyl)-3,6,9-triaza-decanoyl]-poly-L-lysins 4,0 g des in Beispiel 62 b beschriebenen Komplexbildners werden analog Beispiel 60 b mit Gd₂O₃ komplexiert.
Ausbeute: 4,8 g
Gd-Gehalt: 19,1 Gew%
T₁-Relaxivität: 11,75 (L/mmol sec)
T₂-Relaxivität: 12,93 (L/mmol sec)

### BEISPIEL 61

a) 3,6-Bis(carboxymethyl)-9-morpholinocarbonylmethyl-3,6,9-triazaundecandisäure
20,17 g (50 mmol) N³-(2,6-Dioxomorpholinomethyl)-N⁶-((ethoxycarbonylmethyl)-3,6-diaza-octansäure (Beispiel 13a) werden in 250 ml DMF mit 34,7 ml (250 mmol) Triethylamin und 4,8 g (55 mmol) Morpholin in 20 ml DMF versetzt und wie in Beispiel 62a beschrieben, aufgearbeitet. Nach Gafriertrocknung des sauren Ionenaustauscher-eluats erhält man 16,2 g (70% der Theorie) der Titelverbindung.

| | | | | |
|---|---|---|---|---|
| Analyse: | Ber.: | C 46,75 | H 6,54 | N 12,12 |
| | Gef.: | C 46,85 | H 6,59 | N 12,07 |

b) Poly-N⁶[10-carboxy-3,6-bis(carboxymethyl)-9-morpholinocarbonylmethyl-3,6,9-triazadecanoyl]-poly-L-lysin
9,25 g (20 mmol) der in Beispiel 63a beschriebenen Säure werden analog Beispiel 62b mit Acetanhydrid/Pyridin aktiviert und mit 1,6 g (10 mmol) poly-L-Lysin-Hydrochlorid zur Reaktion gebracht und wie beschrieben aufgearbeitet.
Ausbeute: 4,8 g (85% der Theorie)
Schmelzpunkt: oberhalb 200°C
1 g dieser Verbindung komplexiert 230 mg Gd ³⁺
c) Gd-Komplex des Poly-N⁶-[10-carboxy-3,6-bis(carboxymethyl)-9-morpholinocarbonylmethyl-3,6,9-triazadecanoyl]-poly-L-lysins
4,0 g des in Beispiel 63b beschriebenen Komplexbildners werden analog Beispiel 60b mit Gd₂O₃ komplexiert.
Ausbeute: 4,6 g
Gd-Gehalt: 18,6 Gew%
T₁-Relaxivität: 12,31 (L/mmol sec)
T₂-Relaxivität: 13,15 (L/mmol sec)

### BEISPIEL 62

### Poly-N⁶-[4-(2,5,8,11-tetrakis-carboxymethyl-2,5,8,11-tetraazacyclododecylmethyl)-phenoxy-hydroxypropyl]-poly-L-lysin

Man bereitet bei 0°C eine Lösung von 2,5 g der nach 9e erhaltenen Verbindung in 10 ml Tetrahydrofuran und tropft sie bei 0°C zu einer Lösung von 0,3 g Poly-L-Lysin in 15 ml Wasser. Nach beendeter Zugabe erhitzt man noch eine Stunde unter Rückfluß. Nach Abziehen des Lösungsmittels erwärmt man in 25 ml warmer Ameisensäure und hält zwei Stunden auf 50°C. Danach gießt man in 300 ml Wasser ein, dialysiert und unterwirft das Retentat der Gefriertrocknung. Man erhält 1,1 g der Titelverbindung als weißes Pulver.

| | | | | |
|---|---|---|---|---|
| Analyse: | Gef.: | C 51,88 | H 6,35 | N 11,77 |

Der Gadolinium-Komplex wird, wie in Beispiel 38b beschrieben, erhalten.

| | | | | | |
|---|---|---|---|---|---|
| Analyse: | Gef.: | C 41,33 | H 4,72 | N 8,91 | Gd 18,88 |

Natriumsalz

| | | | | | |
|---|---|---|---|---|---|
| Analyse: | Gef.: | C 39,75 | H 4,61 | N 8,47 | Gd 17,91 |

N-Megluminsalz

| | | | | | |
|---|---|---|---|---|---|
| Analyse: | Gef.: | C 42,55 | H 4,52 | N 8,53 | Gd 12,97 |

### BEISPIEL 63

a) 2-(4-Benzyloxycarbonylmethoxybenzyl)-1,4,7,10-tetrakis-(tert.-butoxy-carbonyl-methyl)-1,4,7,10-tetraazacyclododecan
5,0 g der unter 9d erhaltenen Verbindung werden mit 200 mg Natriumhydrid (80% in Paraffin) in 35 ml trockenem Tetrahydrofuran unter Rühren langsam versetzt, dann tropft man bei Raumtemperatur 1,45 g Bromessigsäurebenzylester in 50 ml trockenem Tetrahydrofuran zu. Nach Rühren über Nacht saugt man vom ausgefallenen Natriumbromid ab, engt ein, nimmt in Diethylether auf und entfernt die restlichen anorganischen Bestandteile durch Waschen mit Wasser. Nach Trocknen über Magnesiumsulfat wird über eine Kieselgelsäule gereinigt. Nach dem Einengen zur Trockne erhält man 4,5 g eines farblosen Öls.

| | | | | |
|---|---|---|---|---|
| Analyse: | Ber.: | C 65,60 | H 8,39 | N 7,24 |
| | Gef.: | C 65,81 | H 8,50 | N 7,11 |

b) 2-(4-Carboxymethoxybenzyl)-1,4,7,10-tetrakis-(tert.-butoxy-carbonyl-methyl)-1,4,7,10-tetraazacyclododecan
3,8 g der nach a) erhaltenen Verbindung werden in 70 ml Tetrahydrofuran gelöst und in Gegenwart von 1,4 g 10% Palladium-Kohle hydriert, bis keine weitere Wasserstoffaufnahme stattfindet. Nach Filtration wird das Lösungsmittel abgedampft und die Substanz bei 0,01 torr getrocknet. Man erhält 1,7 g eines farblosen Öls. (Ausbeute. 51% der Theorie)

| | | | | |
|---|---|---|---|---|
| Analyse: | Ber.: | C 62,02 | H 8,48 | N 8,26 |
| | Gef.: | C 62,18 | H 8,62 | N 8,03 |

c) Poly-N⁶-[4-(2,5,8,11-tetrakis-carboxymethyl-2,5,8,11-tetraazacyclododecyl-methyl)-phenoxyacetyl]-poly-L-lysin
Man bereitet bei 0°C eine Lösung von 12,8 g der nach b) erhaltenen Verbindung, 2,35 g Chlorameisensäure-isobutylester und 1 g Triethylamin in 100 ml Tetrahydrofuran und tropft diese Lösung bei 0°C in eine Lösung von 1,6 g Poly-L-Lysin und 1,4 g Kaliumhydroxid in 80 ml Wasser ein. Nach beendeter Zugabe dekantiert man vom Niederschlag und behandelt ihn zwei Stunden bei 50°C mit 35 ml warmer Ameisensäure. Danach gießt man in Wasser ein, dialysiert und unterwirft das Retentat einer Gefriertrocknung. Man erhält 4,1 g eines weißen Pulvers.

| | | | | |
|---|---|---|---|---|
| Analyse: | Gef.: | C 59,21 | H 3,74 | N 13,82 |

Der Gadolinium-Komplex wird, wie in Beispiel 38b beschrieben, erhalten.

| | | | | | |
|---|---|---|---|---|---|
| Analyse: | Gef.: | C 49,76 | H 2,23 | N 11,56 | Gd 21,02 |

Natriumsalz

| | | | | | |
|---|---|---|---|---|---|
| Analyse: | Gef.: | C 47,54 | H 2,11 | N 11,20 | Gd 19,89 |

N-Megluminsalz

| | | | | | |
|---|---|---|---|---|---|
| Analyse: | Gef.: | C 50,88 | H 2,03 | N 11,47 | Gd 14,32 |

### Beispiel für die NMR-Diagnostik in vivo

0,1 mmol Gd/kg wurden in Form des Gd-Komplexes des Poly-N-[10-carboxy-3,6,9-tris-(carboxymethyl)-3,6,9-triazadecanoyl]-poly-L-lysin-polyhydrazids (Beispiel 39c) einer Nacktmaus (Balb/c nu/nu, weibl., 25 g) mit einem subcutanen HT29 Coloncarcinom i.v. appliziert.

Die Substanz war in 300 µl 0,9% NaCl gelöst. Das Tier wurde in einem Kernspintomographen der Firma General Electric mit einem 2 Tesla Magneten untersucht.

Es wurden Aufnahmen vor und nach Applikation des Kontrastmittels mit einer Spin-Echo-Sequenz (T_{R} = 400 msec, T_{E} = 30 msec) im Bereich der Leber und des Tumors gemacht.

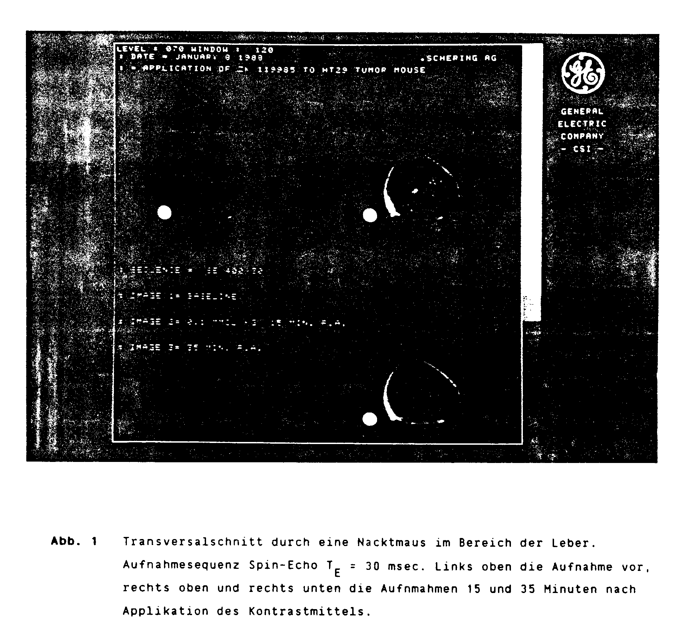

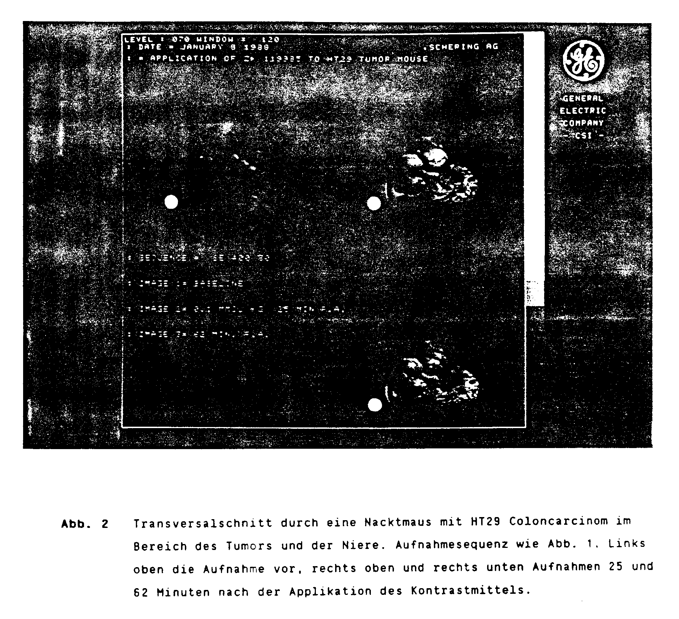

Abbildung 1 zeigt die Aufnahme (Transversalschnitt) im Leberbereich.
Abbildung 2 zeigt einen Transversalschnitt durch den Tumor und die Niere.

## Patentansprüche

1. Polymere bestehend aus einem Carbonsäuregruppen enthaltenden Liganden, mindestens einem Ion eines Elements der Ordnungszahlen 21-29, 42, 44 oder 57-83 sowie gegebenenfalls Kationen anorganischer und/oder organischer Basen, Aminosäuren oder Aminosäureamide,
dadurch gekennzeichnet, daß sie als Polymer-Einheiten komplexbildende Strukturen der allgemeinen Formel I 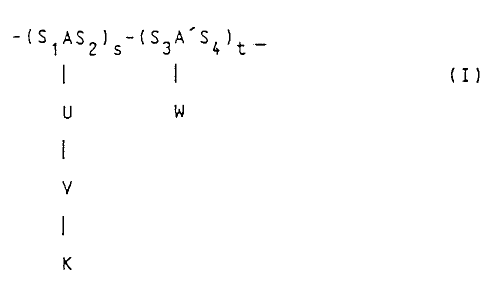 aufweisen, worin
A und A' jeweils 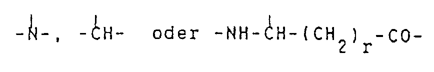 mit r in der Bedeutung der Ziffern 0 oder 1,
s die ganzen Zahlen von 7 bis 20.000
t die ganzen Zahlen von 0 bis 20.000,
U eine direkte Bindung, die Gruppe 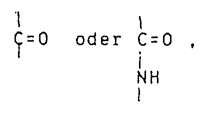
V, S₁,S₂,S₃ und S₄ jeweils eine gegebenenfalls Imino-, Phenylen-, Phenylenoxy-, Phenylenimino-, Amid-, Hydrazid-, Estergruppe(n), Sauerstoff-, Schwefel- und/oder Stickstoff-Atom(e) enthaltende gegebenenfalls durch Hydroxy-, Mercapto-, Imino-, Epoxy-, Oxo-, Thioxo- und/oder Aminogruppe-(n) substituierte geradkettige, verzweigte, gesättigte oder ungesättigte C₀-C₂₀-Alkylengruppe,
K ein Komplexbildner der allgemeinen Formeln IA, IB, IC oder ID 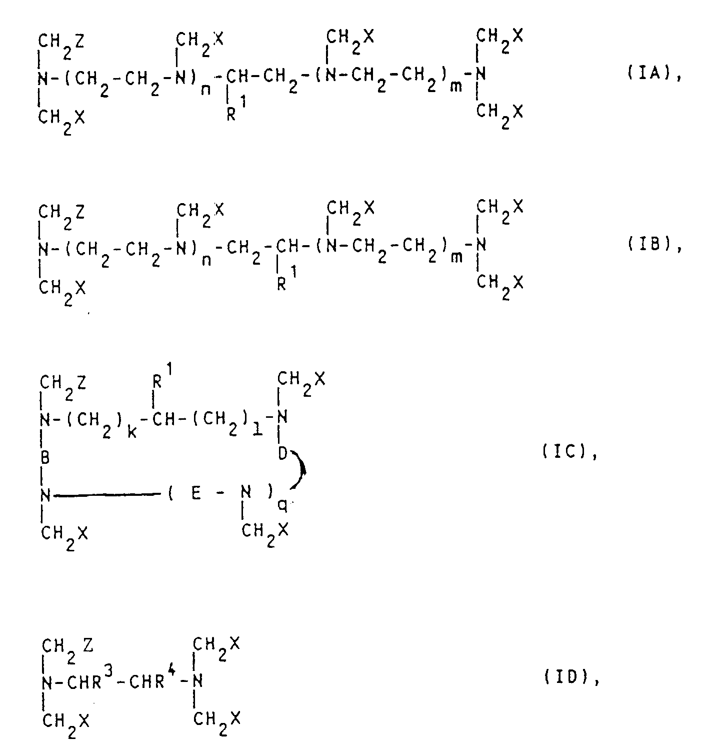 wobei
n und m jeweils für die Ziffern 0, 1, 2, 3 oder 4, wobei n und m zusammen nicht mehr als 4 ergeben,
k für die Ziffern 1, 2, 3, 4 oder 5,
l für die Ziffern 0, 1, 2, 3, 4 oder 5,
q für die Ziffern 0, 1 oder 2,
X unabhängig voneinander jeweils für die Reste -COOH oder V' steht,
worin
V' den am Ende eine funktionelle Gruppe aufweisenden Rest V bedeutet, wobei, falls das Molekül V' enthält, mindestens 0,1% der Substituenten X für V' stehen,
B, D und E, die gleich oder verschieden sind, jeweils für die Gruppe 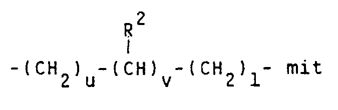 R² in der Bedeutung von Wasserstoff oder einer gegebenenfalls Sauerstoff- und/oder Stickstoffatom(e) enthaltenden gegebenenfalls durch Hydroxy-und/oder Aminogruppe(n) substituierten geradkettigen, verzweigten, gesättigten oder ungesättigten C₁-C₂₀-Alkylgruppe,
u in der Bedeutung der Ziffern 0, 1, 2, 3, 4 oder 5,
v in der Bedeutung der Ziffern 0 oder 1,
wobei B, D und E jeweils mindestens 2 und maximal 5 Kohlenstoffatome enthalten,
Z für die Gruppe 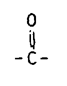 oder den Rest X,
R¹ für eine direkte Bindung oder ein Wasserstoffatom,
R³ und R⁴ gemeinsam für eine gegebenenfalls durch 1-2 Hydroxy- oder 1-3 C₁-C₄-Alkylgruppen substituierte Dimethylen- oder Trimethylen-methingruppe,
stehen,
mit der Maßgabe, daß Z nur dann für die Gruppe 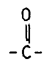 steht, wenn R¹ gleichzeitig ein Wasserstoffatom bedeutet, und daß Z nur dann für den Rest X steht, wenn R¹ gleichzeitig eine direkte Bindung bedeutet,
W ein Wasserstoffatom, die Gruppe U_{w}-V_{w}-K_{w} , wobei U_{w} , V_{w} und K_{w} jeweils eine der für U, V und K genannten Bedeutungen haben,
V' oder die Gruppe 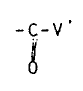
bedeuten, mit der Maßgabe, daß gewünschtenfalls ein Teil der COOH-Gruppen als Ester und/oder Amid vorliegt.

2. Polymer-Komplexe gemäß Anspruch 1, dadurch gekennzeichnet, daß sie als Polymer-Einheiten komplexbildende Strukturen der allgemeinen Formel Iα 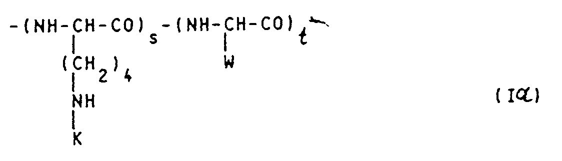 enthalten.

3. Polymer-Komplexe gemäß Anspruch 1, dadurch gekennzeichnet, daß die in V' enthaltene funktionelle Gruppe die Gruppe -NH₂; -NHR; -NHNH₂; -NRNH₂; -SH, -OH, -COCH₃; -CH=CH-CO₂R; 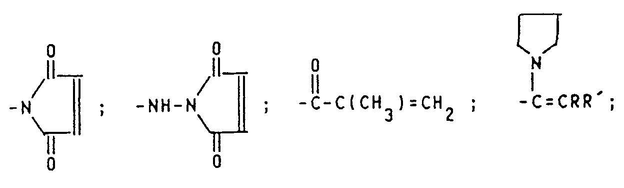 -C≡C-C≡CR; 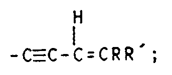 -C₆H₄CH₂Br ; 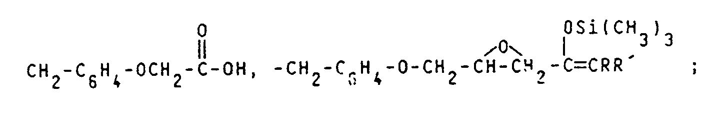 -CH₂Br; -CH₂J; -COCH₂Br; -CH=CH-CH₂Br; 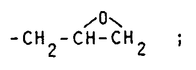 -0-SO₂-C₆H₄CH₃; -SO₂Cl; -SOCl; 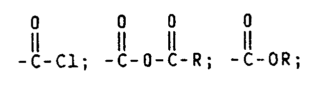 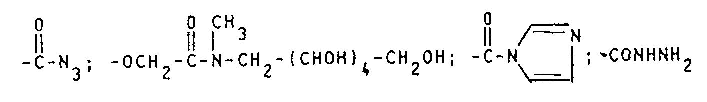 ist,
wobei R und R' gleich oder verschieden sind und jeweils für ein Wasserstoffatom, einen gesättigten oder ungesättigten gegebenenfalls durch eine Phenylgruppe substituierten C₁-C₂₀-Alkylrest oder eine Phenylgruppe stehen, bedeuten.

4. Polymer-Komplexe gemäß Anspruch 2, dadurch gekennzeichnet, daß V für eine -CH₂-O-C₆H₄-CH₂-; -CH₂-CH(OH)-CH₂-O-C₆H₄-CH₂-; -C(=NH)-O-C₆H₄-CH₂-; -(CH₂)₄-NH-CO-CH₂-O-C₆H₄-CH₂-; -(CH₂)₄-NH-CH₂-CH(OH)-CH₂-O-C₆H₄-CH₂-; -(CH₂)₃-O-C₆H₄-CH₂-; -CH₂-CO-NH-(CH₂)₃-O-CH₂-; -CH₂-CO-NH-NH-; -CH₂-CONH-(CH₂)₂-; -CH₂-CO-NH-(CH₂)₁₀-; -CH₂-CONH-(CH₂)₂-S-; -(CH₂)₄-NH-CO-(CH₂)₈-; -CH₂-CO-NH-(CH₂)₃-NH-; -(CH₂)₃-NH-gruppe steht.

5. Pharmazeutische Mittel enthaltend mindestens einen Polymer-Komplex nach Anspruch 1, gegebenenfalls mit den in der Galenik üblichen Zusätzen.

6. Verwendung von mindestens einem Polymer-Komplex gemäß Anspruch 1 für die Herstellung von Mitteln für die NMR-, Röntgen- oder Ultraschall-Diagnostik.

7. Verfahren zur Herstellung von Polymeren gemäß den Ansprüchen 1 bis 4, dadurch
gekennzeichnet, daß man mit Amino-, Imino- und/oder 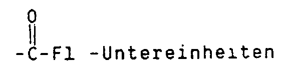 versehene Polymere, worin Fl für eine Fluchtgruppe steht, durch Alkylierung, Acylierung, Amidierung und/oder Hydrazinierung in Verbindungen umwandelt, die als Polymer-Einheiten Strukturen der allgemeinen Formel I' 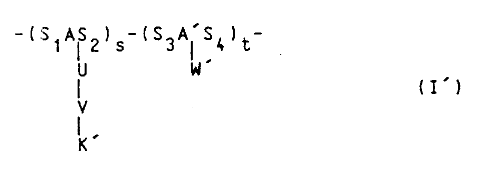 aufweisen, worin
A, A', U, V, S₁, S₂, S₃, S₄, s und t die oben genannte Bedeutung haben und K' Komplexbildner der allgemeinen Formeln I'A, I'B, I'C und I'D, die identisch mit den für IA, IB, IC und ID angegebenen Formeln sind, jedoch anstelle der Substituenten X und Z jeweils X' und Z' tragen, wobei
X' unabhängig voneinander für -COOH und
Z' für die Gruppe 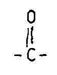 oder den Rest X' stehen, mit der Maßgabe, daß gewünschtenfalls ein Teil der COOH-Gruppen als Ester und/oder Amid vorliegt,
W' ein Wasserstoffatom, die Gruppe U_{w}-V_{w}-K'_{w}, wobei U_{w} und V_{w} die oben genannte Bedeutung haben und K'_{w} die für K' genannte Bedeutung hat,
V'' oder die Gruppe 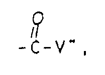 wobei V'' für den am Ende eine funktionelle Gruppe aufweisenden Rest V steht,
bedeuten,
diese dann in an sich bekannter Weise gewünschtenfalls mit mindestens einem Metalloxid oder Metallsalz eines Elements der Ordnungszahlen 21-29, 42, 44 oder 57-83 umsetzt und gewünschtenfalls K' und/oder W' durch Umwandlung mindestens einer der in K' oder W' enthaltenen -CO₂H- Gruppen in die gewünschte, am Ende eine funktionelle Gruppe aufweisende Alkylengruppe in K und W überführt, wobei die angegebenen Reaktionsschritte in beliebiger Reihenfolge durchgeführt werden können, und gegebenenfalls anschließend in den so erhaltenen Polymer-Komplexen noch vorhandene acide Wasserstoffatome ganz oder teilweise durch Kationen anorganischer und/oder organischer Basen, Aminosäuren oder Aminosäureamide substituiert bzw. die entsprechenden Säuregruppen ganz oder teilweise in Ester oder Amide überführt.

8. Verfahren zur Herstellung der pharmazeutischen Mittel gemäß Anspruch 5, dadurch gekennzeichnet, daß man in Wasser oder physiologischer Salzlösung gelösten oder suspendierten Polymer-Komplex gegebenenfalls mit den in der Galenik üblichen Zusätzen, in eine für die enterale oder parenterale Applikation geeignete Form bringt.

## Claims

1. Polymers consisting of a ligand containing carboxylic acid groups, at least one ion of an element having an atomic number 21-29, 42, 44 or 57-83 and optionally cations of inorganic and/or organic bases, amino acids or amino acid amides, characterised in that they comprise as polymer units complex-forming structures of the general formula I 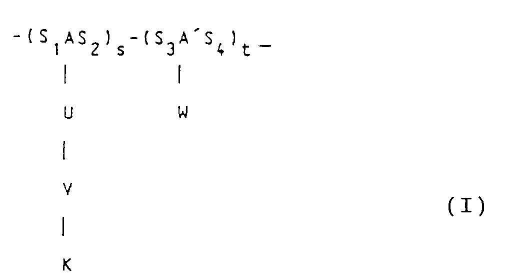 wherein
A and A' are each 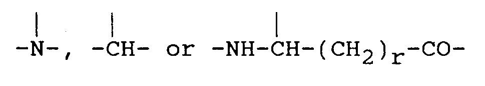 in which r is the number 0 or 1
s represents an integer of from 7 to 20,000,
t represents an integer of from 0 to 20,000,
U represents a direct bond, the group 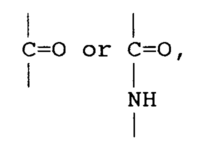
V, S₁, S₂, S₃ and S₄ are each a straight-chain, branched, saturated or unsaturated C₀-C₂₀-alkylene group optionally containing imino, phenylene, phenyleneoxy, phenyleneimino, amide, hydrazide, ester group(s), oxygen, sulphur and/or nitrogen atom(s) and optionally substituted by hydroxy, mercapto, imino, epoxy, oxo, thioxo and/or amino group(s),
K represents a complex former of the general formula IA, IB, IC or ID 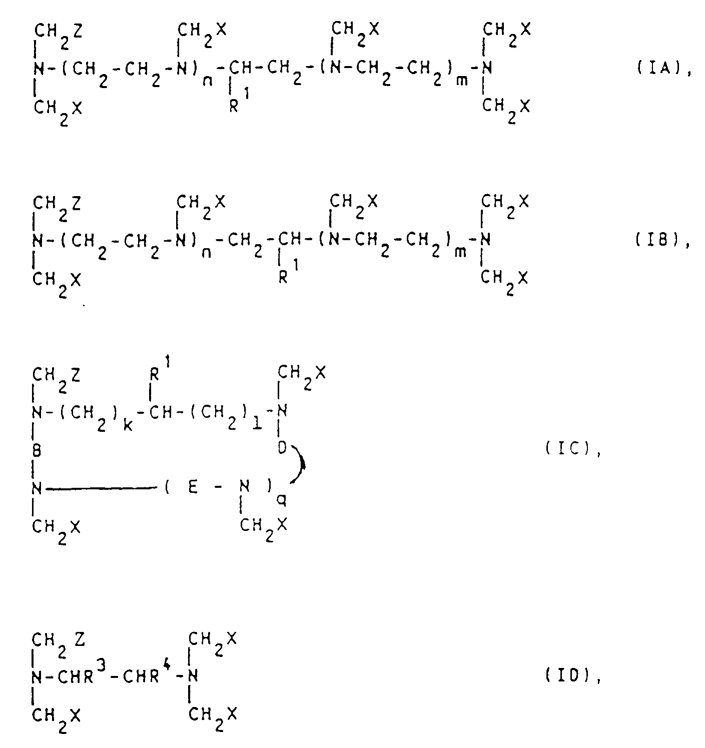
wherein
n and m each represents the number 0, 1, 2, 3 or 4, the sum of n and m not being greater than 4,
k represents the number 1, 2, 3, 4 or 5,
l represents the number 0, 1, 2, 3, 4 or 5,
q represents the number 0, 1 or 2,
each X represents, independently of the others, a radical -COOH or V' wherein
V' represents a radical V terminated with a functional group wherein if the molecule contains V' at least 0.1 % of the substituents X represent V',
B, D and E are the same or different and each represents
the group 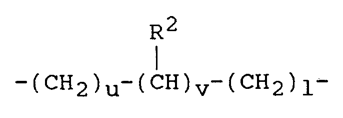 wherein
R² represents hydrogen or a straight-chain, branched, saturated or unsaturated C₁-C₂₀-alkyl group optionally containing oxygen and/or nitrogen atom(s) and optionally substituted by hydroxy and/or amino group(s),
u represents the number 0, 1, 2, 3, 4 or 5,
v represents the number 0 or 1,
B, D and E each containing a minimum of 2 and a maximum of 5 carbon atoms,
Z represents the group 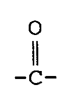 or the radical X,
R¹ represents a direct bond or a hydrogen atom,
R³ and R⁴ together represent a dimethylene- or trimethylene-methyne group optionally substituted by 1 or 2 hydroxy groups or by from 1 to 3 C₁-C₄-alkyl groups,
with the proviso that, only when R¹ simultaneously represents a hydrogen atom can Z represent the group 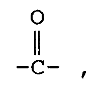 and only when R¹ simultaneously represents a direct bond can Z represent the radical X,
and
W represents a hydrogen atom, the group U_{w}-V_{w}-K_{w} wherein U_{w}, V_{w} and K_{w} each has one of the meanings given for U, V and K, or W represents V' or the group 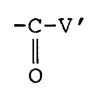
with the proviso that, if desired, part of the COOH groups are present in the form of an ester and/or amide.

2. Polymer complexes according to claim 1, characterised in that they comprise as polymer units complex-forming structures of the general formula Iα

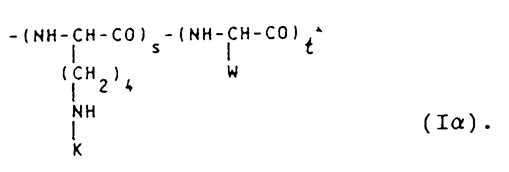

3. Polymer complexes according to claim 1, characterised in that the functional group contained in V' is the group -NH₂; -NHR; -NHNH₂; -NRNH₂; -SH, -OH, -COCH₃; -CH=CH-CO₂R; 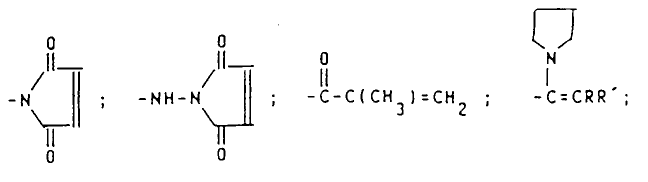 -C≡C-C≡CR; 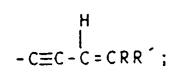 -C₆H₄CH₂Br ; 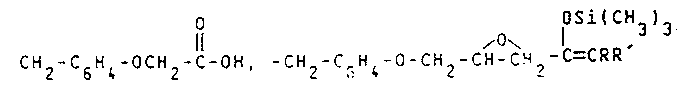 -CH₂Br; -CH₂I; -COCH₂Br; -CH=CH-CH₂Br; 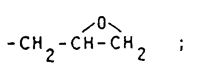 -0-SO₂-C₆H₄CH₃; -SO₂Cl; -SOCl; 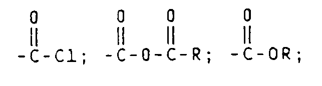 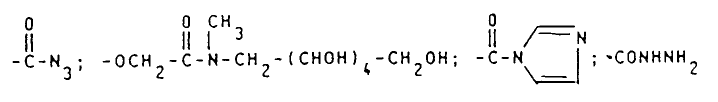 wherein R and R' are the same or different and each represents a hydrogen atom, a saturated or unsaturated C₁-C₂₀-alkyl radical optionally substituted by a phenyl group, or a phenyl group.

4. Polymer complexes according to claim 2, characterised in that V represents a -CH₂-O-C₆H₄-CH₂-, -CH₂-CH(OH)-CH₂-O-C₆H₄-CH₂-, -C(=NH)-O-C₆H₄-CH₂-, -(CH₂)₄-NH-CO-CH₂-O-C₆H₄-CH₂-, -(CH₂)₄-NH-CH₂-CH(OH)-CH₂-O-C₆H₄-CH₂-, -(CH₂)₃-O-C₆H₄-CH₂-, -CH₂-CO-NH-(CH₂)₃-O-CH₂-, -CH₂-CO-NH-NH-, -CH₂-CONH-(CH₂)₂-, -CH₂-CO-NH-(CH₂)₁₀-, -CH₂-CONH-(CH₂)₂-S-, -(CH₂)₄-NH-CO-(CH₂)₈-, -CH₂-CO-NH-(CH₂)₃-NH- or -(CH₂)₃-NH- group.

5. Pharmaceutical composition comprising at least one polymer complex according to claim 1, optionally together with additives customary in galenical pharmacy.

6. Use of at least one polymer complex according to claim 1 for the manufacture of agents for NMR, X-ray or ultrasound diagnostics.

7. Process for the preparation of polymers according to any one of claims 1 to 4, characterised in that polymers provided with amino, imino and/or 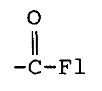 sub-units, wherein Fl represents a leaving group, are converted by alkylation, acylation, amidation and/or hydrazination into compounds that comprise as polymer units structures of the general formula I' 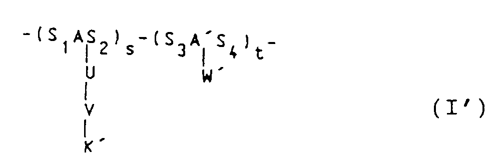 wherein
A, A', U, V, S₁, S₂, S₃, S₄, s and t have the meanings given above and K' represents complex formers of the general formulae I'A, I'B, I'C and I'D, which are identical to the formulae given for IA, IB, IC and ID but each carry, instead of the substituents X and Z, X' and Z' wherein
each X' represents, independently of the others, -COOH and
Z' represents the group 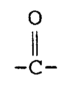 or the radical X', with the proviso that, if desired, part of the COOH groups are present in the form of an ester and/or amide,
and
W' represents a hydrogen atom, the group U_{w}-V_{w}-K'_{w} wherein U_{w} and V_{w} have the meanings given above and K'_{w} has the meaning given for K', or W' represents V'' or the group 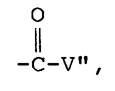 wherein V'' represents a radical V terminated with a functional group,
and this is then, in a manner known per se, if desired reacted with at least one metal oxide or metal salt of an element having an atomic number of 21-29, 42, 44 or 57-83 and, if desired, K' and/or W' is/are converted into K and W by converting at least one of the -CO₂H- groups contained in K' or W' into the desired alkylene group terminated with a functional group, wherein the said reaction steps may be carried out in any sequence, and optionally some or all of the acidic hydrogen atoms still present in the so obtained polymer complexes are replaced by cations of inorganic and/or organic bases, amino acids or amino acid amides, or some or all of the corresponding acid groups are converted into esters or amides.

8. Process for the manufacture of pharmaceutical compositions according to claim 5, characterised in that the polymer complex suspended or dissolved in water or physiological saline is brought, optionally together with additives customary in galenical pharmacy, into a form suitable for enteral or parenteral administration.

## Revendications

1. Polymères composés d'un ligand contenant des groupes d'acides carboxyliques d'au moins un ion d'un élément ayant le nombre atomique de 21 à 29, 42, 44 ou de 57 à 83 ainsi qu'éventuellement de cations de bases minérales et/ou organiques, d'acides aminés ou d'amides d'acides aminés, caractérisés en ce qu'ils présentent comme motifs polymères des structures complexantes de formule générale I : 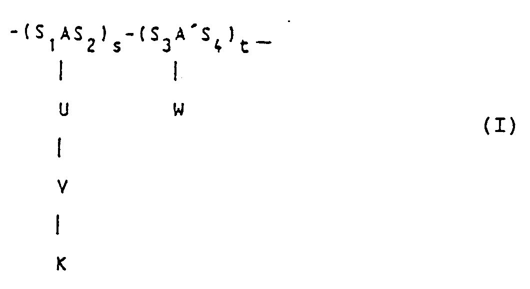 dans laquelle
A et A' représentent chaque fois 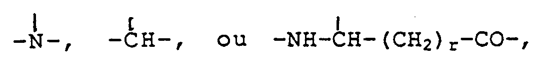 avec r ayant la signification des chiffres 0 ou 1,
s représente le nombre entier de 7 à 20 000,
t représente le nombre entier de 0 à 20 000,
U représente une liaison directe, le groupe 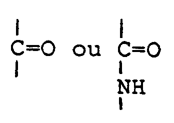 V, S₁, S₂, S₃ et S₄ représentent chacun un groupe alkylène en C₀-C₂₀ saturé ou insaturé contenant éventuellement des groupes imino, phénylène, phénylènoxy, phénylènimino, amido, hydrazido, ester, des atomes d'oxygène, de soufre et/ou d'azote, éventuellement substitués par des groupes hydroxy, mercapto, imino, époxy, oxo, thioxo et/ou amino,
K est un agent complexant de formules générales IA, IB, IC ou ID 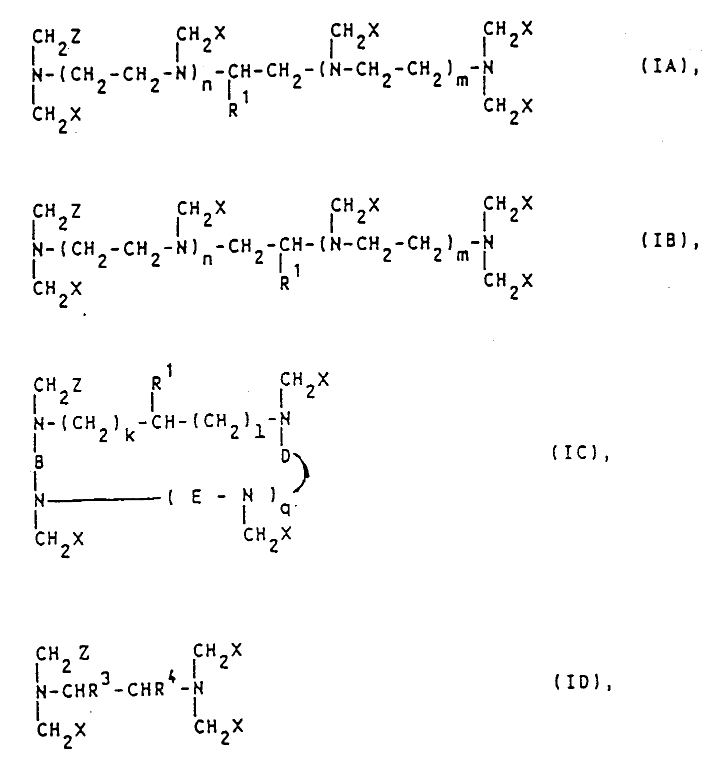 n et m représentent chacun les nombres 0, 1, 2, 3 ou 4,
n et m ensemble ne donnant pas plus que 4,
k représente les nombres 1, 2, 3, 4 ou 5,
l représente les nombres 0, 1, 2, 3, 4 ou 5,
q représente les nombres 0, 1 ou 2,
X indépendamment l'un de l'autre chacun représente des radicaux -COOH ou V', V' représentant un groupe fonctionnel présentant à l'extrémité le radical V, dans le cas où la molécule contient V', au moins 0,1 % des substituants X représente V',
B, D et E, identiques ou différents, représentent chacun le groupe 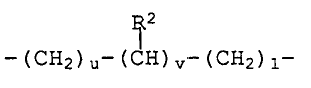 avec
R² ayant la signification de l'hydrogène ou d'un groupe alkyle en C₁-C₂₀ saturé ou insaturé, ramifié, linéaire, éventuellement contenant des atomes d'oxygène et/ou d'hydrogène éventuellement substitués par des groupes hydroxy et/ou amino,
u ayant la signification des nombres de 0, 1, 2, 3, 4 ou 5,
v ayant la signification des nombres 0 ou 1,
B, D et E contenant chaque fois au moins 2 et au maximum 5 atomes de carbone,
Z représentant le groupe 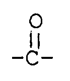 ou le radical X,
R¹ représentant une liaison directe ou un atome d'hydrogène,
R³ et R⁴ ensemble représentent un groupe diméthylène- ou triméthylène-méthyne éventuellement substitué par un à deux groupes hydroxy ou un à trois groupes alkyle en C₁-C₄,
à la conditon, que Z ne représente le groupe 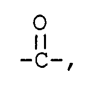 que si R¹ représente simultanément un atome d'hydrogène, et que Z ne corresponde au radical X que si R¹ représente simultanément une liaison directe,
W représente un atome d'hydrogène, le groupe U_{w}-V_{w}-K_{w}, U_{w}, V_{w} et K_{w} ayant chaque fois les significations données pour U, V et K, V' ou du 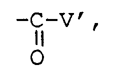 à la condition que, si on le désire, une partie des groupes COOH étant présente sous forme d'ester et/ou d'amide.

2. Complexes polymères selon la revendication 1, caractérisés en ce qu'ils contiennent comme motifs polymères des structures complexantes de formule générale Iα

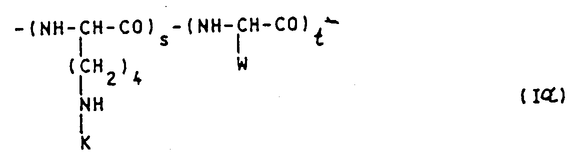

3. Complexes polymères selon la revendication 1, caractérisés en ce que les groupes fonctionnels contenus dans V' sont les groupe -NH₂ ; -NHR ; -NHNH₂ ; -NRNH₂ ; -SH, -OH, -COCH₃ ; -CH=CH-CO₂R ; 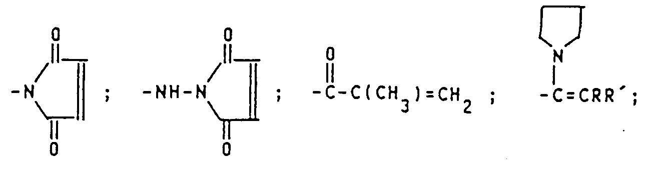 -C≡C-C≡CR; 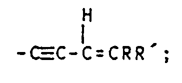 -C₆H₄CH₂Br ; 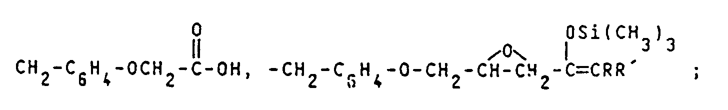 -CH₂Br; -CH₂J; -COCH₂Br; -CH=CH-CH₂Br; 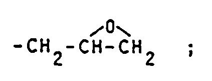 -0-SO₂-C₆H₄CH₃; -SO₂Cl; -SOCl; 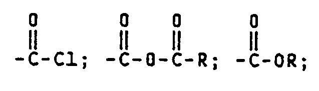 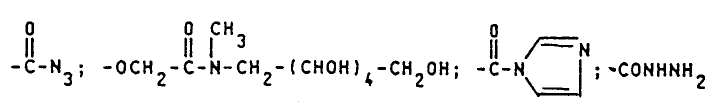 R et R' étant identiques ou différents et représentant chaque fois un atome d'hydrogène, un radical alkyle en C₁-C₂₀ saturé ou insaturé, éventuellement substitués par un groupe phényle, ou un groupe phényle.

4. Complexes polymères selon la revendication 2, caractérisés en ce que V représente un groupe
-CH₂-O-C₆H₄-CH₂-; -CH₂-CH(OH)-CH₂-O-C₆H₄-CH₂-; -C(=NH)-O-C₆H₄-CH₂-; -(CH₂)₄-NH-CO-CH₂-O-C₆H₄-CH₂-; -(CH₂)₄-NH-CH₂-CH(OH)-CH₂-O-C₆H₄-CH₂-; -(CH₂)₃-O-C₆H₄-CH₂-; -CH₂-CO-NH-(CH₂)₃-O-CH₂-; -CH₂-CO-NH-NH-; -CH₂-CONH-(CH₂)₂-; -CH₂-CO-NH-(CH₂)₁₀-; -CH₂-CONH-(CH₂)₂-S-; -(CH₂)₄-NH-CO-(CH₂)₈-; -CH₂-CO-NH-(CH₂)₃-NH-; -(CH₂)₃-NH-

5. Agents pharmaceutiques contenant au moins un complexe polymère selon la revendication 1, éventuellement avec des adjuvants usuels en galénique.

6. Utilisation d'au moins un complexe polymère selon la revendication 1 pour la préparation d'agents pour le diagnostic par la RMN, par la radiographie à rayons X ou par ultrasons.

7. Procédé pour la préparation de polymères selon les revendications 1 à 4, caractérisé en ce qu'on transforme des polymères comportant des sous-unités amino, imino et/ou 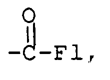 dans laquelle Fl représente un groupe éliminable, par alkylation, par acylation, par amidification et/ou par hydrazination en composés qui présentent en tant que motifs polymères des structures de formule générale I' 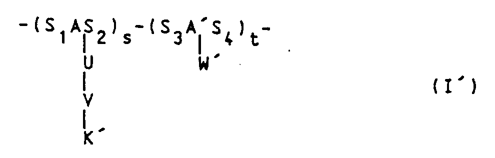 dans laquelle
A, A', U, V, S₁, S₂, S₃, S₄, s et t ont les significations données ci-dessus et K' représente des agents complexants de formules générales I'A, I'B, I'C et I'D qui sont identiques aux formules données pour IA, IB, IC et ID, cependant, au lieu des substituants X et Z ils portent chaque fois les substituants X' et Z',
X' représentent indépendamment l'un de l'autre -COOH, et Z' représentent le groupe 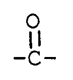 ou le radical X', à la condition que, si on le souhaite, une partie des groupes COOH soit présente un tant qu'ester et/ou amide,
W' représente un atome d'hydrogène, le groupe U_{w}-V_{w}-K'_{w}, U_{w} et V_{w} ayant les significations données et K'_{w} ayant la signification donnée pour K,
V'' ou le groupe 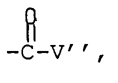 V'' représentant le radical V lequel radical présente à l'extrémité un groupe fonctionnel, puis on fait réagir ceux-ci de façon connue en soi, si on le désire, avec au moins un oxyde métallique au sel métallique d'un élément ayant le nombre atomique de 21 à 29, 42, 44 ou de 57 à 83 et, si on le désire, K' et/ou W' par transformation d'au moins l'un des groupes -CO₂H contenu dans K' ou W' en le groupe alkylène présentant à l'extrémité un groupe fonctionnel dans K et W, dans n'importe quel ordre, et éventuellement ensuite substituer les atomes d'hydrogène d'azides encore présents dans le complexe polymère ainsi obtenu par des cations de bases minérales et/ou organiques, d'acides aminés ou d'amides d'acides aminés, ou bien transformer les groupes acides correspondants entièrement ou partiellement en esters ou amides.

8. Procédé pour la préparation d'agents pharmaceutiques selon la revendication 5, caractérisé en ce que le complexe polymère dissous ou suspendu dans l'eau ou dans une solution physiologique de sel, éventuellement avec des adjuvants usuels en galénique, et mis en forme appropriée pour une application entérale ou parentérale.
